# EUROPEAN PATENT APPLICATION

(11) **EP 1 845 163 A2**
(43) Date of publication of application: **17.10.2007**
(21) Application number: 07007319.2
(22) Date of filing: 02.11.1999
(51) Int. Cl.: C12N 15/86, C12N 15/35, C12N 5/10, A61K 48/00

(54) **Adeno-associated virus serotype l nucleic acid sequences, vetors and host cells containing same**

(30) Priority: 05.11.1998 US 107114 P
(62) Divisional of application: 99961559.4
(71) Applicant: THE TRUSTEES OF THE UNIVERSITY OF PENNSYLVANIA, Philadelphia, PA 19104-3147 (US)
(72) Inventor: Wilson, James N., Galdwyne, PA 19035 (US); Xiao, Weidong, Fort Washington, PA 19034 (US)
(74) Representative: Manaton, Ross Timothy

(57) **Abstract**

The nucleic acid sequences of adeno-associated virus (AAV) serotype 1 are provided, as are vectors and host cells containing these sequences and functional fragments thereof. Also provided are methods of delivering genes via AAV-1 derived vectors.

### Annex to the application documents - subsequently filed sequences listing

SEQUENCE LISTING

<110> Wilson, James M.
      Xiao, Weidong
      The Trustees of the University of Pennsylvania

<120> Adeno-Associated Virus Serotype I Nucleic Acid
      Sequences, Vectors and Host Cells Containing Same

<130> GNVPN.031PCT

<140>
<141>

<150> 60/107,114
<151> 1998-11-05

<160> 20

<170> PatentIn Ver. 2.0

<210> 1
<211> 4718
<212> DNA
<213> AAV-1

<220>
<221> CDS
<222> (335)..(2206)

<220>
<221> CDS
<222> (2223)..(4430)

<400> 1
ttgcccactc cctctctgcg cgctcgctcg ctcggtgggg cctgcggacc aaaggtccgc 60

agacggcaga gctctgctct gccggcccca ccgagcgagc gagcgcgcag agagggagtg 120

ggcaactcca tcactagggg taatcgcgaa gcgcctccca cgctgccgcg tcagcgctga 180

cgtaaattac gtcatagggg agtggtcctg tattagctgt cacgtgagtg cttttgcgac 240

attttgcgac accacgtggc catttagggt atatatggcc gagtgagcga gcaggatctc 300

cattttgacc gcgaaatttg aacgagcagc agcc atg ccg ggc ttc tac gag atc 355
                                      Met Pro Gly Phe Tyr Glu Ile
                                        1               5

gtg atc aag gtg ccg agc gac ctg gac gag cac ctg ccg ggc att tct 403
Val Ile Lys Val Pro Ser Asp Leu Asp Glu His Leu Pro Gly Ile Ser
        10                  15                  20

gac tcg ttt gtg agc tgg gtg gcc gag aag gaa tgg gag ctg ccc ccg 451
Asp Ser Phe Val Ser Trp Val Ala Glu Lys Glu Trp Glu Leu Pro Pro
    25                  30                  35

gat tct gac atg gat ctg aat ctg att gag cag gca ccc ctg acc gtg 499
Asp Ser Asp Met Asp Leu Asn Leu Ile Glu Gln Ala Pro Leu Thr Val
40                  45                  50                  55

gcc gag aag ctg cag cgc gac ttc ctg gtc caa tgg cgc cgc gtg agt 547
Ala Glu Lys Leu Gln Arg Asp Phe Leu Val Gln Trp Arg Arg Val Ser
                60                  65                  70

aag gcc ccg gag gcc ctc ttc ttt gtt cag ttc gag aag ggc gag tcc 595
Lys Ala Pro Glu Ala Leu Phe Phe Val Gln Phe Glu Lys Gly Glu Ser
            75                  80                  85

tac ttc cac ctc cat att ctg gtg gag acc acg ggg gtc aaa tcc atg 643
Tyr Phe His Leu His Ile Leu Val Glu Thr Thr Gly Val Lys Ser Met
        90                  95                  100

gtg ctg ggc cgc ttc ctg agt cag att agg gac aag ctg gtg cag acc 691
Val Leu Gly Arg Phe Leu Ser Gln Ile Arg Asp Lys Leu Val Gln Thr
    105                 110                 115

atc tac cgc ggg atc gag ccg acc ctg ccc aac tgg ttc gcg gtg acc 739
Ile Tyr Arg Gly Ile Glu Pro Thr Leu Pro Asn Trp Phe Ala Val Thr
120                 125                 130                 135

aag acg cgt aat ggc gcc gga ggg ggg aac aag gtg gtg gac gag tgc 787
Lys Thr Arg Asn Gly Ala Gly Gly Gly Asn Lys Val Val Asp Glu Cys
                140                 145                 150

tac atc ccc aac tac ctc ctg ccc aag act cag ccc gag ctg cag tgg 835
Tyr Ile Pro Asn Tyr Leu Leu Pro Lys Thr Gln Pro Glu Leu Gln Trp
            155                 160                 165

gcg tgg act aac atg gag gag tat ata agc gcc tgt ttg aac ctg gcc 883
Ala Trp Thr Asn Met Glu Glu Tyr Ile Ser Ala Cys Leu Asn Leu Ala
        170                 175                 180

gag cgc aaa cgg ctc gtg gcg cag cac ctg acc cac gtc agc cag acc 931
Glu Arg Lys Arg Leu Val Ala Gln His Leu Thr His Val Ser Gln Thr
    185                 190                 195

cag gag cag aac aag gag aat ctg aac ccc aat tct gac gcg cct gtc 979
Gln Glu Gln Asn Lys Glu Asn Leu Asn Pro Asn Ser Asp Ala Pro Val
200                 205                 210                 215

atc cgg tca aaa acc tcc gcg cgc tac atg gag ctg gtc ggg tgg ctg 1027
Ile Arg Ser Lys Thr Ser Ala Arg Tyr Met Glu Leu Val Gly Trp Leu
                220                 225                 230

gtg gac cgg ggc atc acc tcc gag aag cag tgg atc cag gag gac cag 1075
Val Asp Arg Gly Ile Thr Ser Glu Lys Gln Trp Ile Gln Glu Asp Gln
            235                 240                 245

gcc tcg tac atc tcc ttc aac gcc gct tcc aac tcg cgg tcc cag atc 1123
Ala Ser Tyr Ile Ser Phe Asn Ala Ala Ser Asn Ser Arg Ser Gln Ile
        250                 255                 260

aag gcc gct ctg gac aat gcc ggc aag atc atg gcg ctg acc aaa tcc 1171
Lys Ala Ala Leu Asp Asn Ala Gly Lys Ile Met Ala Leu Thr Lys Ser
    265                 270                 275

gcg ccc gac tac ctg gta ggc ccc gct ccg ccc gcg gac att aaa acc 1219
Ala Pro Asp Tyr Leu Val Gly Pro Ala Pro Pro Ala Asp Ile Lys Thr
280                 285                 290                 295

aac cgc atc tac cgc atc ctg gag ctg aac ggc tac gaa cct gcc tac 1267
Asn Arg Ile Tyr Arg Ile Leu Glu Leu Asn Gly Tyr Glu Pro Ala Tyr
                300                 305                 310

gcc ggc tcc gtc ttt ctc ggc tgg gcc cag aaa agg ttc ggg aag cgc 1315
Ala Gly Ser Val Phe Leu Gly Trp Ala Gln Lys Arg Phe Gly Lys Arg
            315                 320                 325

aac acc atc tgg ctg ttt ggg ccg gcc acc acg ggc aag acc aac atc 1363
Asn Thr Ile Trp Leu Phe Gly Pro Ala Thr Thr Gly Lys Thr Asn Ile
        330                 335                 340

gcg gaa gcc atc gcc cac gcc gtg ccc ttc tac ggc tgc gtc aac tgg 1411
Ala Glu Ala Ile Ala His Ala Val Pro Phe Tyr Gly Cys Val Asn Trp
    345                 350                 355

acc aat gag aac ttt ccc ttc aat gat tgc gtc gac aag atg gtg atc 1459
Thr Asn Glu Asn Phe Pro Phe Asn Asp Cys Val Asp Lys Met Val Ile
360                 365                 370                 375

tgg tgg gag gag ggc aag atg acg gcc aag gtc gtg gag tcc gcc aag 1507
Trp Trp Glu Glu Gly Lys Met Thr Ala Lys Val Val Glu Ser Ala Lys
                380                 385                 390

gcc att ctc ggc ggc agc aag gtg cgc gtg gac caa aag tgc aag tcg    1555
Ala Ile Leu Gly Gly Ser Lys Val Arg Val Asp Gln Lys Cys Lys Ser
            395                 400                 405

tcc gcc cag atc gac ccc acc ccc gtg atc gtc acc tcc aac acc aac    1603
Ser Ala Gln Ile Asp Pro Thr Pro Val Ile Val Thr Ser Asn Thr Asn
        410                 415                 420

atg tgc gcc gtg att gac ggg aac agc acc acc ttc gag cac cag cag    1651
Met Cys Ala Val Ile Asp Gly Asn Ser Thr Thr Phe Glu His Gln Gln
    425                 430                 435

ccg ttg cag gac cgg atg ttc aaa ttt gaa ctc acc cgc cgt ctg gag    1699
Pro Leu Gln Asp Arg Met Phe Lys Phe Glu Leu Thr Arg Arg Leu Glu
440                 445                 450                 455

cat gac ttt ggc aag gtg aca aag cag gaa gtc aaa gag ttc ttc cgc    1747
His Asp Phe Gly Lys Val Thr Lys Gln Glu Val Lys Glu Phe Phe Arg
                460                 465                 470

tgg gcg cag gat cac gtg acc gag gtg gcg cat gag ttc tac gtc aga    1795
Trp Ala Gln Asp His Val Thr Glu Val Ala His Glu Phe Tyr Val Arg
            475                 480                 485

aag ggt gga gcc aac aaa aga ccc gcc ccc gat gac gcg gat aaa agc    1843
Lys Gly Gly Ala Asn Lys Arg Pro Ala Pro Asp Asp Ala Asp Lys Ser
        490                 495                 500

gag ccc aag cgg gcc tgc ccc tca gtc gcg gat cca tcg acg tca gac    1891
Glu Pro Lys Arg Ala Cys Pro Ser Val Ala Asp Pro Ser Thr Ser Asp
    505                 510                 515

gcg gaa gga gct ccg gtg gac ttt gcc gac agg tac caa aac aaa tgt    1939
Ala Glu Gly Ala Pro Val Asp Phe Ala Asp Arg Tyr Gln Asn Lys Cys
520                 525                 530                 535

tct cgt cac gcg ggc atg ctt cag atg ctg ttt ccc tgc aag aca tgc    1987
Ser Arg His Ala Gly Met Leu Gln Met Leu Phe Pro Cys Lys Thr Cys
                540                 545                 550

gag aga atg aat cag aat ttc aac att tgc ttc acg cac ggg acg aga    2035
Glu Arg Met Asn Gln Asn Phe Asn Ile Cys Phe Thr His Gly Thr Arg
            555                 560                 565

gac tgt tca gag tgc ttc ccc ggc gtg tca gaa tct caa ccg gtc gtc    2083
Asp Cys Ser Glu Cys Phe Pro Gly Val Ser Glu Ser Gln Pro Val Val
        570                 575                 580

aga aag agg acg tat cgg aaa ctc tgt gcc att cat cat ctg ctg ggg 2131
Arg Lys Arg Thr Tyr Arg Lys Leu Cys Ala Ile His His Leu Leu Gly
    585                 590                 595

cgg gct ccc gag att gct tgc tcg gcc tgc gat ctg gtc aac gtg gac 2179
Arg Ala Pro Glu Ile Ala Cys Ser Ala Cys Asp Leu Val Asn Val Asp
600                 605                 610                 615

ctg gat gac tgt gtt tct gag caa taa atgacttaaa ccaggt atg gct gcc 2231
Leu Asp Asp Cys Val Ser Glu Gln Met Ala Ala
                620                 625

gat ggt tat ctt cca gat tgg ctc gag gac aac ctc tct gag ggc att 2279
Asp Gly Tyr Leu Pro Asp Trp Leu Glu Asp Asn Leu Ser Glu Gly Ile
            630                 635                 640

cgc gag tgg tgg gac ttg aaa cct gga gcc ccg aag ccc aaa gcc aac 2327
Arg Glu Trp Trp Asp Leu Lys Pro Gly Ala Pro Lys Pro Lys Ala Asn
        645                 650                 655

cag caa aag cag gac gac ggc cgg ggt ctg gtg ctt cct ggc tac aag 2375
Gln Gln Lys Gln Asp Asp Gly Arg Gly Leu Val Leu Pro Gly Tyr Lys
660 665 670 675

tac ctc gga ccc ttc aac gga ctc gac aag ggg gag ccc gtc aac gcg 2423
Tyr Leu Gly Pro Phe Asn Gly Leu Asp Lys Gly Glu Pro Val Asn Ala
680 685 690

gcg gac gca gcg gcc ctc gag cac gac aag gcc tac gac cag cag ctc 2471
Ala Asp Ala Ala Ala Leu Glu His Asp Lys Ala Tyr Asp Gln Gln Leu
                695                 700                 705

aaa gcg ggt gac aat ccg tac ctg cgg tat aac cac gcc gac gcc gag 2519
Lys Ala Gly Asp Asn Pro Tyr Leu Arg Tyr Asn His Ala Asp Ala Glu
            710                 715                 720

ttt cag gag cgt ctg caa gaa gat acg tct ttt ggg ggc aac ctc ggg 2567
Phe Gln Glu Arg Leu Gln Glu Asp Thr Ser Phe Gly Gly Asn Leu Gly
        725                 730                 735

cga gca gtc ttc cag gcc aag aag cgg gtt ctc gaa cct ctc ggt ctg 2615
Arg Ala Val Phe Gln Ala Lys Lys Arg Val Leu Glu Pro Leu Gly Leu
740 745 750 755

gtt gag gaa ggc gct aag acg gct cct gga aag aaa cgt ccg gta gag 2663
Val Glu Glu Gly Ala Lys Thr Ala Pro Gly Lys Lys Arg Pro Val Glu
760 765 770

cag tcg cca caa gag cca gac tcc tcc tcg ggc atc ggc aag aca ggc    2711
Gln Ser Pro Gln Glu Pro Asp Ser Ser Ser Gly Ile Gly Lys Thr Gly
            775                 780                 785

cag cag ccc gct aaa aag aga ctc aat ttt ggt cag act ggc gac tca    2759
Gln Gln Pro Ala Lys Lys Arg Leu Asn Phe Gly Gln Thr Gly Asp Ser
        790                 795                 800

gag tca gtc ccc gat cca caa cct ctc gga gaa cct cca gca acc ccc    2807
Glu Ser Val Pro Asp Pro Gln Pro Leu Gly Glu Pro Pro Ala Thr Pro
    805                 810                 815

gct gct gtg gga cct act aca atg gct tca ggc ggt ggc gca cca atg    2855
Ala Ala Val Gly Pro Thr Thr Met Ala Ser Gly Gly Gly Ala Pro Met
820                 825                 830                 835

gca gac aat aac gaa ggc gcc gac gga gtg ggt aat gcc tca gga aat    2903
Ala Asp Asn Asn Glu Gly Ala Asp Gly Val Gly Asn Ala Ser Gly Asn
                840                 845                 850

tgg cat tgc gat tcc aca tgg ctg ggc gac aga gtc atc acc acc agc    2951
Trp His Cys Asp Ser Thr Trp Leu Gly Asp Arg Val Ile Thr Thr Ser
            855                 860                 865

acc cgc acc tgg gcc ttg ccc acc tac aat aac cac ctc tac aag caa    2999
Thr Arg Thr Trp Ala Leu Pro Thr Tyr Asn Asn His Leu Tyr Lys Gln
        870                 875                 880

atc tcc agt gct tca acg ggg gcc agc aac gac aac cac tac ttc ggc    3047
Ile Ser Ser Ala Ser Thr Gly Ala Ser Asn Asp Asn His Tyr Phe Gly
    885                 890                 895

tac agc acc ccc tgg ggg tat ttt gat ttc aac aga ttc cac tgc cac    3095
Tyr Ser Thr Pro Trp Gly Tyr Phe Asp Phe Asn Arg Phe His Cys His
900                 905                 910                 915

ttt tca cca cgt gac tgg cag cga ctc atc aac aac aat tgg gga ttc    3143
Phe Ser Pro Arg Asp Trp Gln Arg Leu Ile Asn Asn Asn Trp Gly Phe
                920                 925                 930

cgg ccc aag aga ctc aac ttc aaa ctc ttc aac atc caa gtc aag gag    3191
Arg Pro Lys Arg Leu Asn Phe Lys Leu Phe Asn Ile Gln Val Lys Glu
            935                 940                 945

gtc acg acg aat gat ggc gtc aca acc atc gct aat aac ctt acc agc    3239
Val Thr Thr Asn Asp Gly Val Thr Thr Ile Ala Asn Asn Leu Thr Ser
        950                 955                 960

acg gtt caa gtc ttc tcg gac tcg gag tac cag ctt ccg tac gtc ctc 3287
Thr Val Gln Val Phe Ser Asp Ser Glu Tyr Gln Leu Pro Tyr Val Leu
        965                 970                 975

ggc tct gcg cac cag ggc tgc ctc cct ccg ttc ccg gcg gac gtg ttc 3335
Gly Ser Ala His Gln Gly Cys Leu Pro Pro Phe Pro Ala Asp Val Phe
980 985 990 995

atg att ccg caa tac ggc tac ctg acg ctc aac aat ggc agc caa gcc 3383
Met Ile Pro Gln Tyr Gly Tyr Leu Thr Leu Asn Asn Gly Ser Gln Ala
1000 1005 1010

gtg gga cgt tca tcc ttt tac tgc ctg gaa tat ttc cct tct cag atg 3431
Val Gly Arg Ser Ser Phe Tyr Cys Leu Glu Tyr Phe Pro Ser Gln Met
                1015                1020                1025

ctg aga acg ggc aac aac ttt acc ttc agc tac acc ttt gag gaa gtg 3479
Leu Arg Thr Gly Asn Asn Phe Thr Phe Ser Tyr Thr Phe Glu Glu Val
            1030                1035                1040

cct ttc cac agc agc tac gcg cac agc cag agc ctg gac cgg ctg atg 3527
Pro Phe His Ser Ser Tyr Ala His Ser Gln Ser Leu Asp Arg Leu Met
        1045                1050                1055

aat cct ctc atc gac caa tac ctg tat tac ctg aac aga act caa aat 3575
Asn Pro Leu Ile Asp Gln Tyr Leu Tyr Tyr Leu Asn Arg Thr Gln Asn
1060 1065 1070 1075

cag tcc gga agt gcc caa aac aag gac ttg ctg ttt agc cgt ggg tct 3623
Gln Ser Gly Ser Ala Gln Asn Lys Asp Leu Leu Phe Ser Arg Gly Ser
1080 1085 1090

cca gct ggc atg tct gtt cag ccc aaa aac tgg cta cct gga ccc tgt 3671
Pro Ala Gly Met Ser Val Gln Pro Lys Asn Trp Leu Pro Gly Pro Cys
                1095                1100                1105

tat cgg cag cag cgc gtt tct aaa aca aaa aca gac aac aac aac agc 3719
Tyr Arg Gln Gln Arg Val Ser Lys Thr Lys Thr Asp Asn Asn Asn Ser
            1110                1115                1120

aat ttt acc tgg act ggt gct tca aaa tat aac ctc aat ggg cgt gaa 3767
Asn Phe Thr Trp Thr Gly Ala Ser Lys Tyr Asn Leu Asn Gly Arg Glu
        1125                1130                1135

tcc atc atc aac cct ggc act gct atg gcc tca cac aaa gac gac gaa 3815
Ser Ile Ile Asn Pro Gly Thr Ala Met Ala Ser His Lys Asp Asp Glu
1140 1145 1150 1155

gac aag ttc ttt ccc atg agc ggt gtc atg att ttt gga aaa gag agc 3863
Asp Lys Phe Phe Pro Met Ser Gly Val Met Ile Phe Gly Lys Glu Ser
1160 1165 1170

gcc gga gct tca aac act gca ttg gac aat gtc atg att aca gac gaa 3911
Ala Gly Ala Ser Asn Thr Ala Leu Asp Asn Val Met Ile Thr Asp Glu
                1175                1180                1185

gag gaa att aaa gcc act aac cct gtg gcc acc gaa aga ttt ggg acc 3959
Glu Glu Ile Lys Ala Thr Asn Pro Val Ala Thr Glu Arg Phe Gly Thr
            1190                1195                1200

gtg gca gtc aat ttc cag agc agc agc aca gac cct gcg acc gga gat 4007
Val Ala Val Asn Phe Gln Ser Ser Ser Thr Asp Pro Ala Thr Gly Asp
        1205                1210                1215

gtg cat gct atg gga gca tta cct ggc atg gtg tgg caa gat aga gac 4055
Val His Ala Met Gly Ala Leu Pro Gly Met Val Trp Gln Asp Arg Asp
1220 1225 1230 1235

gtg tac ctg cag ggt ccc att tgg gcc aaa att cct cac aca gat gga 4103
Val Tyr Leu Gln Gly Pro Ile Trp Ala Lys Ile Pro His Thr Asp Gly
1240 1245 1250

cac ttt cac ccg tct cct ctt atg ggc ggc ttt gga ctc aag aac ccg 4151
His Phe His Pro Ser Pro Leu Met Gly Gly Phe Gly Leu Lys Asn Pro
                1255                1260                1265

cct cct cag atc ctc atc aaa aac acg cct gtt cct gcg aat cct ccg 4199
Pro Pro Gln Ile Leu Ile Lys Asn Thr Pro Val Pro Ala Asn Pro Pro
            1270                1275                1280

gcg gag ttt tca gct aca aag ttt gct tca ttc atc acc caa tac tcc 4247
Ala Glu Phe Ser Ala Thr Lys Phe Ala Ser Phe Ile Thr Gln Tyr Ser
        1285                1290                1295

aca gga caa gtg agt gtg gaa att gaa tgg gag ctg cag aaa gaa aac 4295
Thr Gly Gln Val Ser Val Glu Ile Glu Trp Glu Leu Gln Lys Glu Asn
1300 1305 1310 1315

agc aag cgc tgg aat ccc gaa gtg cag tac aca tcc aat tat gca aaa 4343
Ser Lys Arg Trp Asn Pro Glu Val Gln Tyr Thr Ser Asn Tyr Ala Lys
1320 1325 1330

tct gcc aac gtt gat ttt act gtg gac aac aat gga ctt tat act gag 4391
Ser Ala Asn Val Asp Phe Thr Val Asp Asn Asn Gly Leu Tyr Thr Glu
                1335                1340                1345

cct cgc ccc att ggc acc cgt tac ctt acc cgt ccc ctg taattacgtg 4440
Pro Arg Pro Ile Gly Thr Arg Tyr Leu Thr Arg Pro Leu
1350 1355 1360

ttaatcaata aaccggttga ttcgtttcag ttgaactttg gtctcctgtc cttcttatct 4500

tatcggttac catggttata gcttacacat taactgcttg gttgcgcttc gcgataaaag 4560

acttacgtca tcgggttacc cctagtgatg gagttgccca ctccctctct gcgcgctcgc 4620

tcgctcggtg gggcctgcgg accaaaggtc cgcagacggc agagctctgc tctgccggcc 4680

ccaccgagcg agcgagcgcg cagagaggga gtgggcaa 4718

<210> 2
<211> 623
<212> PRT
<213> AAV-1

<400> 2
Met Pro Gly Phe Tyr Glu Ile Val Ile Lys Val Pro Ser Asp Leu Asp
1               5                   10                  15

Glu His Leu Pro Gly Ile Ser Asp Ser Phe Val Ser Trp Val Ala Glu
            20                  25                  30

Lys Glu Trp Glu Leu Pro Pro Asp Ser Asp Met Asp Leu Asn Leu Ile
        35                  40                  45

Glu Gln Ala Pro Leu Thr Val Ala Glu Lys Leu Gln Arg Asp Phe Leu
    50                  55                  60

Val Gln Trp Arg Arg Val Ser Lys Ala Pro Glu Ala Leu Phe Phe Val
65                  70                  75                  80

Gln Phe Glu Lys Gly Glu Ser Tyr Phe His Leu His Ile Leu Val Glu
                85                  90                  95

Thr Thr Gly Val Lys Ser Met Val Leu Gly Arg Phe Leu Ser Gln Ile
            100                 105                 110

Arg Asp Lys Leu Val Gln Thr Ile Tyr Arg Gly Ile Glu Pro Thr Leu
        115                 120                 125

Pro Asn Trp Phe Ala Val Thr Lys Thr Arg Asn Gly Ala Gly Gly Gly
    130                 135                 140

Asn Lys Val Val Asp Glu Cys Tyr Ile Pro Asn Tyr Leu Leu Pro Lys
145                 150                 155                 160

Thr Gln Pro Glu Leu Gln Trp Ala Trp Thr Asn Met Glu Glu Tyr Ile
                165                 170                 175

Ser Ala Cys Leu Asn Leu Ala Glu Arg Lys Arg Leu Val Ala Gln His
            180                 185                 190

Leu Thr His Val Ser Gln Thr Gln Glu Gln Asn Lys Glu Asn Leu Asn
        195                 200                 205

Pro Asn Ser Asp Ala Pro Val Ile Arg Ser Lys Thr Ser Ala Arg Tyr
    210                 215                 220

Met Glu Leu Val Gly Trp Leu Val Asp Arg Gly Ile Thr Ser Glu Lys
225                 230                 235                 240

Gln Trp Ile Gln Glu Asp Gln Ala Ser Tyr Ile Ser Phe Asn Ala Ala
                245                 250                 255

Ser Asn Ser Arg Ser Gln Ile Lys Ala Ala Leu Asp Asn Ala Gly Lys
            260                 265                 270

Ile Met Ala Leu Thr Lys Ser Ala Pro Asp Tyr Leu Val Gly Pro Ala
        275                 280                 285

Pro Pro Ala Asp Ile Lys Thr Asn Arg Ile Tyr Arg Ile Leu Glu Leu
    290                 295                 300

Asn Gly Tyr Glu Pro Ala Tyr Ala Gly Ser Val Phe Leu Gly Trp Ala
305                 310                 315                 320

Gln Lys Arg Phe Gly Lys Arg Asn Thr Ile Trp Leu Phe Gly Pro Ala
                325                 330                 335

Thr Thr Gly Lys Thr Asn Ile Ala Glu Ala Ile Ala His Ala Val Pro
            340                 345                 350

Phe Tyr Gly Cys Val Asn Trp Thr Asn Glu Asn Phe Pro Phe Asn Asp
        355                 360                 365

Cys Val Asp Lys Met Val Ile Trp Trp Glu Glu Gly Lys Met Thr Ala
    370                 375                 380

Lys Val Val Glu Ser Ala Lys Ala Ile Leu Gly Gly Ser Lys Val Arg
385                 390                 395                 400

Val Asp Gln Lys Cys Lys Ser Ser Ala Gln Ile Asp Pro Thr Pro Val
                405                 410                 415

Ile Val Thr Ser Asn Thr Asn Met Cys Ala Val Ile Asp Gly Asn Ser
            420                 425                 430

Thr Thr Phe Glu His Gln Gln Pro Leu Gln Asp Arg Met Phe Lys Phe
        435                 440                 445

Glu Leu Thr Arg Arg Leu Glu His Asp Phe Gly Lys Val Thr Lys Gln
    450                 455                 460

Glu Val Lys Glu Phe Phe Arg Trp Ala Gln Asp His Val Thr Glu Val
465                 470                 475                 480

Ala His Glu Phe Tyr Val Arg Lys Gly Gly Ala Asn Lys Arg Pro Ala
                485                 490                 495

Pro Asp Asp Ala Asp Lys Ser Glu Pro Lys Arg Ala Cys Pro Ser Val
            500                 505                 510

Ala Asp Pro Ser Thr Ser Asp Ala Glu Gly Ala Pro Val Asp Phe Ala
        515                 520                 525

Asp Arg Tyr Gln Asn Lys Cys Ser Arg His Ala Gly Met Leu Gln Met
    530                 535                 540

Leu Phe Pro Cys Lys Thr Cys Glu Arg Met Asn Gln Asn Phe Asn Ile
545                 550                 555                 560

Cys Phe Thr His Gly Thr Arg Asp Cys Ser Glu Cys Phe Pro Gly Val
                565                 570                 575

Ser Glu Ser Gln Pro Val Val Arg Lys Arg Thr Tyr Arg Lys Leu Cys
            580                 585                 590

Ala Ile His His Leu Leu Gly Arg Ala Pro Glu Ile Ala Cys Ser Ala
        595                 600                 605

Cys Asp Leu Val Asn Val Asp Leu Asp Asp Cys Val Ser Glu Gln
    610                 615                 620

<210> 3
<211> 736
<212> PRT
<213> AAV-1

<400> 3
Met Ala Ala Asp Gly Tyr Leu Pro Asp Trp Leu Glu Asp Asn Leu Ser
1               5                   10                  15

Glu Gly Ile Arg Glu Trp Trp Asp Leu Lys Pro Gly Ala Pro Lys Pro
            20                  25                  30

Lys Ala Asn Gln Gln Lys Gln Asp Asp Gly Arg Gly Leu Val Leu Pro
        35                  40                  45

Gly Tyr Lys Tyr Leu Gly Pro Phe Asn Gly Leu Asp Lys Gly Glu Pro
    50                  55                  60

Val Asn Ala Ala Asp Ala Ala Ala Leu Glu His Asp Lys Ala Tyr Asp
65                  70                  75                  80

Gln Gln Leu Lys Ala Gly Asp Asn Pro Tyr Leu Arg Tyr Asn His Ala
                85                  90                  95

Asp Ala Glu Phe Gln Glu Arg Leu Gln Glu Asp Thr Ser Phe Gly Gly
            100                 105                 110

Asn Leu Gly Arg Ala Val Phe Gln Ala Lys Lys Arg Val Leu Glu Pro
        115                 120                 125

Leu Gly Leu Val Glu Glu Gly Ala Lys Thr Ala Pro Gly Lys Lys Arg
    130                 135                 140

Pro Val Glu Gln Ser Pro Gln Glu Pro Asp Ser Ser Ser Gly Ile Gly
145                 150                 155                 160

Lys Thr Gly Gln Gln Pro Ala Lys Lys Arg Leu Asn Phe Gly Gln Thr
                165                 170                 175

Gly Asp Ser Glu Ser Val Pro Asp Pro Gln Pro Leu Gly Glu Pro Pro
            180                 185                 190

Ala Thr Pro Ala Ala Val Gly Pro Thr Thr Met Ala Ser Gly Gly Gly
        195                 200                 205

Ala Pro Met Ala Asp Asn Asn Glu Gly Ala Asp Gly Val Gly Asn Ala
    210                 215                 220

Ser Gly Asn Trp His Cys Asp Ser Thr Trp Leu Gly Asp Arg Val Ile
225                 230                 235                 240

Thr Thr Ser Thr Arg Thr Trp Ala Leu Pro Thr Tyr Asn Asn His Leu

245                 250                 255

Tyr Lys Gln Ile Ser Ser Ala Ser Thr Gly Ala Ser Asn Asp Asn His
            260                 265                 270

Tyr Phe Gly Tyr Ser Thr Pro Trp Gly Tyr Phe Asp Phe Asn Arg Phe
        275                 280                 285

His Cys His Phe Ser Pro Arg Asp Trp Gln Arg Leu Ile Asn Asn Asn
    290                 295                 300

Trp Gly Phe Arg Pro Lys Arg Leu Asn Phe Lys Leu Phe Asn Ile Gln
305                 310                 315                 320

Val Lys Glu Val Thr Thr Asn Asp Gly Val Thr Thr Ile Ala Asn Asn
                325                 330                 335

Leu Thr Ser Thr Val Gln Val Phe Ser Asp Ser Glu Tyr Gln Leu Pro
            340                 345                 350

Tyr Val Leu Gly Ser Ala His Gln Gly Cys Leu Pro Pro Phe Pro Ala
        355                 360                 365

Asp Val Phe Met Ile Pro Gln Tyr Gly Tyr Leu Thr Leu Asn Asn Gly
    370                 375                 380

Ser Gln Ala Val Gly Arg Ser Ser Phe Tyr Cys Leu Glu Tyr Phe Pro
385                 390                 395                 400

Ser Gln Met Leu Arg Thr Gly Asn Asn Phe Thr Phe Ser Tyr Thr Phe
                405                 410                 415

Glu Glu Val Pro Phe His Ser Ser Tyr Ala His Ser Gln Ser Leu Asp
            420                 425                 430

Arg Leu Met Asn Pro Leu Ile Asp Gln Tyr Leu Tyr Tyr Leu Asn Arg
        435                 440                 445

Thr Gln Asn Gln Ser Gly Ser Ala Gln Asn Lys Asp Leu Leu Phe Ser
    450                 455                 460

Arg Gly Ser Pro Ala Gly Met Ser Val Gln Pro Lys Asn Trp Leu Pro
465                 470                 475                 480

Gly Pro Cys Tyr Arg Gln Gln Arg Val Ser Lys Thr Lys Thr Asp Asn
                485                 490                 495

Asn Asn Ser Asn Phe Thr Trp Thr Gly Ala Ser Lys Tyr Asn Leu Asn

            500                 505                 510

Gly Arg Glu Ser Ile Ile Asn Pro Gly Thr Ala Met Ala Ser His Lys
        515                 520                 525

Asp Asp Glu Asp Lys Phe Phe Pro Met Ser Gly Val Met Ile Phe Gly
    530                 535                 540

Lys Glu Ser Ala Gly Ala Ser Asn Thr Ala Leu Asp Asn Val Met Ile
545                 550                 555                 560

Thr Asp Glu Glu Glu Ile Lys Ala Thr Asn Pro Val Ala Thr Glu Arg
                565                 570                 575

Phe Gly Thr Val Ala Val Asn Phe Gln Ser Ser Ser Thr Asp Pro Ala
            580                 585                 590

Thr Gly Asp Val His Ala Met Gly Ala Leu Pro Gly Met Val Trp Gln
        595                 600                 605

Asp Arg Asp Val Tyr Leu Gln Gly Pro Ile Trp Ala Lys Ile Pro His
    610                 615                 620

Thr Asp Gly His Phe His Pro Ser Pro Leu Met Gly Gly Phe Gly Leu
625                 630                 635                 640

Lys Asn Pro Pro Pro Gln Ile Leu Ile Lys Asn Thr Pro Val Pro Ala
                645                 650                 655

Asn Pro Pro Ala Glu Phe Ser Ala Thr Lys Phe Ala Ser Phe Ile Thr
            660                 665                 670

Gln Tyr Ser Thr Gly Gln Val Ser Val Glu Ile Glu Trp Glu Leu Gln
        675                 680                 685

Lys Glu Asn Ser Lys Arg Trp Asn Pro Glu Val Gln Tyr Thr Ser Asn
    690                 695                 700

Tyr Ala Lys Ser Ala Asn Val Asp Phe Thr Val Asp Asn Asn Gly Leu
705                 710                 715                 720

Tyr Thr Glu Pro Arg Pro Ile Gly Thr Arg Tyr Leu Thr Arg Pro Leu
                725                 730                 735

<210> 4
<211> 1872
<212> DNA

<213> AAV-1

<220>
<221> CDS
<222> (1)..(1869)

<400> 4
atg ccg ggc ttc tac gag atc gtg atc aag gtg ccg agc gac ctg gac     48
Met Pro Gly Phe Tyr Glu Ile Val Ile Lys Val Pro Ser Asp Leu Asp
1               5                   10                  15

gag cac ctg ccg ggc att tct gac tcg ttt gtg agc tgg gtg gcc gag     96
Glu His Leu Pro Gly Ile Ser Asp Ser Phe Val Ser Trp Val Ala Glu
            20                  25                  30

aag gaa tgg gag ctg ccc ccg gat tct gac atg gat ctg aat ctg att     144
Lys Glu Trp Glu Leu Pro Pro Asp Ser Asp Met Asp Leu Asn Leu Ile
        35                  40                  45

gag cag gca ccc ctg acc gtg gcc gag aag ctg cag cgc gac ttc ctg     192
Glu Gln Ala Pro Leu Thr Val Ala Glu Lys Leu Gln Arg Asp Phe Leu
    50                  55                  60

gtc caa tgg cgc cgc gtg agt aag gcc ccg gag gcc ctc ttc ttt gtt     240
Val Gln Trp Arg Arg Val Ser Lys Ala Pro Glu Ala Leu Phe Phe Val
65                  70                  75                  80

cag ttc gag aag ggc gag tcc tac ttc cac ctc cat att ctg gtg gag     288
Gln Phe Glu Lys Gly Glu Ser Tyr Phe His Leu His Ile Leu Val Glu
                85                  90                  95

acc acg ggg gtc aaa tcc atg gtg ctg ggc cgc ttc ctg agt cag att     336
Thr Thr Gly Val Lys Ser Met Val Leu Gly Arg Phe Leu Ser Gln Ile
            100                 105                 110

agg gac aag ctg gtg cag acc atc tac cgc ggg atc gag ccg acc ctg     384
Arg Asp Lys Leu Val Gln Thr Ile Tyr Arg Gly Ile Glu Pro Thr Leu
        115                 120                 125

ccc aac tgg ttc gcg gtg acc aag acg cgt aat ggc gcc gga ggg ggg     432
Pro Asn Trp Phe Ala Val Thr Lys Thr Arg Asn Gly Ala Gly Gly Gly
    130                 135                 140

aac aag gtg gtg gac gag tgc tac atc ccc aac tac ctc ctg ccc aag     480
Asn Lys Val Val Asp Glu Cys Tyr Ile Pro Asn Tyr Leu Leu Pro Lys
145                 150                 155                 160

act cag ccc gag ctg cag tgg gcg tgg act aac atg gag gag tat ata     528

Thr Gln Pro Glu Leu Gln Trp Ala Trp Thr Asn Met Glu Glu Tyr Ile
                165                 170                 175

agc gcc tgt ttg aac ctg gcc gag cgc aaa cgg ctc gtg gcg cag cac     576
Ser Ala Cys Leu Asn Leu Ala Glu Arg Lys Arg Leu Val Ala Gln His
            180                 185                 190

ctg acc cac gtc agc cag acc cag gag cag aac aag gag aat ctg aac     624
Leu Thr His Val Ser Gln Thr Gln Glu Gln Asn Lys Glu Asn Leu Asn
        195                 200                 205

ccc aat tct gac gcg cct gtc atc cgg tca aaa acc tcc gcg cgc tac     672
Pro Asn Ser Asp Ala Pro Val Ile Arg Ser Lys Thr Ser Ala Arg Tyr
    210                 215                 220

atg gag ctg gtc ggg tgg ctg gtg gac cgg ggc atc acc tcc gag aag     720
Met Glu Leu Val Gly Trp Leu Val Asp Arg Gly Ile Thr Ser Glu Lys
225                 230                 235                 240

cag tgg atc cag gag gac cag gcc tcg tac atc tcc ttc aac gcc gct     768
Gln Trp Ile Gln Glu Asp Gln Ala Ser Tyr Ile Ser Phe Asn Ala Ala
                245                 250                 255

tcc aac tcg cgg tcc cag atc aag gcc gct ctg gac aat gcc ggc aag     816
Ser Asn Ser Arg Ser Gln Ile Lys Ala Ala Leu Asp Asn Ala Gly Lys
            260                 265                 270

atc atg gcg ctg acc aaa tcc gcg ccc gac tac ctg gta ggc ccc gct     864
Ile Met Ala Leu Thr Lys Ser Ala Pro Asp Tyr Leu Val Gly Pro Ala
        275                 280                 285

ccg ccc gcg gac att aaa acc aac cgc atc tac cgc atc ctg gag ctg     912
Pro Pro Ala Asp Ile Lys Thr Asn Arg Ile Tyr Arg Ile Leu Glu Leu
    290                 295                 300

aac ggc tac gaa cct gcc tac gcc ggc tcc gtc ttt ctc ggc tgg gcc     960
Asn Gly Tyr Glu Pro Ala Tyr Ala Gly Ser Val Phe Leu Gly Trp Ala
305                 310                 315                 320

cag aaa agg ttc ggg aag cgc aac acc atc tgg ctg ttt ggg ccg gcc     1008
Gln Lys Arg Phe Gly Lys Arg Asn Thr Ile Trp Leu Phe Gly Pro Ala
                325                 330                 335

acc acg ggc aag acc aac atc gcg gaa gcc atc gcc cac gcc gtg ccc     1056
Thr Thr Gly Lys Thr Asn Ile Ala Glu Ala Ile Ala His Ala Val Pro
            340                 345                 350

ttc tac ggc tgc gtc aac tgg acc aat gag aac ttt ccc ttc aat gat     1104

Phe Tyr Gly Cys Val Asn Trp Thr Asn Glu Asn Phe Pro Phe Asn Asp
        355                 360                 365

tgc gtc gac aag atg gtg atc tgg tgg gag gag ggc aag atg acg gcc 1152
Cys Val Asp Lys Met Val Ile Trp Trp Glu Glu Gly Lys Met Thr Ala
    370                 375                 380

aag gtc gtg gag tcc gcc aag gcc att ctc ggc ggc agc aag gtg cgc 1200
Lys Val Val Glu Ser Ala Lys Ala Ile Leu Gly Gly Ser Lys Val Arg
385                 390                 395                 400

gtg gac caa aag tgc aag tcg tcc gcc cag atc gac ccc acc ccc gtg 1248
Val Asp Gln Lys Cys Lys Ser Ser Ala Gln Ile Asp Pro Thr Pro Val
                405                 410                 415

atc gtc acc tcc aac acc aac atg tgc gcc gtg att gac ggg aac agc 1296
Ile Val Thr Ser Asn Thr Asn Met Cys Ala Val Ile Asp Gly Asn Ser
            420                 425                 430

acc acc ttc gag cac cag cag ccg ttg cag gac cgg atg ttc aaa ttt 1344
Thr Thr Phe Glu His Gln Gln Pro Leu Gln Asp Arg Met Phe Lys Phe
        435                 440                 445

gaa ctc acc cgc cgt ctg gag cat gac ttt ggc aag gtg aca aag cag 1392
Glu Leu Thr Arg Arg Leu Glu His Asp Phe Gly Lys Val Thr Lys Gln
    450                 455                 460

gaa gtc aaa gag ttc ttc cgc tgg gcg cag gat cac gtg acc gag gtg 1440
Glu Val Lys Glu Phe Phe Arg Trp Ala Gln Asp His Val Thr Glu Val
465                 470                 475                 480

gcg cat gag ttc tac gtc aga aag ggt gga gcc aac aaa aga ccc gcc 1488
Ala His Glu Phe Tyr Val Arg Lys Gly Gly Ala Asn Lys Arg Pro Ala
                485                 490                 495

ccc gat gac gcg gat aaa agc gag ccc aag cgg gcc tgc ccc tca gtc 1536
Pro Asp Asp Ala Asp Lys Ser Glu Pro Lys Arg Ala Cys Pro Ser Val
            500                 505                 510

gcg gat cca tcg acg tca gac gcg gaa gga gct ccg gtg gac ttt gcc 1584
Ala Asp Pro Ser Thr Ser Asp Ala Glu Gly Ala Pro Val Asp Phe Ala
        515                 520                 525

gac agg tac caa aac aaa tgt tct cgt cac gcg ggc atg ctt cag atg 1632
Asp Arg Tyr Gln Asn Lys Cys Ser Arg His Ala Gly Met Leu Gln Met
    530                 535                 540

ctg ttt ccc tgc aag aca tgc gag aga atg aat cag aat ttc aac att 1680

Leu Phe Pro Cys Lys Thr Cys Glu Arg Met Asn Gln Asn Phe Asn Ile
545                 550                 555                 560

tgc ttc acg cac ggg acg aga gac tgt tca gag tgc ttc ccc ggc gtg    1728
Cys Phe Thr His Gly Thr Arg Asp Cys Ser Glu Cys Phe Pro Gly Val
                565                 570                 575

tca gaa tct caa ccg gtc gtc aga aag agg acg tat cgg aaa ctc tgt    1776
Ser Glu Ser Gln Pro Val Val Arg Lys Arg Thr Tyr Arg Lys Leu Cys
            580                 585                 590

gcc att cat cat ctg ctg ggg cgg gct ccc gag att gct tgc tcg gcc    1824
Ala Ile His His Leu Leu Gly Arg Ala Pro Glu Ile Ala Cys Ser Ala
        595                 600                 605

tgc gat ctg gtc aac gtg gac ctg gat gac tgt gtt tct gag caa taa    1872
Cys Asp Leu Val Asn Val Asp Leu Asp Asp Cys Val Ser Glu Gln
    610                 615                 620

<210> 5
<211> 623
<212> PRT
<213> AAV-1

<400> 5
Met Pro Gly Phe Tyr Glu Ile Val Ile Lys Val Pro Ser Asp Leu Asp
  1               5                  10                  15

Glu His Leu Pro Gly Ile Ser Asp Ser Phe Val Ser Trp Val Ala Glu
             20                  25                  30

Lys Glu Trp Glu Leu Pro Pro Asp Ser Asp Met Asp Leu Asn Leu Ile
         35                  40                  45

Glu Gln Ala Pro Leu Thr Val Ala Glu Lys Leu Gln Arg Asp Phe Leu
     50                  55                  60

Val Gln Trp Arg Arg Val Ser Lys Ala Pro Glu Ala Leu Phe Phe Val
 65                  70                  75                  80

Gln Phe Glu Lys Gly Glu Ser Tyr Phe His Leu His Ile Leu Val Glu
                 85                  90                  95

Thr Thr Gly Val Lys Ser Met Val Leu Gly Arg Phe Leu Ser Gln Ile
            100                 105                 110

Arg Asp Lys Leu Val Gln Thr Ile Tyr Arg Gly Ile Glu Pro Thr Leu

115                 120                 125

Pro Asn Trp Phe Ala Val Thr Lys Thr Arg Asn Gly Ala Gly Gly Gly
    130                 135                 140

Asn Lys Val Val Asp Glu Cys Tyr Ile Pro Asn Tyr Leu Leu Pro Lys
145                 150                 155                 160

Thr Gln Pro Glu Leu Gln Trp Ala Trp Thr Asn Met Glu Glu Tyr Ile
                165                 170                 175

Ser Ala Cys Leu Asn Leu Ala Glu Arg Lys Arg Leu Val Ala Gln His
            180                 185                 190

Leu Thr His Val Ser Gln Thr Gln Glu Gln Asn Lys Glu Asn Leu Asn
        195                 200                 205

Pro Asn Ser Asp Ala Pro Val Ile Arg Ser Lys Thr Ser Ala Arg Tyr
    210                 215                 220

Met Glu Leu Val Gly Trp Leu Val Asp Arg Gly Ile Thr Ser Glu Lys
225                 230                 235                 240

Gln Trp Ile Gln Glu Asp Gln Ala Ser Tyr Ile Ser Phe Asn Ala Ala
                245                 250                 255

Ser Asn Ser Arg Ser Gln Ile Lys Ala Ala Leu Asp Asn Ala Gly Lys
            260                 265                 270

Ile Met Ala Leu Thr Lys Ser Ala Pro Asp Tyr Leu Val Gly Pro Ala
        275                 280                 285

Pro Pro Ala Asp Ile Lys Thr Asn Arg Ile Tyr Arg Ile Leu Glu Leu
    290                 295                 300

Asn Gly Tyr Glu Pro Ala Tyr Ala Gly Ser Val Phe Leu Gly Trp Ala
305                 310                 315                 320

Gln Lys Arg Phe Gly Lys Arg Asn Thr Ile Trp Leu Phe Gly Pro Ala
                325                 330                 335

Thr Thr Gly Lys Thr Asn Ile Ala Glu Ala Ile Ala His Ala Val Pro
            340                 345                 350

Phe Tyr Gly Cys Val Asn Trp Thr Asn Glu Asn Phe Pro Phe Asn Asp
        355                 360                 365

Cys Val Asp Lys Met Val Ile Trp Trp Glu Glu Gly Lys Met Thr Ala

370                 375                 380

Lys Val Val Glu Ser Ala Lys Ala Ile Leu Gly Gly Ser Lys Val Arg
385                 390                 395                 400

Val Asp Gln Lys Cys Lys Ser Ser Ala Gln Ile Asp Pro Thr Pro Val
                405                 410                 415

Ile Val Thr Ser Asn Thr Asn Met Cys Ala Val Ile Asp Gly Asn Ser
            420                 425                 430

Thr Thr Phe Glu His Gln Gln Pro Leu Gln Asp Arg Met Phe Lys Phe
        435                 440                 445

Glu Leu Thr Arg Arg Leu Glu His Asp Phe Gly Lys Val Thr Lys Gln
    450                 455                 460

Glu Val Lys Glu Phe Phe Arg Trp Ala Gln Asp His Val Thr Glu Val
465                 470                 475                 480

Ala His Glu Phe Tyr Val Arg Lys Gly Gly Ala Asn Lys Arg Pro Ala
                485                 490                 495

Pro Asp Asp Ala Asp Lys Ser Glu Pro Lys Arg Ala Cys Pro Ser Val
            500                 505                 510

Ala Asp Pro Ser Thr Ser Asp Ala Glu Gly Ala Pro Val Asp Phe Ala
        515                 520                 525

Asp Arg Tyr Gln Asn Lys Cys Ser Arg His Ala Gly Met Leu Gln Met
    530                 535                 540

Leu Phe Pro Cys Lys Thr Cys Glu Arg Met Asn Gln Asn Phe Asn Ile
545                 550                 555                 560

Cys Phe Thr His Gly Thr Arg Asp Cys Ser Glu Cys Phe Pro Gly Val
                565                 570                 575

Ser Glu Ser Gln Pro Val Val Arg Lys Arg Thr Tyr Arg Lys Leu Cys
            580                 585                 590

Ala Ile His His Leu Leu Gly Arg Ala Pro Glu Ile Ala Cys Ser Ala
        595                 600                 605

Cys Asp Leu Val Asn Val Asp Leu Asp Asp Cys Val Ser Glu Gln
    610                 615                 620

<210> 6
<211> 1641
<212> DNA
<213> AAV-1

<220>
<221> CDS
<222> (1)..(1638)

<400> 6
atg ccg ggc ttc tac gag atc gtg atc aag gtg ccg agc gac ctg gac 48
Met Pro Gly Phe Tyr Glu Ile Val Ile Lys Val Pro Ser Asp Leu Asp
1               5                   10                  15

gag cac ctg ccg ggc att tct gac tcg ttt gtg agc tgg gtg gcc gag 96
Glu His Leu Pro Gly Ile Ser Asp Ser Phe Val Ser Trp Val Ala Glu
            20                  25                  30

aag gaa tgg gag ctg ccc ccg gat tct gac atg gat ctg aat ctg att 144
Lys Glu Trp Glu Leu Pro Pro Asp Ser Asp Met Asp Leu Asn Leu Ile
        35                  40                  45

gag cag gca ccc ctg acc gtg gcc gag aag ctg cag cgc gac ttc ctg 192
Glu Gln Ala Pro Leu Thr Val Ala Glu Lys Leu Gln Arg Asp Phe Leu
    50                  55                  60

gtc caa tgg cgc cgc gtg agt aag gcc ccg gag gcc ctc ttc ttt gtt 240
Val Gln Trp Arg Arg Val Ser Lys Ala Pro Glu Ala Leu Phe Phe Val
65                  70                  75                  80

cag ttc gag aag ggc gag tcc tac ttc cac ctc cat att ctg gtg gag 288
Gln Phe Glu Lys Gly Glu Ser Tyr Phe His Leu His Ile Leu Val Glu
                85                  90                  95

acc acg ggg gtc aaa tcc atg gtg ctg ggc cgc ttc ctg agt cag att 336
Thr Thr Gly Val Lys Ser Met Val Leu Gly Arg Phe Leu Ser Gln Ile
            100                 105                 110

agg gac aag ctg gtg cag acc atc tac cgc ggg atc gag ccg acc ctg 384
Arg Asp Lys Leu Val Gln Thr Ile Tyr Arg Gly Ile Glu Pro Thr Leu
        115                 120                 125

ccc aac tgg ttc gcg gtg acc aag acg cgt aat ggc gcc gga ggg ggg 432
Pro Asn Trp Phe Ala Val Thr Lys Thr Arg Asn Gly Ala Gly Gly Gly
    130                 135                 140

aac aag gtg gtg gac gag tgc tac atc ccc aac tac ctc ctg ccc aag 480
Asn Lys Val Val Asp Glu Cys Tyr Ile Pro Asn Tyr Leu Leu Pro Lys

145                 150                 155                 160

act cag ccc gag ctg cag tgg gcg tgg act aac atg gag gag tat ata     528
Thr Gln Pro Glu Leu Gln Trp Ala Trp Thr Asn Met Glu Glu Tyr Ile
                165                 170                 175

agc gcc tgt ttg aac ctg gcc gag cgc aaa cgg ctc gtg gcg cag cac     576
Ser Ala Cys Leu Asn Leu Ala Glu Arg Lys Arg Leu Val Ala Gln His
            180                 185                 190

ctg acc cac gtc agc cag acc cag gag cag aac aag gag aat ctg aac     624
Leu Thr His Val Ser Gln Thr Gln Glu Gln Asn Lys Glu Asn Leu Asn
        195                 200                 205

ccc aat tct gac gcg cct gtc atc cgg tca aaa acc tcc gcg cgc tac     672
Pro Asn Ser Asp Ala Pro Val Ile Arg Ser Lys Thr Ser Ala Arg Tyr
    210                 215                 220

atg gag ctg gtc ggg tgg ctg gtg gac cgg ggc atc acc tcc gag aag     720
Met Glu Leu Val Gly Trp Leu Val Asp Arg Gly Ile Thr Ser Glu Lys
225                 230                 235                 240

cag tgg atc cag gag gac cag gcc tcg tac atc tcc ttc aac gcc gct     768
Gln Trp Ile Gln Glu Asp Gln Ala Ser Tyr Ile Ser Phe Asn Ala Ala
                245                 250                 255

tcc aac tcg cgg tcc cag atc aag gcc gct ctg gac aat gcc ggc aag     816
Ser Asn Ser Arg Ser Gln Ile Lys Ala Ala Leu Asp Asn Ala Gly Lys
            260                 265                 270

atc atg gcg ctg acc aaa tcc gcg ccc gac tac ctg gta ggc ccc gct     864
Ile Met Ala Leu Thr Lys Ser Ala Pro Asp Tyr Leu Val Gly Pro Ala
        275                 280                 285

ccg ccc gcg gac att aaa acc aac cgc atc tac cgc atc ctg gag ctg     912
Pro Pro Ala Asp Ile Lys Thr Asn Arg Ile Tyr Arg Ile Leu Glu Leu
    290                 295                 300

aac ggc tac gaa cct gcc tac gcc ggc tcc gtc ttt ctc ggc tgg gcc     960
Asn Gly Tyr Glu Pro Ala Tyr Ala Gly Ser Val Phe Leu Gly Trp Ala
305                 310                 315                 320

cag aaa agg ttc ggg aag cgc aac acc atc tgg ctg ttt ggg ccg gcc     1008
Gln Lys Arg Phe Gly Lys Arg Asn Thr Ile Trp Leu Phe Gly Pro Ala
                325                 330                 335

acc acg ggc aag acc aac atc gcg gaa gcc atc gcc cac gcc gtg ccc     1056
Thr Thr Gly Lys Thr Asn Ile Ala Glu Ala Ile Ala His Ala Val Pro

            340                 345                 350

ttc tac ggc tgc gtc aac tgg acc aat gag aac ttt ccc ttc aat gat 1104
Phe Tyr Gly Cys Val Asn Trp Thr Asn Glu Asn Phe Pro Phe Asn Asp
        355                 360                 365

tgc gtc gac aag atg gtg atc tgg tgg gag gag ggc aag atg acg gcc 1152
Cys Val Asp Lys Met Val Ile Trp Trp Glu Glu Gly Lys Met Thr Ala
    370                 375                 380

aag gtc gtg gag tcc gcc aag gcc att ctc ggc ggc agc aag gtg cgc 1200
Lys Val Val Glu Ser Ala Lys Ala Ile Leu Gly Gly Ser Lys Val Arg
385                 390                 395                 400

gtg gac caa aag tgc aag tcg tcc gcc cag atc gac ccc acc ccc gtg 1248
Val Asp Gln Lys Cys Lys Ser Ser Ala Gln Ile Asp Pro Thr Pro Val
                405                 410                 415

atc gtc acc tcc aac acc aac atg tgc gcc gtg att gac ggg aac agc 1296
Ile Val Thr Ser Asn Thr Asn Met Cys Ala Val Ile Asp Gly Asn Ser
            420                 425                 430

acc acc ttc gag cac cag cag ccg ttg cag gac cgg atg ttc aaa ttt 1344
Thr Thr Phe Glu His Gln Gln Pro Leu Gln Asp Arg Met Phe Lys Phe
        435                 440                 445

gaa ctc acc cgc cgt ctg gag cat gac ttt ggc aag gtg aca aag cag 1392
Glu Leu Thr Arg Arg Leu Glu His Asp Phe Gly Lys Val Thr Lys Gln
    450                 455                 460

gaa gtc aaa gag ttc ttc cgc tgg gcg cag gat cac gtg acc gag gtg 1440
Glu Val Lys Glu Phe Phe Arg Trp Ala Gln Asp His Val Thr Glu Val
465                 470                 475                 480

gcg cat gag ttc tac gtc aga aag ggt gga gcc aac aaa aga ccc gcc 1488
Ala His Glu Phe Tyr Val Arg Lys Gly Gly Ala Asn Lys Arg Pro Ala
                485                 490                 495

ccc gat gac gcg gat aaa agc gag ccc aag cgg gcc tgc ccc tca gtc 1536
Pro Asp Asp Ala Asp Lys Ser Glu Pro Lys Arg Ala Cys Pro Ser Val
            500                 505                 510

gcg gat cca tcg acg tca gac gcg gaa gga gct ccg gtg gac ttt gcc 1584
Ala Asp Pro Ser Thr Ser Asp Ala Glu Gly Ala Pro Val Asp Phe Ala
        515                 520                 525

gac agg tat ggc tgc cga tgg tta tct tcc aga ttg gct cga gga caa 1632
Asp Arg Tyr Gly Cys Arg Trp Leu Ser Ser Arg Leu Ala Arg Gly Gln

530                 535                 540

cct ctc tga                                                          1641
Pro Leu
545

<210> 7
<211> 546
<212> PRT
<213> AAV-1

<400> 7
Met Pro Gly Phe Tyr Glu Ile Val Ile Lys Val Pro Ser Asp Leu Asp
1               5                   10                  15

Glu His Leu Pro Gly Ile Ser Asp Ser Phe Val Ser Trp Val Ala Glu
            20                  25                  30

Lys Glu Trp Glu Leu Pro Pro Asp Ser Asp Met Asp Leu Asn Leu Ile
        35                  40                  45

Glu Gln Ala Pro Leu Thr Val Ala Glu Lys Leu Gln Arg Asp Phe Leu
    50                  55                  60

Val Gln Trp Arg Arg Val Ser Lys Ala Pro Glu Ala Leu Phe Phe Val
65                  70                  75                  80

Gln Phe Glu Lys Gly Glu Ser Tyr Phe His Leu His Ile Leu Val Glu
                85                  90                  95

Thr Thr Gly Val Lys Ser Met Val Leu Gly Arg Phe Leu Ser Gln Ile
            100                 105                 110

Arg Asp Lys Leu Val Gln Thr Ile Tyr Arg Gly Ile Glu Pro Thr Leu
        115                 120                 125

Pro Asn Trp Phe Ala Val Thr Lys Thr Arg Asn Gly Ala Gly Gly Gly
    130                 135                 140

Asn Lys Val Val Asp Glu Cys Tyr Ile Pro Asn Tyr Leu Leu Pro Lys
145                 150                 155                 160

Thr Gln Pro Glu Leu Gln Trp Ala Trp Thr Asn Met Glu Glu Tyr Ile
                165                 170                 175

Ser Ala Cys Leu Asn Leu Ala Glu Arg Lys Arg Leu Val Ala Gln His
            180                 185                 190

Leu Thr His Val Ser Gln Thr Gln Glu Gln Asn Lys Glu Asn Leu Asn
        195                 200                 205

Pro Asn Ser Asp Ala Pro Val Ile Arg Ser Lys Thr Ser Ala Arg Tyr
    210                 215                 220

Met Glu Leu Val Gly Trp Leu Val Asp Arg Gly Ile Thr Ser Glu Lys
225                 230                 235                 240

Gln Trp Ile Gln Glu Asp Gln Ala Ser Tyr Ile Ser Phe Asn Ala Ala
                245                 250                 255

Ser Asn Ser Arg Ser Gln Ile Lys Ala Ala Leu Asp Asn Ala Gly Lys
            260                 265                 270

Ile Met Ala Leu Thr Lys Ser Ala Pro Asp Tyr Leu Val Gly Pro Ala
        275                 280                 285

Pro Pro Ala Asp Ile Lys Thr Asn Arg Ile Tyr Arg Ile Leu Glu Leu
    290                 295                 300

Asn Gly Tyr Glu Pro Ala Tyr Ala Gly Ser Val Phe Leu Gly Trp Ala
305                 310                 315                 320

Gln Lys Arg Phe Gly Lys Arg Asn Thr Ile Trp Leu Phe Gly Pro Ala
                325                 330                 335

Thr Thr Gly Lys Thr Asn Ile Ala Glu Ala Ile Ala His Ala Val Pro
            340                 345                 350

Phe Tyr Gly Cys Val Asn Trp Thr Asn Glu Asn Phe Pro Phe Asn Asp
        355                 360                 365

Cys Val Asp Lys Met Val Ile Trp Trp Glu Glu Gly Lys Met Thr Ala
    370                 375                 380

Lys Val Val Glu Ser Ala Lys Ala Ile Leu Gly Gly Ser Lys Val Arg
385                 390                 395                 400

Val Asp Gln Lys Cys Lys Ser Ser Ala Gln Ile Asp Pro Thr Pro Val
                405                 410                 415

Ile Val Thr Ser Asn Thr Asn Met Cys Ala Val Ile Asp Gly Asn Ser
            420                 425                 430

Thr Thr Phe Glu His Gln Gln Pro Leu Gln Asp Arg Met Phe Lys Phe
        435                 440                 445

Glu Leu Thr Arg Arg Leu Glu His Asp Phe Gly Lys Val Thr Lys Gln
    450                 455                 460

Glu Val Lys Glu Phe Phe Arg Trp Ala Gln Asp His Val Thr Glu Val
465                 470                 475                 480

Ala His Glu Phe Tyr Val Arg Lys Gly Gly Ala Asn Lys Arg Pro Ala
                485                 490                 495

Pro Asp Asp Ala Asp Lys Ser Glu Pro Lys Arg Ala Cys Pro Ser Val
            500                 505                 510

Ala Asp Pro Ser Thr Ser Asp Ala Glu Gly Ala Pro Val Asp Phe Ala
        515                 520                 525

Asp Arg Tyr Gly Cys Arg Trp Leu Ser Ser Arg Leu Ala Arg Gly Gln
    530                 535                 540

Pro Leu
545

<210> 8
<211> 1200
<212> DNA
<213> AAV-1

<220>
<221> CDS
<222> (1)..(1197)

<400> 8
atg gag ctg gtc ggg tgg ctg gtg gac cgg ggc atc acc tcc gag aag      48
Met Glu Leu Val Gly Trp Leu Val Asp Arg Gly Ile Thr Ser Glu Lys
  1               5                  10                  15

cag tgg atc cag gag gac cag gcc tcg tac atc tcc ttc aac gcc gct      96
Gln Trp Ile Gln Glu Asp Gln Ala Ser Tyr Ile Ser Phe Asn Ala Ala
             20                  25                  30

tcc aac tcg cgg tcc cag atc aag gcc gct ctg gac aat gcc ggc aag     144
Ser Asn Ser Arg Ser Gln Ile Lys Ala Ala Leu Asp Asn Ala Gly Lys
         35                  40                  45

atc atg gcg ctg acc aaa tcc gcg ccc gac tac ctg gta ggc ccc gct     192
Ile Met Ala Leu Thr Lys Ser Ala Pro Asp Tyr Leu Val Gly Pro Ala
     50                  55                  60

ccg ccc gcg gac att aaa acc aac cgc atc tac cgc atc ctg gag ctg     240
Pro Pro Ala Asp Ile Lys Thr Asn Arg Ile Tyr Arg Ile Leu Glu Leu
65                  70                  75                  80

aac ggc tac gaa cct gcc tac gcc ggc tcc gtc ttt ctc ggc tgg gcc     288
Asn Gly Tyr Glu Pro Ala Tyr Ala Gly Ser Val Phe Leu Gly Trp Ala
                85                  90                  95

cag aaa agg ttc ggg aag cgc aac acc atc tgg ctg ttt ggg ccg gcc     336
Gln Lys Arg Phe Gly Lys Arg Asn Thr Ile Trp Leu Phe Gly Pro Ala
            100                 105                 110

acc acg ggc aag acc aac atc gcg gaa gcc atc gcc cac gcc gtg ccc     384
Thr Thr Gly Lys Thr Asn Ile Ala Glu Ala Ile Ala His Ala Val Pro
        115                 120                 125

ttc tac ggc tgc gtc aac tgg acc aat gag aac ttt ccc ttc aat gat     432
Phe Tyr Gly Cys Val Asn Trp Thr Asn Glu Asn Phe Pro Phe Asn Asp
    130                 135                 140

tgc gtc gac aag atg gtg atc tgg tgg gag gag ggc aag atg acg gcc     480
Cys Val Asp Lys Met Val Ile Trp Trp Glu Glu Gly Lys Met Thr Ala
145                 150                 155                 160

aag gtc gtg gag tcc gcc aag gcc att ctc ggc ggc agc aag gtg cgc     528
Lys Val Val Glu Ser Ala Lys Ala Ile Leu Gly Gly Ser Lys Val Arg
                165                 170                 175

gtg gac caa aag tgc aag tcg tcc gcc cag atc gac ccc acc ccc gtg     576
Val Asp Gln Lys Cys Lys Ser Ser Ala Gln Ile Asp Pro Thr Pro Val
            180                 185                 190

atc gtc acc tcc aac acc aac atg tgc gcc gtg att gac ggg aac agc     624
Ile Val Thr Ser Asn Thr Asn Met Cys Ala Val Ile Asp Gly Asn Ser
        195                 200                 205

acc acc ttc gag cac cag cag ccg ttg cag gac cgg atg ttc aaa ttt     672
Thr Thr Phe Glu His Gln Gln Pro Leu Gln Asp Arg Met Phe Lys Phe
    210                 215                 220

gaa ctc acc cgc cgt ctg gag cat gac ttt ggc aag gtg aca aag cag     720
Glu Leu Thr Arg Arg Leu Glu His Asp Phe Gly Lys Val Thr Lys Gln
225                 230                 235                 240

gaa gtc aaa gag ttc ttc cgc tgg gcg cag gat cac gtg acc gag gtg     768
Glu Val Lys Glu Phe Phe Arg Trp Ala Gln Asp His Val Thr Glu Val
                245                 250                 255

gcg cat gag ttc tac gtc aga aag ggt gga gcc aac aaa aga ccc gcc    816
Ala His Glu Phe Tyr Val Arg Lys Gly Gly Ala Asn Lys Arg Pro Ala
            260                 265                 270

ccc gat gac gcg gat aaa agc gag ccc aag cgg gcc tgc ccc tca gtc    864
Pro Asp Asp Ala Asp Lys Ser Glu Pro Lys Arg Ala Cys Pro Ser Val
        275                 280                 285

gcg gat cca tcg acg tca gac gcg gaa gga gct ccg gtg gac ttt gcc    912
Ala Asp Pro Ser Thr Ser Asp Ala Glu Gly Ala Pro Val Asp Phe Ala
    290                 295                 300

gac agg tac caa aac aaa tgt tct cgt cac gcg ggc atg ctt cag atg    960
Asp Arg Tyr Gln Asn Lys Cys Ser Arg His Ala Gly Met Leu Gln Met
305                 310                 315                 320

ctg ttt ccc tgc aag aca tgc gag aga atg aat cag aat ttc aac att    1008
Leu Phe Pro Cys Lys Thr Cys Glu Arg Met Asn Gln Asn Phe Asn Ile
                325                 330                 335

tgc ttc acg cac ggg acg aga gac tgt tca gag tgc ttc ccc ggc gtg    1056
Cys Phe Thr His Gly Thr Arg Asp Cys Ser Glu Cys Phe Pro Gly Val
            340                 345                 350

tca gaa tct caa ccg gtc gtc aga aag agg acg tat cgg aaa ctc tgt    1104
Ser Glu Ser Gln Pro Val Val Arg Lys Arg Thr Tyr Arg Lys Leu Cys
        355                 360                 365

gcc att cat cat ctg ctg ggg cgg gct ccc gag att gct tgc tcg gcc    1152
Ala Ile His His Leu Leu Gly Arg Ala Pro Glu Ile Ala Cys Ser Ala
    370                 375                 380

tgc gat ctg gtc aac gtg gac ctg gat gac tgt gtt tct gag caa taa    1200
Cys Asp Leu Val Asn Val Asp Leu Asp Asp Cys Val Ser Glu Gln
385                 390                 395

<210> 9
<211> 399
<212> PRT
<213> AAV-1

<400> 9
Met Glu Leu Val Gly Trp Leu Val Asp Arg Gly Ile Thr Ser Glu Lys
1               5                   10                  15

Gln Trp Ile Gln Glu Asp Gln Ala Ser Tyr Ile Ser Phe Asn Ala Ala

            20                  25                  30

Ser Asn Ser Arg Ser Gln Ile Lys Ala Ala Leu Asp Asn Ala Gly Lys
        35                  40                  45

Ile Met Ala Leu Thr Lys Ser Ala Pro Asp Tyr Leu Val Gly Pro Ala
    50                  55                  60

Pro Pro Ala Asp Ile Lys Thr Asn Arg Ile Tyr Arg Ile Leu Glu Leu
65                  70                  75                  80

Asn Gly Tyr Glu Pro Ala Tyr Ala Gly Ser Val Phe Leu Gly Trp Ala
                85                  90                  95

Gln Lys Arg Phe Gly Lys Arg Asn Thr Ile Trp Leu Phe Gly Pro Ala
            100                 105                 110

Thr Thr Gly Lys Thr Asn Ile Ala Glu Ala Ile Ala His Ala Val Pro
        115                 120                 125

Phe Tyr Gly Cys Val Asn Trp Thr Asn Glu Asn Phe Pro Phe Asn Asp
    130                 135                 140

Cys Val Asp Lys Met Val Ile Trp Trp Glu Glu Gly Lys Met Thr Ala
145                 150                 155                 160

Lys Val Val Glu Ser Ala Lys Ala Ile Leu Gly Gly Ser Lys Val Arg
                165                 170                 175

Val Asp Gln Lys Cys Lys Ser Ser Ala Gln Ile Asp Pro Thr Pro Val
            180                 185                 190

Ile Val Thr Ser Asn Thr Asn Met Cys Ala Val Ile Asp Gly Asn Ser
        195                 200                 205

Thr Thr Phe Glu His Gln Gln Pro Leu Gln Asp Arg Met Phe Lys Phe
    210                 215                 220

Glu Leu Thr Arg Arg Leu Glu His Asp Phe Gly Lys Val Thr Lys Gln
225                 230                 235                 240

Glu Val Lys Glu Phe Phe Arg Trp Ala Gln Asp His Val Thr Glu Val
                245                 250                 255

Ala His Glu Phe Tyr Val Arg Lys Gly Gly Ala Asn Lys Arg Pro Ala
            260                 265                 270

Pro Asp Asp Ala Asp Lys Ser Glu Pro Lys Arg Ala Cys Pro Ser Val

275                 280                 285

Ala Asp Pro Ser Thr Ser Asp Ala Glu Gly Ala Pro Val Asp Phe Ala
    290                 295                 300

Asp Arg Tyr Gln Asn Lys Cys Ser Arg His Ala Gly Met Leu Gln Met
305                 310                 315                 320

Leu Phe Pro Cys Lys Thr Cys Glu Arg Met Asn Gln Asn Phe Asn Ile
                325                 330                 335

Cys Phe Thr His Gly Thr Arg Asp Cys Ser Glu Cys Phe Pro Gly Val
            340                 345                 350

Ser Glu Ser Gln Pro Val Val Arg Lys Arg Thr Tyr Arg Lys Leu Cys
        355                 360                 365

Ala Ile His His Leu Leu Gly Arg Ala Pro Glu Ile Ala Cys Ser Ala
    370                 375                 380

Cys Asp Leu Val Asn Val Asp Leu Asp Asp Cys Val Ser Glu Gln
385                 390                 395


<210> 10
<211> 969
<212> DNA
<213> AAV-1

<220>
<221> CDS
<222> (1)..(966)

<220>
<221> misc_feature
<222> (943)..(944)
<223> minor splice site

<400> 10
atg gag ctg gtc ggg tgg ctg gtg gac cgg ggc atc acc tcc gag aag      48
Met Glu Leu Val Gly Trp Leu Val Asp Arg Gly Ile Thr Ser Glu Lys
1               5                   10                  15

cag tgg atc cag gag gac cag gcc tcg tac atc tcc ttc aac gcc gct      96
Gln Trp Ile Gln Glu Asp Gln Ala Ser Tyr Ile Ser Phe Asn Ala Ala
            20                  25                  30

tcc aac tcg cgg tcc cag atc aag gcc gct ctg gac aat gcc ggc aag      144

Ser Asn Ser Arg Ser Gln Ile Lys Ala Ala Leu Asp Asn Ala Gly Lys
        35                  40                  45

atc atg gcg ctg acc aaa tcc gcg ccc gac tac ctg gta ggc ccc gct 192
Ile Met Ala Leu Thr Lys Ser Ala Pro Asp Tyr Leu Val Gly Pro Ala
    50                  55                  60

ccg ccc gcg gac att aaa acc aac cgc atc tac cgc atc ctg gag ctg 240
Pro Pro Ala Asp Ile Lys Thr Asn Arg Ile Tyr Arg Ile Leu Glu Leu
65                  70                  75                  80

aac ggc tac gaa cct gcc tac gcc ggc tcc gtc ttt ctc ggc tgg gcc 288
Asn Gly Tyr Glu Pro Ala Tyr Ala Gly Ser Val Phe Leu Gly Trp Ala
                85                  90                  95

cag aaa agg ttc ggg aag cgc aac acc atc tgg ctg ttt ggg ccg gcc 336
Gln Lys Arg Phe Gly Lys Arg Asn Thr Ile Trp Leu Phe Gly Pro Ala
            100                 105                 110

acc acg ggc aag acc aac atc gcg gaa gcc atc gcc cac gcc gtg ccc 384
Thr Thr Gly Lys Thr Asn Ile Ala Glu Ala Ile Ala His Ala Val Pro
        115                 120                 125

ttc tac ggc tgc gtc aac tgg acc aat gag aac ttt ccc ttc aat gat 432
Phe Tyr Gly Cys Val Asn Trp Thr Asn Glu Asn Phe Pro Phe Asn Asp
    130                 135                 140

tgc gtc gac aag atg gtg atc tgg tgg gag gag ggc aag atg acg gcc 480
Cys Val Asp Lys Met Val Ile Trp Trp Glu Glu Gly Lys Met Thr Ala
145                 150                 155                 160

aag gtc gtg gag tcc gcc aag gcc att ctc ggc ggc agc aag gtg cgc 528
Lys Val Val Glu Ser Ala Lys Ala Ile Leu Gly Gly Ser Lys Val Arg
                165                 170                 175

gtg gac caa aag tgc aag tcg tcc gcc cag atc gac ccc acc ccc gtg 576
Val Asp Gln Lys Cys Lys Ser Ser Ala Gln Ile Asp Pro Thr Pro Val
            180                 185                 190

atc gtc acc tcc aac acc aac atg tgc gcc gtg att gac ggg aac agc 624
Ile Val Thr Ser Asn Thr Asn Met Cys Ala Val Ile Asp Gly Asn Ser
        195                 200                 205

acc acc ttc gag cac cag cag ccg ttg cag gac cgg atg ttc aaa ttt 672
Thr Thr Phe Glu His Gln Gln Pro Leu Gln Asp Arg Met Phe Lys Phe
    210                 215                 220

gaa ctc acc cgc cgt ctg gag cat gac ttt ggc aag gtg aca aag cag 720

Glu Leu Thr Arg Arg Leu Glu His Asp Phe Gly Lys Val Thr Lys Gln
225                 230                 235                 240

gaa gtc aaa gag ttc ttc cgc tgg gcg cag gat cac gtg acc gag gtg     768
Glu Val Lys Glu Phe Phe Arg Trp Ala Gln Asp His Val Thr Glu Val
                245                 250                 255

gcg cat gag ttc tac gtc aga aag ggt gga gcc aac aaa aga ccc gcc     816
Ala His Glu Phe Tyr Val Arg Lys Gly Gly Ala Asn Lys Arg Pro Ala
            260                 265                 270

ccc gat gac gcg gat aaa agc gag ccc aag cgg gcc tgc ccc tca gtc     864
Pro Asp Asp Ala Asp Lys Ser Glu Pro Lys Arg Ala Cys Pro Ser Val
        275                 280                 285

gcg gat cca tcg acg tca gac gcg gaa gga gct ccg gtg gac ttt gcc     912
Ala Asp Pro Ser Thr Ser Asp Ala Glu Gly Ala Pro Val Asp Phe Ala
    290                 295                 300

gac agg tat ggc tgc cga tgg tta tct tcc aga ttg gct cga gga caa     960
Asp Arg Tyr Gly Cys Arg Trp Leu Ser Ser Arg Leu Ala Arg Gly Gln
305                 310                 315                 320

cct ctc tga                                                         969
Pro Leu

<210> 11
<211> 322
<212> PRT
<213> AAV-1

<400> 11
Met Glu Leu Val Gly Trp Leu Val Asp Arg Gly Ile Thr Ser Glu Lys
1               5                   10                  15

Gln Trp Ile Gln Glu Asp Gln Ala Ser Tyr Ile Ser Phe Asn Ala Ala
            20                  25                  30

Ser Asn Ser Arg Ser Gln Ile Lys Ala Ala Leu Asp Asn Ala Gly Lys
        35                  40                  45

Ile Met Ala Leu Thr Lys Ser Ala Pro Asp Tyr Leu Val Gly Pro Ala
    50                  55                  60

Pro Pro Ala Asp Ile Lys Thr Asn Arg Ile Tyr Arg Ile Leu Glu Leu
65                  70                  75                  80

Asn Gly Tyr Glu Pro Ala Tyr Ala Gly Ser Val Phe Leu Gly Trp Ala
                85                  90                  95

Gln Lys Arg Phe Gly Lys Arg Asn Thr Ile Trp Leu Phe Gly Pro Ala
            100                 105                 110

Thr Thr Gly Lys Thr Asn Ile Ala Glu Ala Ile Ala His Ala Val Pro
        115                 120                 125

Phe Tyr Gly Cys Val Asn Trp Thr Asn Glu Asn Phe Pro Phe Asn Asp
    130                 135                 140

Cys Val Asp Lys Met Val Ile Trp Trp Glu Glu Gly Lys Met Thr Ala
145                 150                 155                 160

Lys Val Val Glu Ser Ala Lys Ala Ile Leu Gly Gly Ser Lys Val Arg
                165                 170                 175

Val Asp Gln Lys Cys Lys Ser Ser Ala Gln Ile Asp Pro Thr Pro Val
            180                 185                 190

Ile Val Thr Ser Asn Thr Asn Met Cys Ala Val Ile Asp Gly Asn Ser
        195                 200                 205

Thr Thr Phe Glu His Gln Gln Pro Leu Gln Asp Arg Met Phe Lys Phe
    210                 215                 220

Glu Leu Thr Arg Arg Leu Glu His Asp Phe Gly Lys Val Thr Lys Gln
225                 230                 235                 240

Glu Val Lys Glu Phe Phe Arg Trp Ala Gln Asp His Val Thr Glu Val
                245                 250                 255

Ala His Glu Phe Tyr Val Arg Lys Gly Gly Ala Asn Lys Arg Pro Ala
            260                 265                 270

Pro Asp Asp Ala Asp Lys Ser Glu Pro Lys Arg Ala Cys Pro Ser Val
        275                 280                 285

Ala Asp Pro Ser Thr Ser Asp Ala Glu Gly Ala Pro Val Asp Phe Ala
    290                 295                 300

Asp Arg Tyr Gly Cys Arg Trp Leu Ser Ser Arg Leu Ala Arg Gly Gln
305                 310                 315                 320

Pro Leu

<210> 12
<211> 2211
<212> DNA
<213> AAV-1

<220>
<221> CDS
<222> (1)..(2208)

<400> 12
atg gct gcc gat ggt tat ctt cca gat tgg ctc gag gac aac ctc tct 48
Met Ala Ala Asp Gly Tyr Leu Pro Asp Trp Leu Glu Asp Asn Leu Ser
1               5                   10                  15

gag ggc att cgc gag tgg tgg gac ttg aaa cct gga gcc ccg aag ccc 96
Glu Gly Ile Arg Glu Trp Trp Asp Leu Lys Pro Gly Ala Pro Lys Pro
            20                  25                  30

aaa gcc aac cag caa aag cag gac gac ggc cgg ggt ctg gtg ctt cct 144
Lys Ala Asn Gln Gln Lys Gln Asp Asp Gly Arg Gly Leu Val Leu Pro
        35                  40                  45

ggc tac aag tac ctc gga ccc ttc aac gga ctc gac aag ggg gag ccc 192
Gly Tyr Lys Tyr Leu Gly Pro Phe Asn Gly Leu Asp Lys Gly Glu Pro
    50                  55                  60

gtc aac gcg gcg gac gca gcg gcc ctc gag cac gac aag gcc tac gac 240
Val Asn Ala Ala Asp Ala Ala Ala Leu Glu His Asp Lys Ala Tyr Asp
65                  70                  75                  80

cag cag ctc aaa gcg ggt gac aat ccg tac ctg cgg tat aac cac gcc 288
Gln Gln Leu Lys Ala Gly Asp Asn Pro Tyr Leu Arg Tyr Asn His Ala
                85                  90                  95

gac gcc gag ttt cag gag cgt ctg caa gaa gat acg tct ttt ggg ggc 336
Asp Ala Glu Phe Gln Glu Arg Leu Gln Glu Asp Thr Ser Phe Gly Gly
            100                 105                 110

aac ctc ggg cga gca gtc ttc cag gcc aag aag cgg gtt ctc gaa cct 384
Asn Leu Gly Arg Ala Val Phe Gln Ala Lys Lys Arg Val Leu Glu Pro
        115                 120                 125

ctc ggt ctg gtt gag gaa ggc gct aag acg gct cct gga aag aaa cgt 432
Leu Gly Leu Val Glu Glu Gly Ala Lys Thr Ala Pro Gly Lys Lys Arg
    130                 135                 140

ccg gta gag cag tcg cca caa gag cca gac tcc tcc tcg ggc atc ggc 480

Pro Val Glu Gln Ser Pro Gln Glu Pro Asp Ser Ser Ser Gly Ile Gly
145                 150                 155                 160

aag aca ggc cag cag ccc gct aaa aag aga ctc aat ttt ggt cag act     528
Lys Thr Gly Gln Gln Pro Ala Lys Lys Arg Leu Asn Phe Gly Gln Thr
                165                 170                 175

ggc gac tca gag tca gtc ccc gat cca caa cct ctc gga gaa cct cca     576
Gly Asp Ser Glu Ser Val Pro Asp Pro Gln Pro Leu Gly Glu Pro Pro
            180                 185                 190

gca acc ccc gct gct gtg gga cct act aca atg gct tca ggc ggt ggc     624
Ala Thr Pro Ala Ala Val Gly Pro Thr Thr Met Ala Ser Gly Gly Gly
        195                 200                 205

gca cca atg gca gac aat aac gaa ggc gcc gac gga gtg ggt aat gcc     672
Ala Pro Met Ala Asp Asn Asn Glu Gly Ala Asp Gly Val Gly Asn Ala
    210                 215                 220

tca gga aat tgg cat tgc gat tcc aca tgg ctg ggc gac aga gtc atc     720
Ser Gly Asn Trp His Cys Asp Ser Thr Trp Leu Gly Asp Arg Val Ile
225                 230                 235                 240

acc acc agc acc cgc acc tgg gcc ttg ccc acc tac aat aac cac ctc     768
Thr Thr Ser Thr Arg Thr Trp Ala Leu Pro Thr Tyr Asn Asn His Leu
                245                 250                 255

tac aag caa atc tcc agt gct tca acg ggg gcc agc aac gac aac cac     816
Tyr Lys Gln Ile Ser Ser Ala Ser Thr Gly Ala Ser Asn Asp Asn His
            260                 265                 270

tac ttc ggc tac agc acc ccc tgg ggg tat ttt gat ttc aac aga ttc     864
Tyr Phe Gly Tyr Ser Thr Pro Trp Gly Tyr Phe Asp Phe Asn Arg Phe
        275                 280                 285

cac tgc cac ttt tca cca cgt gac tgg cag cga ctc atc aac aac aat     912
His Cys His Phe Ser Pro Arg Asp Trp Gln Arg Leu Ile Asn Asn Asn
    290                 295                 300

tgg gga ttc cgg ccc aag aga ctc aac ttc aaa ctc ttc aac atc caa     960
Trp Gly Phe Arg Pro Lys Arg Leu Asn Phe Lys Leu Phe Asn Ile Gln
305                 310                 315                 320

gtc aag gag gtc acg acg aat gat ggc gtc aca acc atc gct aat aac     1008
Val Lys Glu Val Thr Thr Asn Asp Gly Val Thr Thr Ile Ala Asn Asn
                325                 330                 335

ctt acc agc acg gtt caa gtc ttc tcg gac tcg gag tac cag ctt ccg     1056

Leu Thr Ser Thr Val Gln Val Phe Ser Asp Ser Glu Tyr Gln Leu Pro
            340                 345                 350

tac gtc ctc ggc tct gcg cac cag ggc tgc ctc cct ccg ttc ccg gcg 1104
Tyr Val Leu Gly Ser Ala His Gln Gly Cys Leu Pro Pro Phe Pro Ala
        355                 360                 365

gac gtg ttc atg att ccg caa tac ggc tac ctg acg ctc aac aat ggc 1152
Asp Val Phe Met Ile Pro Gln Tyr Gly Tyr Leu Thr Leu Asn Asn Gly
    370                 375                 380

agc caa gcc gtg gga cgt tca tcc ttt tac tgc ctg gaa tat ttc cct 1200
Ser Gln Ala Val Gly Arg Ser Ser Phe Tyr Cys Leu Glu Tyr Phe Pro
385                 390                 395                 400

tct cag atg ctg aga acg ggc aac aac ttt acc ttc agc tac acc ttt 1248
Ser Gln Met Leu Arg Thr Gly Asn Asn Phe Thr Phe Ser Tyr Thr Phe
                405                 410                 415

gag gaa gtg cct ttc cac agc agc tac gcg cac agc cag agc ctg gac 1296
Glu Glu Val Pro Phe His Ser Ser Tyr Ala His Ser Gln Ser Leu Asp
            420                 425                 430

cgg ctg atg aat cct ctc atc gac caa tac ctg tat tac ctg aac aga 1344
Arg Leu Met Asn Pro Leu Ile Asp Gln Tyr Leu Tyr Tyr Leu Asn Arg
        435                 440                 445

act caa aat cag tcc gga agt gcc caa aac aag gac ttg ctg ttt agc 1392
Thr Gln Asn Gln Ser Gly Ser Ala Gln Asn Lys Asp Leu Leu Phe Ser
    450                 455                 460

cgt ggg tct cca gct ggc atg tct gtt cag ccc aaa aac tgg cta cct 1440
Arg Gly Ser Pro Ala Gly Met Ser Val Gln Pro Lys Asn Trp Leu Pro
465                 470                 475                 480

gga ccc tgt tat cgg cag cag cgc gtt tct aaa aca aaa aca gac aac 1488
Gly Pro Cys Tyr Arg Gln Gln Arg Val Ser Lys Thr Lys Thr Asp Asn
                485                 490                 495

aac aac agc aat ttt acc tgg act ggt gct tca aaa tat aac ctc aat 1536
Asn Asn Ser Asn Phe Thr Trp Thr Gly Ala Ser Lys Tyr Asn Leu Asn
            500                 505                 510

ggg cgt gaa tcc atc atc aac cct ggc act gct atg gcc tca cac aaa 1584
Gly Arg Glu Ser Ile Ile Asn Pro Gly Thr Ala Met Ala Ser His Lys
        515                 520                 525

gac gac gaa gac aag ttc ttt ccc atg agc ggt gtc atg att ttt gga 1632

Asp Asp Glu Asp Lys Phe Phe Pro Met Ser Gly Val Met Ile Phe Gly
    530                 535                 540

aaa gag agc gcc gga gct tca aac act gca ttg gac aat gtc atg att 1680
Lys Glu Ser Ala Gly Ala Ser Asn Thr Ala Leu Asp Asn Val Met Ile
545                 550                 555                 560

aca gac gaa gag gaa att aaa gcc act aac cct gtg gcc acc gaa aga 1728
Thr Asp Glu Glu Glu Ile Lys Ala Thr Asn Pro Val Ala Thr Glu Arg
                565                 570                 575

ttt ggg acc gtg gca gtc aat ttc cag agc agc agc aca gac cct gcg 1776
Phe Gly Thr Val Ala Val Asn Phe Gln Ser Ser Ser Thr Asp Pro Ala
            580                 585                 590

acc gga gat gtg cat gct atg gga gca tta cct ggc atg gtg tgg caa 1824
Thr Gly Asp Val His Ala Met Gly Ala Leu Pro Gly Met Val Trp Gln
        595                 600                 605

gat aga gac gtg tac ctg cag ggt ccc att tgg gcc aaa att cct cac 1872
Asp Arg Asp Val Tyr Leu Gln Gly Pro Ile Trp Ala Lys Ile Pro His
    610                 615                 620

aca gat gga cac ttt cac ccg tct cct ctt atg ggc ggc ttt gga ctc 1920
Thr Asp Gly His Phe His Pro Ser Pro Leu Met Gly Gly Phe Gly Leu
625                 630                 635                 640

aag aac ccg cct cct cag atc ctc atc aaa aac acg cct gtt cct gcg 1968
Lys Asn Pro Pro Pro Gln Ile Leu Ile Lys Asn Thr Pro Val Pro Ala
                645                 650                 655

aat cct ccg gcg gag ttt tca gct aca aag ttt gct tca ttc atc acc 2016
Asn Pro Pro Ala Glu Phe Ser Ala Thr Lys Phe Ala Ser Phe Ile Thr
            660                 665                 670

caa tac tcc aca gga caa gtg agt gtg gaa att gaa tgg gag ctg cag 2064
Gln Tyr Ser Thr Gly Gln Val Ser Val Glu Ile Glu Trp Glu Leu Gln
        675                 680                 685

aaa gaa aac agc aag cgc tgg aat ccc gaa gtg cag tac aca tcc aat 2112
Lys Glu Asn Ser Lys Arg Trp Asn Pro Glu Val Gln Tyr Thr Ser Asn
    690                 695                 700

tat gca aaa tct gcc aac gtt gat ttt act gtg gac aac aat gga ctt 2160
Tyr Ala Lys Ser Ala Asn Val Asp Phe Thr Val Asp Asn Asn Gly Leu
705                 710                 715                 720

tat act gag cct cgc ccc att ggc acc cgt tac ctt acc cgt ccc ctg 2208

Tyr Thr Glu Pro Arg Pro Ile Gly Thr Arg Tyr Leu Thr Arg Pro Leu
                725                 730                 735

taa                                                                 2211

<210> 13
<211> 736
<212> PRT
<213> AAV-1

<400> 13
Met Ala Ala Asp Gly Tyr Leu Pro Asp Trp Leu Glu Asp Asn Leu Ser
  1               5                  10                  15

Glu Gly Ile Arg Glu Trp Trp Asp Leu Lys Pro Gly Ala Pro Lys Pro
             20                  25                  30

Lys Ala Asn Gln Gln Lys Gln Asp Asp Gly Arg Gly Leu Val Leu Pro
         35                  40                  45

Gly Tyr Lys Tyr Leu Gly Pro Phe Asn Gly Leu Asp Lys Gly Glu Pro
     50                  55                  60

Val Asn Ala Ala Asp Ala Ala Ala Leu Glu His Asp Lys Ala Tyr Asp
 65                  70                  75                  80

Gln Gln Leu Lys Ala Gly Asp Asn Pro Tyr Leu Arg Tyr Asn His Ala
                 85                  90                  95

Asp Ala Glu Phe Gln Glu Arg Leu Gln Glu Asp Thr Ser Phe Gly Gly
            100                 105                 110

Asn Leu Gly Arg Ala Val Phe Gln Ala Lys Lys Arg Val Leu Glu Pro
        115                 120                 125

Leu Gly Leu Val Glu Glu Gly Ala Lys Thr Ala Pro Gly Lys Lys Arg
    130                 135                 140

Pro Val Glu Gln Ser Pro Gln Glu Pro Asp Ser Ser Ser Gly Ile Gly
145                 150                 155                 160

Lys Thr Gly Gln Gln Pro Ala Lys Lys Arg Leu Asn Phe Gly Gln Thr
                165                 170                 175

Gly Asp Ser Glu Ser Val Pro Asp Pro Gln Pro Leu Gly Glu Pro Pro
            180                 185                 190

Ala Thr Pro Ala Ala Val Gly Pro Thr Thr Met Ala Ser Gly Gly Gly
        195                 200                 205

Ala Pro Met Ala Asp Asn Asn Glu Gly Ala Asp Gly Val Gly Asn Ala
    210                 215                 220

Ser Gly Asn Trp His Cys Asp Ser Thr Trp Leu Gly Asp Arg Val Ile
225                 230                 235                 240

Thr Thr Ser Thr Arg Thr Trp Ala Leu Pro Thr Tyr Asn Asn His Leu
                245                 250                 255

Tyr Lys Gln Ile Ser Ser Ala Ser Thr Gly Ala Ser Asn Asp Asn His
            260                 265                 270

Tyr Phe Gly Tyr Ser Thr Pro Trp Gly Tyr Phe Asp Phe Asn Arg Phe
        275                 280                 285

His Cys His Phe Ser Pro Arg Asp Trp Gln Arg Leu Ile Asn Asn Asn
    290                 295                 300

Trp Gly Phe Arg Pro Lys Arg Leu Asn Phe Lys Leu Phe Asn Ile Gln
305                 310                 315                 320

Val Lys Glu Val Thr Thr Asn Asp Gly Val Thr Thr Ile Ala Asn Asn
                325                 330                 335

Leu Thr Ser Thr Val Gln Val Phe Ser Asp Ser Glu Tyr Gln Leu Pro
            340                 345                 350

Tyr Val Leu Gly Ser Ala His Gln Gly Cys Leu Pro Pro Phe Pro Ala
        355                 360                 365

Asp Val Phe Met Ile Pro Gln Tyr Gly Tyr Leu Thr Leu Asn Asn Gly
    370                 375                 380

Ser Gln Ala Val Gly Arg Ser Ser Phe Tyr Cys Leu Glu Tyr Phe Pro
385                 390                 395                 400

Ser Gln Met Leu Arg Thr Gly Asn Asn Phe Thr Phe Ser Tyr Thr Phe
                405                 410                 415

Glu Glu Val Pro Phe His Ser Ser Tyr Ala His Ser Gln Ser Leu Asp
            420                 425                 430

Arg Leu Met Asn Pro Leu Ile Asp Gln Tyr Leu Tyr Tyr Leu Asn Arg
        435                 440                 445

Thr Gln Asn Gln Ser Gly Ser Ala Gln Asn Lys Asp Leu Leu Phe Ser
    450                 455                 460

Arg Gly Ser Pro Ala Gly Met Ser Val Gln Pro Lys Asn Trp Leu Pro
465                 470                 475                 480

Gly Pro Cys Tyr Arg Gln Gln Arg Val Ser Lys Thr Lys Thr Asp Asn
                485                 490                 495

Asn Asn Ser Asn Phe Thr Trp Thr Gly Ala Ser Lys Tyr Asn Leu Asn
            500                 505                 510

Gly Arg Glu Ser Ile Ile Asn Pro Gly Thr Ala Met Ala Ser His Lys
        515                 520                 525

Asp Asp Glu Asp Lys Phe Phe Pro Met Ser Gly Val Met Ile Phe Gly
    530                 535                 540

Lys Glu Ser Ala Gly Ala Ser Asn Thr Ala Leu Asp Asn Val Met Ile
545                 550                 555                 560

Thr Asp Glu Glu Glu Ile Lys Ala Thr Asn Pro Val Ala Thr Glu Arg
                565                 570                 575

Phe Gly Thr Val Ala Val Asn Phe Gln Ser Ser Ser Thr Asp Pro Ala
            580                 585                 590

Thr Gly Asp Val His Ala Met Gly Ala Leu Pro Gly Met Val Trp Gln
        595                 600                 605

Asp Arg Asp Val Tyr Leu Gln Gly Pro Ile Trp Ala Lys Ile Pro His
    610                 615                 620

Thr Asp Gly His Phe His Pro Ser Pro Leu Met Gly Gly Phe Gly Leu
625                 630                 635                 640

Lys Asn Pro Pro Pro Gln Ile Leu Ile Lys Asn Thr Pro Val Pro Ala
                645                 650                 655

Asn Pro Pro Ala Glu Phe Ser Ala Thr Lys Phe Ala Ser Phe Ile Thr
            660                 665                 670

Gln Tyr Ser Thr Gly Gln Val Ser Val Glu Ile Glu Trp Glu Leu Gln
        675                 680                 685

Lys Glu Asn Ser Lys Arg Trp Asn Pro Glu Val Gln Tyr Thr Ser Asn
    690                 695                 700

Tyr Ala Lys Ser Ala Asn Val Asp Phe Thr Val Asp Asn Asn Gly Leu
705                 710                 715                 720

Tyr Thr Glu Pro Arg Pro Ile Gly Thr Arg Tyr Leu Thr Arg Pro Leu
                725                 730                 735

<210> 14
<211> 1800
<212> DNA
<213> AAV-1

<220>
<221> CDS
<222> (1)..(1797)

<400> 14
acg gct cct gga aag aaa cgt ccg gta gag cag tcg cca caa gag cca      48
Thr Ala Pro Gly Lys Lys Arg Pro Val Glu Gln Ser Pro Gln Glu Pro
1               5                   10                  15

gac tcc tcc tcg ggc atc ggc aag aca ggc cag cag ccc gct aaa aag      96
Asp Ser Ser Ser Gly Ile Gly Lys Thr Gly Gln Gln Pro Ala Lys Lys
            20                  25                  30

aga ctc aat ttt ggt cag act ggc gac tca gag tca gtc ccc gat cca      144
Arg Leu Asn Phe Gly Gln Thr Gly Asp Ser Glu Ser Val Pro Asp Pro
        35                  40                  45

caa cct ctc gga gaa cct cca gca acc ccc gct gct gtg gga cct act      192
Gln Pro Leu Gly Glu Pro Pro Ala Thr Pro Ala Ala Val Gly Pro Thr
    50                  55                  60

aca atg gct tca ggc ggt ggc gca cca atg gca gac aat aac gaa ggc      240
Thr Met Ala Ser Gly Gly Gly Ala Pro Met Ala Asp Asn Asn Glu Gly
65                  70                  75                  80

gcc gac gga gtg ggt aat gcc tca gga aat tgg cat tgc gat tcc aca      288
Ala Asp Gly Val Gly Asn Ala Ser Gly Asn Trp His Cys Asp Ser Thr
                85                  90                  95

tgg ctg ggc gac aga gtc atc acc acc agc acc cgc acc tgg gcc ttg      336
Trp Leu Gly Asp Arg Val Ile Thr Thr Ser Thr Arg Thr Trp Ala Leu
            100                 105                 110

ccc acc tac aat aac cac ctc tac aag caa atc tcc agt gct tca acg      384
Pro Thr Tyr Asn Asn His Leu Tyr Lys Gln Ile Ser Ser Ala Ser Thr
        115                 120                 125

ggg gcc agc aac gac aac cac tac ttc ggc tac agc acc ccc tgg ggg 432
Gly Ala Ser Asn Asp Asn His Tyr Phe Gly Tyr Ser Thr Pro Trp Gly
    130                 135                 140

tat ttt gat ttc aac aga ttc cac tgc cac ttt tca cca cgt gac tgg 480
Tyr Phe Asp Phe Asn Arg Phe His Cys His Phe Ser Pro Arg Asp Trp
145                 150                 155                 160

cag cga ctc atc aac aac aat tgg gga ttc cgg ccc aag aga ctc aac 528
Gln Arg Leu Ile Asn Asn Asn Trp Gly Phe Arg Pro Lys Arg Leu Asn
                165                 170                 175

ttc aaa ctc ttc aac atc caa gtc aag gag gtc acg acg aat gat ggc 576
Phe Lys Leu Phe Asn Ile Gln Val Lys Glu Val Thr Thr Asn Asp Gly
            180                 185                 190

gtc aca acc atc gct aat aac ctt acc agc acg gtt caa gtc ttc tcg 624
Val Thr Thr Ile Ala Asn Asn Leu Thr Ser Thr Val Gln Val Phe Ser
        195                 200                 205

gac tcg gag tac cag ctt ccg tac gtc ctc ggc tct gcg cac cag ggc 672
Asp Ser Glu Tyr Gln Leu Pro Tyr Val Leu Gly Ser Ala His Gln Gly
    210                 215                 220

tgc ctc cct ccg ttc ccg gcg gac gtg ttc atg att ccg caa tac ggc 720
Cys Leu Pro Pro Phe Pro Ala Asp Val Phe Met Ile Pro Gln Tyr Gly
225                 230                 235                 240

tac ctg acg ctc aac aat ggc agc caa gcc gtg gga cgt tca tcc ttt 768
Tyr Leu Thr Leu Asn Asn Gly Ser Gln Ala Val Gly Arg Ser Ser Phe
                245                 250                 255

tac tgc ctg gaa tat ttc cct tct cag atg ctg aga acg ggc aac aac 816
Tyr Cys Leu Glu Tyr Phe Pro Ser Gln Met Leu Arg Thr Gly Asn Asn
            260                 265                 270

ttt acc ttc agc tac acc ttt gag gaa gtg cct ttc cac agc agc tac 864
Phe Thr Phe Ser Tyr Thr Phe Glu Glu Val Pro Phe His Ser Ser Tyr
        275                 280                 285

gcg cac agc cag agc ctg gac cgg ctg atg aat cct ctc atc gac caa 912
Ala His Ser Gln Ser Leu Asp Arg Leu Met Asn Pro Leu Ile Asp Gln
    290                 295                 300

tac ctg tat tac ctg aac aga act caa aat cag tcc gga agt gcc caa 960
Tyr Leu Tyr Tyr Leu Asn Arg Thr Gln Asn Gln Ser Gly Ser Ala Gln
305                 310                 315                 320

aac aag gac ttg ctg ttt agc cgt ggg tct cca gct ggc atg tct gtt 1008
Asn Lys Asp Leu Leu Phe Ser Arg Gly Ser Pro Ala Gly Met Ser Val
                325                 330                 335

cag ccc aaa aac tgg cta cct gga ccc tgt tat cgg cag cag cgc gtt 1056
Gln Pro Lys Asn Trp Leu Pro Gly Pro Cys Tyr Arg Gln Gln Arg Val
            340                 345                 350

tct aaa aca aaa aca gac aac aac aac agc aat ttt acc tgg act ggt 1104
Ser Lys Thr Lys Thr Asp Asn Asn Asn Ser Asn Phe Thr Trp Thr Gly
        355                 360                 365

gct tca aaa tat aac ctc aat ggg cgt gaa tcc atc atc aac cct ggc 1152
Ala Ser Lys Tyr Asn Leu Asn Gly Arg Glu Ser Ile Ile Asn Pro Gly
    370                 375                 380

act gct atg gcc tca cac aaa gac gac gaa gac aag ttc ttt ccc atg 1200
Thr Ala Met Ala Ser His Lys Asp Asp Glu Asp Lys Phe Phe Pro Met
385                 390                 395                 400

agc ggt gtc atg att ttt gga aaa gag agc gcc gga gct tca aac act 1248
Ser Gly Val Met Ile Phe Gly Lys Glu Ser Ala Gly Ala Ser Asn Thr
                405                 410                 415

gca ttg gac aat gtc atg att aca gac gaa gag gaa att aaa gcc act 1296
Ala Leu Asp Asn Val Met Ile Thr Asp Glu Glu Glu Ile Lys Ala Thr
            420                 425                 430

aac cct gtg gcc acc gaa aga ttt ggg acc gtg gca gtc aat ttc cag 1344
Asn Pro Val Ala Thr Glu Arg Phe Gly Thr Val Ala Val Asn Phe Gln
        435                 440                 445

agc agc agc aca gac cct gcg acc gga gat gtg cat gct atg gga gca 1392
Ser Ser Ser Thr Asp Pro Ala Thr Gly Asp Val His Ala Met Gly Ala
    450                 455                 460

tta cct ggc atg gtg tgg caa gat aga gac gtg tac ctg cag ggt ccc 1440
Leu Pro Gly Met Val Trp Gln Asp Arg Asp Val Tyr Leu Gln Gly Pro
465                 470                 475                 480

att tgg gcc aaa att cct cac aca gat gga cac ttt cac ccg tct cct 1488
Ile Trp Ala Lys Ile Pro His Thr Asp Gly His Phe His Pro Ser Pro
                485                 490                 495

ctt atg ggc ggc ttt gga ctc aag aac ccg cct cct cag atc ctc atc 1536
Leu Met Gly Gly Phe Gly Leu Lys Asn Pro Pro Pro Gln Ile Leu Ile
            500                 505                 510

aaa aac acg cct gtt cct gcg aat cct ccg gcg gag ttt tca gct aca    1584
Lys Asn Thr Pro Val Pro Ala Asn Pro Pro Ala Glu Phe Ser Ala Thr
        515                 520                 525

aag ttt gct tca ttc atc acc caa tac tcc aca gga caa gtg agt gtg    1632
Lys Phe Ala Ser Phe Ile Thr Gln Tyr Ser Thr Gly Gln Val Ser Val
    530                 535                 540

gaa att gaa tgg gag ctg cag aaa gaa aac agc aag cgc tgg aat ccc    1680
Glu Ile Glu Trp Glu Leu Gln Lys Glu Asn Ser Lys Arg Trp Asn Pro
545                 550                 555                 560

gaa gtg cag tac aca tcc aat tat gca aaa tct gcc aac gtt gat ttt    1728
Glu Val Gln Tyr Thr Ser Asn Tyr Ala Lys Ser Ala Asn Val Asp Phe
                565                 570                 575

act gtg gac aac aat gga ctt tat act gag cct cgc ccc att ggc acc    1776
Thr Val Asp Asn Asn Gly Leu Tyr Thr Glu Pro Arg Pro Ile Gly Thr
            580                 585                 590

cgt tac ctt acc cgt ccc ctg taa                                    1800
Arg Tyr Leu Thr Arg Pro Leu
        595

<210> 15
<211> 599
<212> PRT
<213> AAV-1

<400> 15
Thr Ala Pro Gly Lys Lys Arg Pro Val Glu Gln Ser Pro Gln Glu Pro
1               5                   10                  15

Asp Ser Ser Ser Gly Ile Gly Lys Thr Gly Gln Gln Pro Ala Lys Lys
            20                  25                  30

Arg Leu Asn Phe Gly Gln Thr Gly Asp Ser Glu Ser Val Pro Asp Pro
        35                  40                  45

Gln Pro Leu Gly Glu Pro Pro Ala Thr Pro Ala Ala Val Gly Pro Thr
    50                  55                  60

Thr Met Ala Ser Gly Gly Gly Ala Pro Met Ala Asp Asn Asn Glu Gly
65                  70                  75                  80

Ala Asp Gly Val Gly Asn Ala Ser Gly Asn Trp His Cys Asp Ser Thr

                85                  90                  95

Trp Leu Gly Asp Arg Val Ile Thr Thr Ser Thr Arg Thr Trp Ala Leu
            100                 105                 110

Pro Thr Tyr Asn Asn His Leu Tyr Lys Gln Ile Ser Ser Ala Ser Thr
        115                 120                 125

Gly Ala Ser Asn Asp Asn His Tyr Phe Gly Tyr Ser Thr Pro Trp Gly
    130                 135                 140

Tyr Phe Asp Phe Asn Arg Phe His Cys His Phe Ser Pro Arg Asp Trp
145                 150                 155                 160

Gln Arg Leu Ile Asn Asn Asn Trp Gly Phe Arg Pro Lys Arg Leu Asn
                165                 170                 175

Phe Lys Leu Phe Asn Ile Gln Val Lys Glu Val Thr Thr Asn Asp Gly
            180                 185                 190

Val Thr Thr Ile Ala Asn Asn Leu Thr Ser Thr Val Gln Val Phe Ser
        195                 200                 205

Asp Ser Glu Tyr Gln Leu Pro Tyr Val Leu Gly Ser Ala His Gln Gly
    210                 215                 220

Cys Leu Pro Pro Phe Pro Ala Asp Val Phe Met Ile Pro Gln Tyr Gly
225                 230                 235                 240

Tyr Leu Thr Leu Asn Asn Gly Ser Gln Ala Val Gly Arg Ser Ser Phe
                245                 250                 255

Tyr Cys Leu Glu Tyr Phe Pro Ser Gln Met Leu Arg Thr Gly Asn Asn
            260                 265                 270

Phe Thr Phe Ser Tyr Thr Phe Glu Glu Val Pro Phe His Ser Ser Tyr
        275                 280                 285

Ala His Ser Gln Ser Leu Asp Arg Leu Met Asn Pro Leu Ile Asp Gln
    290                 295                 300

Tyr Leu Tyr Tyr Leu Asn Arg Thr Gln Asn Gln Ser Gly Ser Ala Gln
305                 310                 315                 320

Asn Lys Asp Leu Leu Phe Ser Arg Gly Ser Pro Ala Gly Met Ser Val
                325                 330                 335

Gln Pro Lys Asn Trp Leu Pro Gly Pro Cys Tyr Arg Gln Gln Arg Val

340                 345                 350

Ser Lys Thr Lys Thr Asp Asn Asn Asn Ser Asn Phe Thr Trp Thr Gly
        355                 360                 365

Ala Ser Lys Tyr Asn Leu Asn Gly Arg Glu Ser Ile Ile Asn Pro Gly
    370                 375                 380

Thr Ala Met Ala Ser His Lys Asp Asp Glu Asp Lys Phe Phe Pro Met
385                 390                 395                 400

Ser Gly Val Met Ile Phe Gly Lys Glu Ser Ala Gly Ala Ser Asn Thr
                405                 410                 415

Ala Leu Asp Asn Val Met Ile Thr Asp Glu Glu Glu Ile Lys Ala Thr
            420                 425                 430

Asn Pro Val Ala Thr Glu Arg Phe Gly Thr Val Ala Val Asn Phe Gln
        435                 440                 445

Ser Ser Ser Thr Asp Pro Ala Thr Gly Asp Val His Ala Met Gly Ala
    450                 455                 460

Leu Pro Gly Met Val Trp Gln Asp Arg Asp Val Tyr Leu Gln Gly Pro
465                 470                 475                 480

Ile Trp Ala Lys Ile Pro His Thr Asp Gly His Phe His Pro Ser Pro
                485                 490                 495

Leu Met Gly Gly Phe Gly Leu Lys Asn Pro Pro Pro Gln Ile Leu Ile
            500                 505                 510

Lys Asn Thr Pro Val Pro Ala Asn Pro Pro Ala Glu Phe Ser Ala Thr
        515                 520                 525

Lys Phe Ala Ser Phe Ile Thr Gln Tyr Ser Thr Gly Gln Val Ser Val
    530                 535                 540

Glu Ile Glu Trp Glu Leu Gln Lys Glu Asn Ser Lys Arg Trp Asn Pro
545                 550                 555                 560

Glu Val Gln Tyr Thr Ser Asn Tyr Ala Lys Ser Ala Asn Val Asp Phe
                565                 570                 575

Thr Val Asp Asn Asn Gly Leu Tyr Thr Glu Pro Arg Pro Ile Gly Thr
            580                 585                 590

Arg Tyr Leu Thr Arg Pro Leu

        595

<210> 16
<211> 1605
<212> DNA
<213> AAV-1

<220>
<221> CDS
<222> (1)..(1602)

<400> 16
atg gct tca ggc ggt ggc gca cca atg gca gac aat aac gaa ggc gcc 48
Met Ala Ser Gly Gly Gly Ala Pro Met Ala Asp Asn Asn Glu Gly Ala
1               5                   10                  15

gac gga gtg ggt aat gcc tca gga aat tgg cat tgc gat tcc aca tgg 96
Asp Gly Val Gly Asn Ala Ser Gly Asn Trp His Cys Asp Ser Thr Trp
            20                  25                  30

ctg ggc gac aga gtc atc acc acc agc acc cgc acc tgg gcc ttg ccc 144
Leu Gly Asp Arg Val Ile Thr Thr Ser Thr Arg Thr Trp Ala Leu Pro
        35                  40                  45

acc tac aat aac cac ctc tac aag caa atc tcc agt gct tca acg ggg 192
Thr Tyr Asn Asn His Leu Tyr Lys Gln Ile Ser Ser Ala Ser Thr Gly
    50                  55                  60

gcc agc aac gac aac cac tac ttc ggc tac agc acc ccc tgg ggg tat 240
Ala Ser Asn Asp Asn His Tyr Phe Gly Tyr Ser Thr Pro Trp Gly Tyr
65                  70                  75                  80

ttt gat ttc aac aga ttc cac tgc cac ttt tca cca cgt gac tgg cag 288
Phe Asp Phe Asn Arg Phe His Cys His Phe Ser Pro Arg Asp Trp Gln
                85                  90                  95

cga ctc atc aac aac aat tgg gga ttc cgg ccc aag aga ctc aac ttc 336
Arg Leu Ile Asn Asn Asn Trp Gly Phe Arg Pro Lys Arg Leu Asn Phe
            100                 105                 110

aaa ctc ttc aac atc caa gtc aag gag gtc acg acg aat gat ggc gtc 384
Lys Leu Phe Asn Ile Gln Val Lys Glu Val Thr Thr Asn Asp Gly Val
        115                 120                 125

aca acc atc gct aat aac ctt acc agc acg gtt caa gtc ttc tcg gac 432
Thr Thr Ile Ala Asn Asn Leu Thr Ser Thr Val Gln Val Phe Ser Asp
    130                 135                 140

tcg gag tac cag ctt ccg tac gtc ctc ggc tct gcg cac cag ggc tgc 480
Ser Glu Tyr Gln Leu Pro Tyr Val Leu Gly Ser Ala His Gln Gly Cys
145                 150                 155                 160

ctc cct ccg ttc ccg gcg gac gtg ttc atg att ccg caa tac ggc tac 528
Leu Pro Pro Phe Pro Ala Asp Val Phe Met Ile Pro Gln Tyr Gly Tyr
                165                 170                 175

ctg acg ctc aac aat ggc agc caa gcc gtg gga cgt tca tcc ttt tac 576
Leu Thr Leu Asn Asn Gly Ser Gln Ala Val Gly Arg Ser Ser Phe Tyr
            180                 185                 190

tgc ctg gaa tat ttc cct tct cag atg ctg aga acg ggc aac aac ttt 624
Cys Leu Glu Tyr Phe Pro Ser Gln Met Leu Arg Thr Gly Asn Asn Phe
        195                 200                 205

acc ttc agc tac acc ttt gag gaa gtg cct ttc cac agc agc tac gcg 672
Thr Phe Ser Tyr Thr Phe Glu Glu Val Pro Phe His Ser Ser Tyr Ala
    210                 215                 220

cac agc cag agc ctg gac cgg ctg atg aat cct ctc atc gac caa tac 720
His Ser Gln Ser Leu Asp Arg Leu Met Asn Pro Leu Ile Asp Gln Tyr
225                 230                 235                 240

ctg tat tac ctg aac aga act caa aat cag tcc gga agt gcc caa aac 768
Leu Tyr Tyr Leu Asn Arg Thr Gln Asn Gln Ser Gly Ser Ala Gln Asn
                245                 250                 255

aag gac ttg ctg ttt agc cgt ggg tct cca gct ggc atg tct gtt cag 816
Lys Asp Leu Leu Phe Ser Arg Gly Ser Pro Ala Gly Met Ser Val Gln
            260                 265                 270

ccc aaa aac tgg cta cct gga ccc tgt tat cgg cag cag cgc gtt tct 864
Pro Lys Asn Trp Leu Pro Gly Pro Cys Tyr Arg Gln Gln Arg Val Ser
        275                 280                 285

aaa aca aaa aca gac aac aac aac agc aat ttt acc tgg act ggt gct 912
Lys Thr Lys Thr Asp Asn Asn Asn Ser Asn Phe Thr Trp Thr Gly Ala
    290                 295                 300

tca aaa tat aac ctc aat ggg cgt gaa tcc atc atc aac cct ggc act 960
Ser Lys Tyr Asn Leu Asn Gly Arg Glu Ser Ile Ile Asn Pro Gly Thr
305                 310                 315                 320

gct atg gcc tca cac aaa gac gac gaa gac aag ttc ttt ccc atg agc 1008
Ala Met Ala Ser His Lys Asp Asp Glu Asp Lys Phe Phe Pro Met Ser
                325                 330                 335

ggt gtc atg att ttt gga aaa gag agc gcc gga gct tca aac act gca 1056
Gly Val Met Ile Phe Gly Lys Glu Ser Ala Gly Ala Ser Asn Thr Ala
            340                 345                 350

ttg gac aat gtc atg att aca gac gaa gag gaa att aaa gcc act aac 1104
Leu Asp Asn Val Met Ile Thr Asp Glu Glu Glu Ile Lys Ala Thr Asn
        355                 360                 365

cct gtg gcc acc gaa aga ttt ggg acc gtg gca gtc aat ttc cag agc 1152
Pro Val Ala Thr Glu Arg Phe Gly Thr Val Ala Val Asn Phe Gln Ser
    370                 375                 380

agc agc aca gac cct gcg acc gga gat gtg cat gct atg gga gca tta 1200
Ser Ser Thr Asp Pro Ala Thr Gly Asp Val His Ala Met Gly Ala Leu
385                 390                 395                 400

cct ggc atg gtg tgg caa gat aga gac gtg tac ctg cag ggt ccc att 1248
Pro Gly Met Val Trp Gln Asp Arg Asp Val Tyr Leu Gln Gly Pro Ile
                405                 410                 415

tgg gcc aaa att cct cac aca gat gga cac ttt cac ccg tct cct ctt 1296
Trp Ala Lys Ile Pro His Thr Asp Gly His Phe His Pro Ser Pro Leu
            420                 425                 430

atg ggc ggc ttt gga ctc aag aac ccg cct cct cag atc ctc atc aaa 1344
Met Gly Gly Phe Gly Leu Lys Asn Pro Pro Pro Gln Ile Leu Ile Lys
        435                 440                 445

aac acg cct gtt cct gcg aat cct ccg gcg gag ttt tca gct aca aag 1392
Asn Thr Pro Val Pro Ala Asn Pro Pro Ala Glu Phe Ser Ala Thr Lys
    450                 455                 460

ttt gct tca ttc atc acc caa tac tcc aca gga caa gtg agt gtg gaa 1440
Phe Ala Ser Phe Ile Thr Gln Tyr Ser Thr Gly Gln Val Ser Val Glu
465                 470                 475                 480

att gaa tgg gag ctg cag aaa gaa aac agc aag cgc tgg aat ccc gaa 1488
Ile Glu Trp Glu Leu Gln Lys Glu Asn Ser Lys Arg Trp Asn Pro Glu
                485                 490                 495

gtg cag tac aca tcc aat tat gca aaa tct gcc aac gtt gat ttt act 1536
Val Gln Tyr Thr Ser Asn Tyr Ala Lys Ser Ala Asn Val Asp Phe Thr
            500                 505                 510

gtg gac aac aat gga ctt tat act gag cct cgc ccc att ggc acc cgt 1584
Val Asp Asn Asn Gly Leu Tyr Thr Glu Pro Arg Pro Ile Gly Thr Arg
        515                 520                 525

tac ctt acc cgt ccc ctg taa 1605
Tyr Leu Thr Arg Pro Leu
    530

<210> 17
<211> 534
<212> PRT
<213> AAV-1

<400> 17
Met Ala Ser Gly Gly Gly Ala Pro Met Ala Asp Asn Asn Glu Gly Ala
1               5                   10                  15

Asp Gly Val Gly Asn Ala Ser Gly Asn Trp His Cys Asp Ser Thr Trp
            20                  25                  30

Leu Gly Asp Arg Val Ile Thr Thr Ser Thr Arg Thr Trp Ala Leu Pro
        35                  40                  45

Thr Tyr Asn Asn His Leu Tyr Lys Gln Ile Ser Ser Ala Ser Thr Gly
    50                  55                  60

Ala Ser Asn Asp Asn His Tyr Phe Gly Tyr Ser Thr Pro Trp Gly Tyr
65                  70                  75                  80

Phe Asp Phe Asn Arg Phe His Cys His Phe Ser Pro Arg Asp Trp Gln
                85                  90                  95

Arg Leu Ile Asn Asn Asn Trp Gly Phe Arg Pro Lys Arg Leu Asn Phe
            100                 105                 110

Lys Leu Phe Asn Ile Gln Val Lys Glu Val Thr Thr Asn Asp Gly Val
        115                 120                 125

Thr Thr Ile Ala Asn Asn Leu Thr Ser Thr Val Gln Val Phe Ser Asp
    130                 135                 140

Ser Glu Tyr Gln Leu Pro Tyr Val Leu Gly Ser Ala His Gln Gly Cys
145                 150                 155                 160

Leu Pro Pro Phe Pro Ala Asp Val Phe Met Ile Pro Gln Tyr Gly Tyr
                165                 170                 175

Leu Thr Leu Asn Asn Gly Ser Gln Ala Val Gly Arg Ser Ser Phe Tyr
            180                 185                 190

Cys Leu Glu Tyr Phe Pro Ser Gln Met Leu Arg Thr Gly Asn Asn Phe
        195                 200                 205

Thr Phe Ser Tyr Thr Phe Glu Glu Val Pro Phe His Ser Ser Tyr Ala
    210                 215                 220

His Ser Gln Ser Leu Asp Arg Leu Met Asn Pro Leu Ile Asp Gln Tyr
225                 230                 235                 240

Leu Tyr Tyr Leu Asn Arg Thr Gln Asn Gln Ser Gly Ser Ala Gln Asn
                245                 250                 255

Lys Asp Leu Leu Phe Ser Arg Gly Ser Pro Ala Gly Met Ser Val Gln
            260                 265                 270

Pro Lys Asn Trp Leu Pro Gly Pro Cys Tyr Arg Gln Gln Arg Val Ser
        275                 280                 285

Lys Thr Lys Thr Asp Asn Asn Asn Ser Asn Phe Thr Trp Thr Gly Ala
    290                 295                 300

Ser Lys Tyr Asn Leu Asn Gly Arg Glu Ser Ile Ile Asn Pro Gly Thr
305                 310                 315                 320

Ala Met Ala Ser His Lys Asp Asp Glu Asp Lys Phe Phe Pro Met Ser
                325                 330                 335

Gly Val Met Ile Phe Gly Lys Glu Ser Ala Gly Ala Ser Asn Thr Ala
            340                 345                 350

Leu Asp Asn Val Met Ile Thr Asp Glu Glu Glu Ile Lys Ala Thr Asn
        355                 360                 365

Pro Val Ala Thr Glu Arg Phe Gly Thr Val Ala Val Asn Phe Gln Ser
    370                 375                 380

Ser Ser Thr Asp Pro Ala Thr Gly Asp Val His Ala Met Gly Ala Leu
385                 390                 395                 400

Pro Gly Met Val Trp Gln Asp Arg Asp Val Tyr Leu Gln Gly Pro Ile
                405                 410                 415

Trp Ala Lys Ile Pro His Thr Asp Gly His Phe His Pro Ser Pro Leu
            420                 425                 430

Met Gly Gly Phe Gly Leu Lys Asn Pro Pro Pro Gln Ile Leu Ile Lys
        435                 440                 445

Asn Thr Pro Val Pro Ala Asn Pro Pro Ala Glu Phe Ser Ala Thr Lys
    450                 455                 460

Phe Ala Ser Phe Ile Thr Gln Tyr Ser Thr Gly Gln Val Ser Val Glu
465                 470                 475                 480

Ile Glu Trp Glu Leu Gln Lys Glu Asn Ser Lys Arg Trp Asn Pro Glu
                485                 490                 495

Val Gln Tyr Thr Ser Asn Tyr Ala Lys Ser Ala Asn Val Asp Phe Thr
            500                 505                 510

Val Asp Asn Asn Gly Leu Tyr Thr Glu Pro Arg Pro Ile Gly Thr Arg
        515                 520                 525

Tyr Leu Thr Arg Pro Leu
    530

<210> 18
<211> 4681
<212> DNA
<213> aav-2

<400> 18
ttggccactc cctctctgcg cgctcgctcg ctcactgagg ccgggcgacc aaaggtcgcc 60

cgacgcccgg gctttgcccg ggcggcctca gtgagcgagc gagcgcgcag agagggagtg 120

gccaactcca tcactagggg ttcctggagg ggtggagtcg tgacgtgaat tacgtcatag 180

ggttagggag gtcctgtatt agaggtcacg tgagtgtttt gcgacatttt gcgacaccat 240

gtggtcacgc tgggtattta agcccgagtg agcacgcagg gtctccattt tgaagcggga 300

ggtttgaacg cgcagccgcc atgccggggt tttacgagat tgtgattaag gtccccagcg 360

accttgacgg gcatctgccc ggcatttctg acagctttgt gaactgggtg gccgagaagg 420

aatgggagtt gccgccagat tctgacatgg atctgaatct gattgagcag gcacccctga 480

ccgtggccga gaagctgcag cgcgactttc tgacggaatg gcgccgtgtg agtaaggccc 540

cggaggccct tttctttgtg caatttgaga agggagagag ctacttccac atgcacgtgc 600

tcgtggaaac caccggggtg aaatccatgg ttttgggacg tttcctgagt cagattcgcg 660

aaaaactgat tcagagaatt taccgcggga tcgagccgac tttgccaaac tggttcgcgg 720

tcacaaagac cagaaatggc gccggaggcg ggaacaaggt ggtggatgag tgctacatcc 780

ccaattactt gctccccaaa acccagcctg agctccagtg ggcgtggact aatatggaac 840

agtatttaag cgcctgtttg aatctcacgg agcgtaaacg gttggtggcg cagcatctga 900

cgcacgtgtc gcagacgcag gagcagaaca aagagaatca gaatcccaat tctgatgcgc 960

cggtgatcag atcaaaaact tcagccaggt acatggagct ggtcgggtgg ctcgtggaca 1020

aggggattac ctcggagaag cagtggatcc aggaggacca ggcctcatac atctccttca 1080

atgcggcctc caactcgcgg tcccaaatca aggctgcctt ggacaatgcg ggaaagatta 1140

tgagcctgac taaaaccgcc cccgactacc tggtgggcca gcagcccgtg gaggacattt 1200

ccagcaatcg gatttataaa attttggaac taaacgggta cgatccccaa tatgcggctt 1260

ccgtctttct gggatgggcc acgaaaaagt tcggcaagag gaacaccatc tggctgtttg 1320

ggcctgcaac taccgggaag accaacatcg cggaggccat agcccacact gtgcccttct 1380

acgggtgcgt aaactggacc aatgagaact ttcccttcaa cgactgtgtc gacaagatgg 1440

tgatctggtg ggaggagggg aagatgaccg ccaaggtcgt ggagtcggcc aaagccattc 1500

tcggaggaag caaggtgcgc gtggaccaga aatgcaagtc ctcggcccag atagacccga 1560

ctcccgtgat cgtcacctcc aacaccaaca tgtgcgccgt gattgacggg aactcaacga 1620

ccttcgaaca ccagcagccg ttgcaagacc ggatgttcaa atttgaactc acccgccgtc 1680

tggatcatga ctttgggaag gtcaccaagc aggaagtcaa agactttttc cggtgggcaa 1740

aggatcacgt ggttgaggtg gagcatgaat tctacgtcaa aaagggtgga gccaagaaaa 1800

gacccgcccc cagtgacgca gatataagtg agcccaaacg ggtgcgcgag tcagttgcgc 1860

agccatcgac gtcagacgcg gaagcttcga tcaactacgc agacaggtac caaaacaaat 1920

gttctcgtca cgtgggcatg aatctgatgc tgtttccctg cagacaatgc gagagaatga 1980

atcagaattc aaatatctgc ttcactcacg gacagaaaga ctgtttagag tgctttcccg 2040

tgtcagaatc tcaaccggtt tctgtcgtca aaaaggcgta tcagaaactg tgctacattc 2100

atcatatcat gggaaaggtg ccagacgctt gcactgcctg cgatctggtc aatgtggatt 2160

tggatgactg catctttgaa caataaatga tttaaatcag gtatggctgc cgatggttat 2220

cttccagatt ggctcgagga cactctctct gaaggaataa gacagtggtg gaagctcaaa 2280

cctggcccac caccaccaaa gcccgcagag cggcataagg acgacagcag gggtcttgtg 2340

cttcctgggt acaagtacct cggacccttc aacggactcg acaagggaga gccggtcaac 2400

gaggcagacg ccgcggccct cgagcacgac aaagcctacg accggcagct cgacagcgga 2460

gacaacccgt acctcaagta caaccacgcc gacgcggagt ttcaggagcg ccttaaagaa 2520

gatacgtctt ttgggggcaa cctcggacga gcagtcttcc aggcgaaaaa gagggttctt 2580

gaacctctcg gcctggttga ggaacctgtt aagacggctc cgggaaaaaa gaggccggta 2640

gagcactctc ctgtggagcc agactcctcc tcgggaaccg gaaagccggg ccagcagcct 2700

gcaagaaaaa gattgaattt tggtcagact ggagacgcag actcagtacc tgacccccag 2760

cctctcggac agccaccagc agccccctct ggtctgggaa ctaatacgat ggctacaggc 2820

agtggcgcac caatggcaga caataacgag ggcgccgacg gagtgggtaa ttcctccgga 2880

aattggcatt gcgattccac atggatgggc gacagagtca tcaccaccag cacccgaacc 2940

tgggccctgc ccacctacaa caaccacctc tacaaacaaa tttccagcca atcaggagcc 3000

tcgaacgaca atcactactt tggctacagc accccttggg ggtattttga cttcaacaga 3060

ttccactgcc acttttcacc acgtgactgg caaagactca tcaacaacaa ctggggattc 3120

cgacccaaga gactcaactt caacctcttt aacattcaag tcaaagaggt cacgcagaat 3180

gacggtacga cgacgattgc caataacctt accagcacgg ttcaggtgtt tactgactcg 3240

gagtaccagc tcccgtacgt cctcggctcg gcgcatcaag gatgcctccc gccgttccca 3300

gcagacgtct tcatggtgcc acagtatgga tacctcaccc tgaacaacgg gagtcaggca 3360

gtaggacgct cttcatttta ctgcctggag tactttcctt ctcagatgct gcgtaccgga 3420

aacaacttta ccttcagcta cacttttgag gacgttcctt tccacagcag ctacgctcac 3480

agccagagtc tggaccgtct catgaatcct ctcatcgacc agtacctgta ttacttgagc 3540

agaacaaaca ctccaagtgg aaccaccacg cagtcaaggc ttcagttttc tcaggcccca 3600

gccagtgaca ttcgggacca gtctaggaac tggcttcctg gaccctgtta ccgccagcag 3660

cgagtatgaa agacatctgc ggataacaac aacagtgaat actcgtggac tggagctacc 3720

aagtaccacc tcaatggcag agactctctg gtgaatccgg ggcccgccat ggcaagccac 3780

aaggacgatg aagaaaagtt ttttcctcag agcggggttc tcatctttgg gaagcaaggc 3840

tcagagaaaa caaatgtgaa cattgaaaag gtcatgatta cagacgaaga ggaaatccca 3900

acaaccaatc ccgtggctac ggagcagtat ggttctgtat ctaccaacct ccagagaggc 3960

aacagacaag cagctaccgc agatgtcaac acacaaggcg ttcttccagg catggtctgg 4020

caggacagag atgtgtacct tcaggggccc atctgggcaa agattccaca cacggacgga 4080

cattttcacc cctctcccct catgggtgga ttcggactta aacaccctcc tccacagatt 4140

ctcatcaaga acaccccggt acctgcgaat ccttcgacca ccttcagtgc ggcaaagttt 4200

gcttccttca tcacacagta ctccacggga cacggtcagc gtggagatcg agtgggagct 4260

gcagaacgaa aacagcaaac gctggaatcc cgaaattcag tacacttcca actacaacaa 4320

gtctgttaat cgtggacttt accgtggata ctaatggcgt gtattcagag cctcgcccca 4380

ttggcaccag atacctgact cgtaatctgt aattgcttgt taatcaataa accgtttaat 4440

tcgtttcagt tgaactttgg tctctgcgta tttctttctt atctagtttc catggctacg 4500

tagataagta gcatggcggg ttaatcatta actacaagga acccctagtg atggagttgg 4560

ccactccctc tctgcgcgct cgctcgctca ctgaggccgg gcgaccaaag gtcgcccgac 4620

gcccgggctt tgcccgggcg gcctcagtga gcgagcgagc gcgcagagag ggagtggcca 4680

a 4681

<210> 19
<211> 4683
<212> DNA
<213> aav-6

<400> 19
ttggccactc cctctctgcg cgctcgctcg ctcactgagg ccgggcgacc aaaggtcgcc 60

cgacgcccgg gctttgcccg ggcggcctca gtgagcgagc gagcgcgcag agagggagtg 120

gccaactcca tcactagggg ttcctggagg ggtggagtcg tgacgtgaat tacgtcatag 180

ggttagggag gtcctgtatt agaggtcacg tgagtgtttt gcgacatttt gcgacaccat 240

gtggtcacgc tgggtattta agcccgagtg agcacgcagg gtctccattt tgaagcggga 300

ggtttgaacg cgcagcgcca tgccggggtt ttacgagatt gtgattaagg tccccagcga 360

ccttgacgag catctgcccg gcatttctga cagctttgtg aactgggtgg ccgagaagga 420

atgggagttg ccgccagatt ctgacatgga tctgaatctg attgagcagg cacccctgac 480

cgtggccgag aagctgcagc gcgacttcct ggtccactgg cgccgcgtga gtaaggcccc 540

ggaggccctc ttctttgttc agttcgagaa gggcgagtcc tacttccacc tccatattct 600

ggtggagacc acgggggtca aatccatggt gctgggccgc ttcctgagtc agattagcga 660

caagctggtg cagaccatct accgcgggat cgagccgacc ctgcccaact ggttcgcggt 720

gaccaagacg cgtaatggcg ccggaggggg gaacaaggtg gtggacgagt gctacatccc 780

caactacctc ctgcccaaga ctcagcccga gctgcagtgg gcgtggacta acatggagga 840

gtatataagc gcgtgtttaa acctggccga gcgcaaacgg ctcgtggcgc acgacctgac 900

ccacgtcagc cagacccagg agcagaacaa ggagaatctg aaccccaatt ctgacgcgcc 960

tgtcatccgg tcaaaaacct ccgcacgcta catggagctg gtcgggtggc tggtggaccg 1020

gggcatcacc tccgagaagc agtggatcca ggaggaccag gcctcgtaca tctccttcaa 1080

cgccgcctcc aactcgcggt cccagatcaa ggccgctctg gacaatgccg gcaagatcat 1140

ggcgctgacc aaatccgcgc ccgactacct ggtaggcccc gctccgcccg ccgacattaa 1200

aaccaaccgc atttaccgca tcctggagct gaacggctac gaccctgcct acgccggctc 1260

cgtctttctc ggctgggccc agaaaaggtt cggaaaacgc aacaccatct ggctgtttgg 1320

gccggccacc acgggcaaga ccaacatcgc ggaagccatc gcccacgccg tgcccttcta 1380

cggctgcgtc aactggacca atgagaactt tcccttcaac gattgcgtcg acaagatggt 1440

gatctggtgg gaggagggca agatgacggc caaggtcgtg gagtccgcca aggccattct 1500

cggcggcagc aaggtgcgcg tggaccaaaa gtgcaagtcg tccgcccaga tcgatcccac 1560

ccccgtgatc gtcacctcca acaccaacat gtgcgccgtg attgacggga acagcaccac 1620

cttcgagcac cagcagccgt tgcaggaccg gatgttcaaa tttgaactca cccgccgtct 1680

ggagcatgac tttggcaagg tgacaaagca ggaagtcaaa gagttcttcc gctgggcgca 1740

ggatcacgtg accgaggtgg cgcatgagtt ctacgtcaga aagggtggag ccaacaacag 1800

acccgccccc gatgacgcgg ataaaagcga gcccaagcgg gcctgcccct cagtcgcgga 1860

tccatcgacg tcagacgcgg aaggagctcc ggtggacttt gccgacaggt accaaaacaa 1920

atgttctcgt cacgcgggca tgcttcagat gctgtttccc tgcaaaacat gcgagagaat 1980

gaatcagaat ttcaacattt gcttcacgca cgggaccaga gactgttcag aatgtttccc 2040

cggcgtgtca gaatctcaac cggtcgtcag aaagaggacg tatcggaaac tctgtgccat 2100

tcatcatctg ctggggcggg ctcccgagat tgcttgctcg gcctgcgatc tggtcaacgt 2160

ggatctggat gactgtgttt ctgagcaata aatgacttaa accaggtatg gctgccgatg 2220

gttatcttcc agattggctc gaggacaacc tctctgaggg cattcggcag tggtgggact 2280

tgaaacctgg agccccgaaa cccaaagcca accagcaaaa gcaggacgac ggccggggtc 2340

tggtgcttcc tggctacaag tacctcggac ccttcaacgg actcgacaag ggggagcccg 2400

tcaacgcggc ggatgcagcg gccctcgagc acgacaaggc ctacgaccag cagctcaaag 2460

cgggtgacaa tccgtacctg cggtataacc acgccgacgc cgagtttcag gagcgtctgc 2520

aagaagatac gtcttttggg ggcaacctcg ggcgagcagt cttccaggcc aagaagaggg 2580

ttctcgaacc ttttggtctg gttgaggaag gtgctaagac ggctcctgga aagaaacgtc 2640

cggtagagca gtcgccacaa gagccagact cctcctcggg cattggcaag acaggccagc 2700

agcccgctaa aaagagactc aattttggtc agactggcga ctcagagtca gtccccgacc 2760

cacaacctct cggagaacct ccagcaaccc ccgctgctgt gggacctact acaatggctt 2820

caggcggtgg cgcaccaatg gcagacaata acgaaggcgc cgacggagtg ggtaatgcct 2880

caggaaattg gcattgcgat tccacatggc tgggcgacag agtcatcacc accagcaccc 2940

gaacatgggc cttgcccacc tataacaacc acctctacaa gcaaatctcc agtgcttcaa 3000

cgggggccag caacgacaac cactacttcg gctacagcac cccctggggg tattttgatt 3060

tcaacagatt ccactgccat ttctcaccac gtgactggca gcgactcatc aacaacaatt 3120

ggggattccg gcccaagaga ctcaacttca agctcttcaa catccaagtc aaggaggtca 3180

cgacgaatga tggcgtcacg accatcgcta ataaccttac cagcacggtt caagtcttgt 3240

cggactcgga gtaccagttc ccgtacgtcc tcggctctgc gcaccagggc tgcctccctc 3300

cgttcccggc ggacgtgttc atgattccgc agtacggcta cctaacgctc aacaatggca 3360

gccaggcagt gggacgctca tccttttact gcctggaata tttcccatcg cagatgctga 3420

gaacgggcaa taactttacc ttcagctaca ccttcgagga cgtgcctttc cacagcagct 3480

acgcgcacag ccagagcctg gaccggctga tgaatcctct catcgaccag tacctgtatt 3540

acctgaacag aactcacaat cagtccggaa gtgcccaaaa caaggacttg ctgtttagcc 3600

gtgggtctcc agctggcatg tctgttcagc ccaaaaactg gctacctgga ccctgttacc 3660

ggcagcagcg cgtttctaaa acaaaaacag acaacaacaa cagcaacttt acctggactg 3720

gtgcttcaaa atataacctt aatgggcgtg aatctataat caaccctggc actgctatgg 3780

cctcacacaa agacgacaaa gacaagttct ttcccatgag cggtgtcatg atttttggaa 3840

aggagagcgc cggagcttca aacactgcat tggacaatgt catgatcaca gacgaagagg 3900

aaatcaaagc cactaacccc gtggccaccg aaagatttgg gactgtggca gtcaatctcc 3960

agagcagcag cacagaccct gcgaccggag atgtgcatgt tatgggagcc ttacctggaa 4020

tggtgtggca agacagagac gtatacctgc agggtcctat ttgggccaaa attcctcaca 4080

cggatggaca ctttcacccg tctcctctca tgggcggctt tggacttaag cacccgcctc 4140

ctcagatcct catcaaaaac acgcctgttc ctgcgaatcc tccggcagag ttttcggcta 4200

caaagtttgc ttcattcatc acccagtatt ccacaggaca agtgagcgtg gagattgaat 4260

gggagctgca gaaagaaaac agcaaacgct ggaatcccga agtgcagtat acatctaact 4320

atgcaaaatc tgccaacgtt gatttcactg tggacaacaa tggactttat actgagcctc 4380

gccccattgg cacccgttac ctcacccgtc ccctgtaatt gtgtgttaat caataaaccg 4440

gttaattcgt gtcagttgaa ctttggtctc atgtccttat tatcttatct ggtcaccata 4500

gcaaccggtt acacattaac tgcttagttg cgcttcgcga atacccctag tgatggagtt 4560

gcccactccc tctatgcgcg ctcgctcgct cggtggggcc ggcagagcag agctctgccg 4620

tctgcggacc tttggtccgc aggccccacc gagcgagcga gcgcgcatag agggagtggc 4680

caa 4683

<210> 20
<211> 16
<212> DNA
<213> rep binding motif

<400> 20
gctcgctcgc tcgctg 16

SEQUENCE LISTING

<110> Wilson, James M.
 Xiao, Weidong
 The Trustees of the University of Pennsylvania

<120> Adeno-Associated Virus Serotype I Nucleic Acid Sequences,
 Vectors and Host Cells Containing Same

<130> GNVPN.031PCT

<140> EP 07007319.2
<141> 2007-04-10

<150> US 60/107,114
<151> 1998-11-05

<160> 20

<170> PatentIn version 2.0

<210> 1
<211> 4718
<212> DNA
<213> AAV-1

<220>
<221> CDS
<222> (335)..(2206)
<223>

<220>
<221> CDS
<222> (2223)..(4430)
<223>

<400> 1
ttgcccactc cctctctgcg cgctcgctcg ctcggtgggg cctgcggacc aaaggtccgc 60

agacggcaga gctctgctct gccggcccca ccgagcgagc gagcgcgcag agagggagtg 120

ggcaactcca tcactagggg taatcgcgaa gcgcctccca cgctgccgcg tcagcgctga 180

cgtaaattac gtcatagggg agtggtcctg tattagctgt cacgtgagtg cttttgcgac 240

attttgcgac accacgtggc catttagggt atatatggcc gagtgagcga gcaggatctc 300

cattttgacc gcgaaatttg aacgagcagc agcc atg ccg ggc ttc tac gag atc 355
 Met Pro Gly Phe Tyr Glu Ile
 1               5

gtg atc aag gtg ccg agc gac ctg gac gag cac ctg ccg ggc att tct 403
Val Ile Lys Val Pro Ser Asp Leu Asp Glu His Leu Pro Gly Ile Ser
        10                  15                  20

gac tcg ttt gtg agc tgg gtg gcc gag aag gaa tgg gag ctg ccc ccg 451
Asp Ser Phe Val Ser Trp Val Ala Glu Lys Glu Trp Glu Leu Pro Pro
    25                  30                  35

gat tct gac atg gat ctg aat ctg att gag cag gca ccc ctg acc gtg 499
Asp Ser Asp Met Asp Leu Asn Leu Ile Glu Gln Ala Pro Leu Thr Val
40                  45                  50                  55

gcc gag aag ctg cag cgc gac ttc ctg gtc caa tgg cgc cgc gtg agt 547
Ala Glu Lys Leu Gln Arg Asp Phe Leu Val Gln Trp Arg Arg Val Ser
                60                  65                  70

aag gcc ccg gag gcc ctc ttc ttt gtt cag ttc gag aag ggc gag tcc 595

Lys Ala Pro Glu Ala Leu Phe Phe Val Gln Phe Glu Lys Gly Glu Ser
            75                  80                  85

tac ttc cac ctc cat att ctg gtg gag acc acg ggg gtc aaa tcc atg 643
Tyr Phe His Leu His Ile Leu Val Glu Thr Thr Gly Val Lys Ser Met
        90                  95                  100

gtg ctg ggc cgc ttc ctg agt cag att agg gac aag ctg gtg cag acc 691
Val Leu Gly Arg Phe Leu Ser Gln Ile Arg Asp Lys Leu Val Gln Thr
    105                 110                 115

atc tac cgc ggg atc gag ccg acc ctg ccc aac tgg ttc gcg gtg acc 739
Ile Tyr Arg Gly Ile Glu Pro Thr Leu Pro Asn Trp Phe Ala Val Thr
120                 125                 130                 135

aag acg cgt aat ggc gcc gga ggg ggg aac aag gtg gtg gac gag tgc 787
Lys Thr Arg Asn Gly Ala Gly Gly Gly Asn Lys Val Val Asp Glu Cys
                140                 145                 150

tac atc ccc aac tac ctc ctg ccc aag act cag ccc gag ctg cag tgg 835
Tyr Ile Pro Asn Tyr Leu Leu Pro Lys Thr Gln Pro Glu Leu Gln Trp
            155                 160                 165

gcg tgg act aac atg gag gag tat ata agc gcc tgt ttg aac ctg gcc 883
Ala Trp Thr Asn Met Glu Glu Tyr Ile Ser Ala Cys Leu Asn Leu Ala
        170                 175                 180

gag cgc aaa cgg ctc gtg gcg cag cac ctg acc cac gtc agc cag acc 931
Glu Arg Lys Arg Leu Val Ala Gln His Leu Thr His Val Ser Gln Thr
    185                 190                 195

cag gag cag aac aag gag aat ctg aac ccc aat tct gac gcg cct gtc 979
Gln Glu Gln Asn Lys Glu Asn Leu Asn Pro Asn Ser Asp Ala Pro Val
200                 205                 210                 215

atc cgg tca aaa acc tcc gcg cgc tac atg gag ctg gtc ggg tgg ctg 1027
Ile Arg Ser Lys Thr Ser Ala Arg Tyr Met Glu Leu Val Gly Trp Leu
                220                 225                 230

gtg gac cgg ggc atc acc tcc gag aag cag tgg atc cag gag gac cag 1075
Val Asp Arg Gly Ile Thr Ser Glu Lys Gln Trp Ile Gln Glu Asp Gln
            235                 240                 245

gcc tcg tac atc tcc ttc aac gcc gct tcc aac tcg cgg tcc cag atc 1123
Ala Ser Tyr Ile Ser Phe Asn Ala Ala Ser Asn Ser Arg Ser Gln Ile
        250                 255                 260

aag gcc gct ctg gac aat gcc ggc aag atc atg gcg ctg acc aaa tcc 1171
Lys Ala Ala Leu Asp Asn Ala Gly Lys Ile Met Ala Leu Thr Lys Ser
    265                 270                 275

gcg ccc gac tac ctg gta ggc ccc gct ccg ccc gcg gac att aaa acc 1219
Ala Pro Asp Tyr Leu Val Gly Pro Ala Pro Pro Ala Asp Ile Lys Thr
280                 285                 290                 295

aac cgc atc tac cgc atc ctg gag ctg aac ggc tac gaa cct gcc tac 1267
Asn Arg Ile Tyr Arg Ile Leu Glu Leu Asn Gly Tyr Glu Pro Ala Tyr
                300                 305                 310

gcc ggc tcc gtc ttt ctc ggc tgg gcc cag aaa agg ttc ggg aag cgc 1315
Ala Gly Ser Val Phe Leu Gly Trp Ala Gln Lys Arg Phe Gly Lys Arg
            315                 320                 325

aac acc atc tgg ctg ttt ggg ccg gcc acc acg ggc aag acc aac atc 1363
Asn Thr Ile Trp Leu Phe Gly Pro Ala Thr Thr Gly Lys Thr Asn Ile
        330                 335                 340

gcg gaa gcc atc gcc cac gcc gtg ccc ttc tac ggc tgc gtc aac tgg 1411

Ala Glu Ala Ile Ala His Ala Val Pro Phe Tyr Gly Cys Val Asn Trp
    345                 350                 355

acc aat gag aac ttt ccc ttc aat gat tgc gtc gac aag atg gtg atc      1459
Thr Asn Glu Asn Phe Pro Phe Asn Asp Cys Val Asp Lys Met Val Ile
360                 365                 370                 375

tgg tgg gag gag ggc aag atg acg gcc aag gtc gtg gag tcc gcc aag      1507
Trp Trp Glu Glu Gly Lys Met Thr Ala Lys Val Val Glu Ser Ala Lys
                380                 385                 390

gcc att ctc ggc ggc agc aag gtg cgc gtg gac caa aag tgc aag tcg      1555
Ala Ile Leu Gly Gly Ser Lys Val Arg Val Asp Gln Lys Cys Lys Ser
            395                 400                 405

tcc gcc cag atc gac ccc acc ccc gtg atc gtc acc tcc aac acc aac      1603
Ser Ala Gln Ile Asp Pro Thr Pro Val Ile Val Thr Ser Asn Thr Asn
        410                 415                 420

atg tgc gcc gtg att gac ggg aac agc acc acc ttc gag cac cag cag      1651
Met Cys Ala Val Ile Asp Gly Asn Ser Thr Thr Phe Glu His Gln Gln
    425                 430                 435

ccg ttg cag gac cgg atg ttc aaa ttt gaa ctc acc cgc cgt ctg gag      1699
Pro Leu Gln Asp Arg Met Phe Lys Phe Glu Leu Thr Arg Arg Leu Glu
440                 445                 450                 455

cat gac ttt ggc aag gtg aca aag cag gaa gtc aaa gag ttc ttc cgc      1747
His Asp Phe Gly Lys Val Thr Lys Gln Glu Val Lys Glu Phe Phe Arg
                460                 465                 470

tgg gcg cag gat cac gtg acc gag gtg gcg cat gag ttc tac gtc aga      1795
Trp Ala Gln Asp His Val Thr Glu Val Ala His Glu Phe Tyr Val Arg
            475                 480                 485

aag ggt gga gcc aac aaa aga ccc gcc ccc gat gac gcg gat aaa agc      1843
Lys Gly Gly Ala Asn Lys Arg Pro Ala Pro Asp Asp Ala Asp Lys Ser
        490                 495                 500

gag ccc aag cgg gcc tgc ccc tca gtc gcg gat cca tcg acg tca gac      1891
Glu Pro Lys Arg Ala Cys Pro Ser Val Ala Asp Pro Ser Thr Ser Asp
    505                 510                 515

gcg gaa gga gct ccg gtg gac ttt gcc gac agg tac caa aac aaa tgt      1939
Ala Glu Gly Ala Pro Val Asp Phe Ala Asp Arg Tyr Gln Asn Lys Cys
520                 525                 530                 535

tct cgt cac gcg ggc atg ctt cag atg ctg ttt ccc tgc aag aca tgc      1987
Ser Arg His Ala Gly Met Leu Gln Met Leu Phe Pro Cys Lys Thr Cys
                540                 545                 550

gag aga atg aat cag aat ttc aac att tgc ttc acg cac ggg acg aga      2035
Glu Arg Met Asn Gln Asn Phe Asn Ile Cys Phe Thr His Gly Thr Arg
            555                 560                 565

gac tgt tca gag tgc ttc ccc ggc gtg tca gaa tct caa ccg gtc gtc      2083
Asp Cys Ser Glu Cys Phe Pro Gly Val Ser Glu Ser Gln Pro Val Val
        570                 575                 580

aga aag agg acg tat cgg aaa ctc tgt gcc att cat cat ctg ctg ggg      2131
Arg Lys Arg Thr Tyr Arg Lys Leu Cys Ala Ile His His Leu Leu Gly
    585                 590                 595

cgg gct ccc gag att gct tgc tcg gcc tgc gat ctg gtc aac gtg gac      2179
Arg Ala Pro Glu Ile Ala Cys Ser Ala Cys Asp Leu Val Asn Val Asp
600                 605                 610                 615

ctg gat gac tgt gtt tct gag caa taa atgacttaaa ccaggt atg gct gcc    2231

Leu Asp Asp Cys Val Ser Glu Gln                              Met Ala Ala
                620                                                 625

gat ggt tat ctt cca gat tgg ctc gag gac aac ctc tct gag ggc att      2279
Asp Gly Tyr Leu Pro Asp Trp Leu Glu Asp Asn Leu Ser Glu Gly Ile
            630                 635                 640

cgc gag tgg tgg gac ttg aaa cct gga gcc ccg aag ccc aaa gcc aac      2327
Arg Glu Trp Trp Asp Leu Lys Pro Gly Ala Pro Lys Pro Lys Ala Asn
        645                 650                 655

cag caa aag cag gac gac ggc cgg ggt ctg gtg ctt cct ggc tac aag      2375
Gln Gln Lys Gln Asp Asp Gly Arg Gly Leu Val Leu Pro Gly Tyr Lys
    660                 665                 670

tac ctc gga ccc ttc aac gga ctc gac aag ggg gag ccc gtc aac gcg      2423
Tyr Leu Gly Pro Phe Asn Gly Leu Asp Lys Gly Glu Pro Val Asn Ala
675                 680                 685                 690

gcg gac gca gcg gcc ctc gag cac gac aag gcc tac gac cag cag ctc      2471
Ala Asp Ala Ala Ala Leu Glu His Asp Lys Ala Tyr Asp Gln Gln Leu
                695                 700                 705

aaa gcg ggt gac aat ccg tac ctg cgg tat aac cac gcc gac gcc gag      2519
Lys Ala Gly Asp Asn Pro Tyr Leu Arg Tyr Asn His Ala Asp Ala Glu
            710                 715                 720

ttt cag gag cgt ctg caa gaa gat acg tct ttt ggg ggc aac ctc ggg      2567
Phe Gln Glu Arg Leu Gln Glu Asp Thr Ser Phe Gly Gly Asn Leu Gly
        725                 730                 735

cga gca gtc ttc cag gcc aag aag cgg gtt ctc gaa cct ctc ggt ctg      2615
Arg Ala Val Phe Gln Ala Lys Lys Arg Val Leu Glu Pro Leu Gly Leu
    740                 745                 750

gtt gag gaa ggc gct aag acg gct cct gga aag aaa cgt ccg gta gag      2663
Val Glu Glu Gly Ala Lys Thr Ala Pro Gly Lys Lys Arg Pro Val Glu
755                 760                 765                 770

cag tcg cca caa gag cca gac tcc tcc tcg ggc atc ggc aag aca ggc      2711
Gln Ser Pro Gln Glu Pro Asp Ser Ser Ser Gly Ile Gly Lys Thr Gly
                775                 780                 785

cag cag ccc gct aaa aag aga ctc aat ttt ggt cag act ggc gac tca      2759
Gln Gln Pro Ala Lys Lys Arg Leu Asn Phe Gly Gln Thr Gly Asp Ser
            790                 795                 800

gag tca gtc ccc gat cca caa cct ctc gga gaa cct cca gca acc ccc      2807
Glu Ser Val Pro Asp Pro Gln Pro Leu Gly Glu Pro Pro Ala Thr Pro
        805                 810                 815

gct gct gtg gga cct act aca atg gct tca ggc ggt ggc gca cca atg      2855
Ala Ala Val Gly Pro Thr Thr Met Ala Ser Gly Gly Gly Ala Pro Met
    820                 825                 830

gca gac aat aac gaa ggc gcc gac gga gtg ggt aat gcc tca gga aat      2903
Ala Asp Asn Asn Glu Gly Ala Asp Gly Val Gly Asn Ala Ser Gly Asn
835                 840                 845                 850

tgg cat tgc gat tcc aca tgg ctg ggc gac aga gtc atc acc acc agc      2951
Trp His Cys Asp Ser Thr Trp Leu Gly Asp Arg Val Ile Thr Thr Ser
                855                 860                 865

acc cgc acc tgg gcc ttg ccc acc tac aat aac cac ctc tac aag caa      2999
Thr Arg Thr Trp Ala Leu Pro Thr Tyr Asn Asn His Leu Tyr Lys Gln
            870                 875                 880

atc tcc agt gct tca acg ggg gcc agc aac gac aac cac tac ttc ggc      3047

Ile Ser Ser Ala Ser Thr Gly Ala Ser Asn Asp Asn His Tyr Phe Gly
        885                 890                 895

tac agc acc ccc tgg ggg tat ttt gat ttc aac aga ttc cac tgc cac 3095
Tyr Ser Thr Pro Trp Gly Tyr Phe Asp Phe Asn Arg Phe His Cys His
    900                 905                 910

ttt tca cca cgt gac tgg cag cga ctc atc aac aac aat tgg gga ttc 3143
Phe Ser Pro Arg Asp Trp Gln Arg Leu Ile Asn Asn Asn Trp Gly Phe
915                 920                 925                 930

cgg ccc aag aga ctc aac ttc aaa ctc ttc aac atc caa gtc aag gag 3191
Arg Pro Lys Arg Leu Asn Phe Lys Leu Phe Asn Ile Gln Val Lys Glu
                935                 940                 945

gtc acg acg aat gat ggc gtc aca acc atc gct aat aac ctt acc agc 3239
Val Thr Thr Asn Asp Gly Val Thr Thr Ile Ala Asn Asn Leu Thr Ser
            950                 955                 960

acg gtt caa gtc ttc tcg gac tcg gag tac cag ctt ccg tac gtc ctc 3287
Thr Val Gln Val Phe Ser Asp Ser Glu Tyr Gln Leu Pro Tyr Val Leu
        965                 970                 975

ggc tct gcg cac cag ggc tgc ctc cct ccg ttc ccg gcg gac gtg ttc 3335
Gly Ser Ala His Gln Gly Cys Leu Pro Pro Phe Pro Ala Asp Val Phe
    980                 985                 990

atg att ccg caa tac ggc tac ctg acg ctc aac aat ggc agc caa 3380
Met Ile Pro Gln Tyr Gly Tyr Leu Thr Leu Asn Asn Gly Ser Gln
995                 1000                1005

gcc gtg gga cgt tca tcc ttt tac tgc ctg gaa tat ttc cct tct 3425
Ala Val Gly Arg Ser Ser Phe Tyr Cys Leu Glu Tyr Phe Pro Ser
1010                1015                1020

cag atg ctg aga acg ggc aac aac ttt acc ttc agc tac acc ttt 3470
Gln Met Leu Arg Thr Gly Asn Asn Phe Thr Phe Ser Tyr Thr Phe
1025                1030                1035

gag gaa gtg cct ttc cac agc agc tac gcg cac agc cag agc ctg 3515
Glu Glu Val Pro Phe His Ser Ser Tyr Ala His Ser Gln Ser Leu
1040                1045                1050

gac cgg ctg atg aat cct ctc atc gac caa tac ctg tat tac ctg 3560
Asp Arg Leu Met Asn Pro Leu Ile Asp Gln Tyr Leu Tyr Tyr Leu
1055                1060                1065

aac aga act caa aat cag tcc gga agt gcc caa aac aag gac ttg 3605
Asn Arg Thr Gln Asn Gln Ser Gly Ser Ala Gln Asn Lys Asp Leu
1070                1075                1080

ctg ttt agc cgt ggg tct cca gct ggc atg tct gtt cag ccc aaa 3650
Leu Phe Ser Arg Gly Ser Pro Ala Gly Met Ser Val Gln Pro Lys
1085                1090                1095

aac tgg cta cct gga ccc tgt tat cgg cag cag cgc gtt tct aaa 3695
Asn Trp Leu Pro Gly Pro Cys Tyr Arg Gln Gln Arg Val Ser Lys
1100                1105                1110

aca aaa aca gac aac aac aac agc aat ttt acc tgg act ggt gct 3740
Thr Lys Thr Asp Asn Asn Asn Ser Asn Phe Thr Trp Thr Gly Ala
1115                1120                1125

tca aaa tat aac ctc aat ggg cgt gaa tcc atc atc aac cct ggc 3785
Ser Lys Tyr Asn Leu Asn Gly Arg Glu Ser Ile Ile Asn Pro Gly
1130                1135                1140

act gct atg gcc tca cac aaa gac gac gaa gac aag ttc ttt ccc 3830

Thr Ala Met Ala Ser His Lys Asp Asp Glu Asp Lys Phe Phe Pro
1145                1150                1155

atg agc ggt gtc atg att ttt gga aaa gag agc gcc gga gct tca 3875
Met Ser Gly Val Met Ile Phe Gly Lys Glu Ser Ala Gly Ala Ser
1160                1165                1170

aac act gca ttg gac aat gtc atg att aca gac gaa gag gaa att 3920
Asn Thr Ala Leu Asp Asn Val Met Ile Thr Asp Glu Glu Glu Ile
1175                1180                1185

aaa gcc act aac cct gtg gcc acc gaa aga ttt ggg acc gtg gca 3965
Lys Ala Thr Asn Pro Val Ala Thr Glu Arg Phe Gly Thr Val Ala
1190                1195                1200

gtc aat ttc cag agc agc agc aca gac cct gcg acc gga gat gtg 4010
Val Asn Phe Gln Ser Ser Ser Thr Asp Pro Ala Thr Gly Asp Val
1205                1210                1215

cat gct atg gga gca tta cct ggc atg gtg tgg caa gat aga gac 4055
His Ala Met Gly Ala Leu Pro Gly Met Val Trp Gln Asp Arg Asp
1220                1225                1230

gtg tac ctg cag ggt ccc att tgg gcc aaa att cct cac aca gat 4100
Val Tyr Leu Gln Gly Pro Ile Trp Ala Lys Ile Pro His Thr Asp
1235                1240                1245

gga cac ttt cac ccg tct cct ctt atg ggc ggc ttt gga ctc aag 4145
Gly His Phe His Pro Ser Pro Leu Met Gly Gly Phe Gly Leu Lys
1250                1255                1260

aac ccg cct cct cag atc ctc atc aaa aac acg cct gtt cct gcg 4190
Asn Pro Pro Pro Gln Ile Leu Ile Lys Asn Thr Pro Val Pro Ala
1265                1270                1275

aat cct ccg gcg gag ttt tca gct aca aag ttt gct tca ttc atc 4235
Asn Pro Pro Ala Glu Phe Ser Ala Thr Lys Phe Ala Ser Phe Ile
1280                1285                1290

acc caa tac tcc aca gga caa gtg agt gtg gaa att gaa tgg gag 4280
Thr Gln Tyr Ser Thr Gly Gln Val Ser Val Glu Ile Glu Trp Glu
1295                1300                1305

ctg cag aaa gaa aac agc aag cgc tgg aat ccc gaa gtg cag tac 4325
Leu Gln Lys Glu Asn Ser Lys Arg Trp Asn Pro Glu Val Gln Tyr
1310                1315                1320

aca tcc aat tat gca aaa tct gcc aac gtt gat ttt act gtg gac 4370
Thr Ser Asn Tyr Ala Lys Ser Ala Asn Val Asp Phe Thr Val Asp
1325                1330                1335

aac aat gga ctt tat act gag cct cgc ccc att ggc acc cgt tac 4415
Asn Asn Gly Leu Tyr Thr Glu Pro Arg Pro Ile Gly Thr Arg Tyr
1340                1345                1350

ctt acc cgt ccc ctg taattacgtg ttaatcaata aaccggttga ttcgtttcag 4470
Leu Thr Arg Pro Leu
1355

ttgaactttg gtctcctgtc cttcttatct tatcggttac catggttata gcttacacat 4530

taactgcttg gttgcgcttc gcgataaaag acttacgtca tcgggttacc cctagtgatg 4590

gagttgccca ctccctctct gcgcgctcgc tcgctcggtg gggcctgcgg accaaaggtc 4650

cgcagacggc agagctctgc tctgccggcc ccaccgagcg agcgagcgcg cagagaggga 4710

gtgggcaa 4718

<210>  2
<211>  623
<212>  PRT
<213>  AAV-1

<400>  2
Met Pro Gly Phe Tyr Glu Ile Val Ile Lys Val Pro Ser Asp Leu Asp
1               5                   10                  15

Glu His Leu Pro Gly Ile Ser Asp Ser Phe Val Ser Trp Val Ala Glu
            20                  25                  30

Lys Glu Trp Glu Leu Pro Pro Asp Ser Asp Met Asp Leu Asn Leu Ile
        35                  40                  45

Glu Gln Ala Pro Leu Thr Val Ala Glu Lys Leu Gln Arg Asp Phe Leu
    50                  55                  60

Val Gln Trp Arg Arg Val Ser Lys Ala Pro Glu Ala Leu Phe Phe Val
65                  70                  75                  80

Gln Phe Glu Lys Gly Glu Ser Tyr Phe His Leu His Ile Leu Val Glu
                85                  90                  95

Thr Thr Gly Val Lys Ser Met Val Leu Gly Arg Phe Leu Ser Gln Ile
            100                 105                 110

Arg Asp Lys Leu Val Gln Thr Ile Tyr Arg Gly Ile Glu Pro Thr Leu
        115                 120                 125

Pro Asn Trp Phe Ala Val Thr Lys Thr Arg Asn Gly Ala Gly Gly Gly
    130                 135                 140

Asn Lys Val Val Asp Glu Cys Tyr Ile Pro Asn Tyr Leu Leu Pro Lys
145                 150                 155                 160

Thr Gln Pro Glu Leu Gln Trp Ala Trp Thr Asn Met Glu Glu Tyr Ile
                165                 170                 175

Ser Ala Cys Leu Asn Leu Ala Glu Arg Lys Arg Leu Val Ala Gln His
            180                 185                 190

Leu Thr His Val Ser Gln Thr Gln Glu Gln Asn Lys Glu Asn Leu Asn
        195                 200                 205

Pro Asn Ser Asp Ala Pro Val Ile Arg Ser Lys Thr Ser Ala Arg Tyr
    210                 215                 220

Met Glu Leu Val Gly Trp Leu Val Asp Arg Gly Ile Thr Ser Glu Lys
225                 230                 235                 240

Gln Trp Ile Gln Glu Asp Gln Ala Ser Tyr Ile Ser Phe Asn Ala Ala
                245                 250                 255

Ser Asn Ser Arg Ser Gln Ile Lys Ala Ala Leu Asp Asn Ala Gly Lys
            260                 265                 270

Ile Met Ala Leu Thr Lys Ser Ala Pro Asp Tyr Leu Val Gly Pro Ala
        275                 280                 285

Pro Pro Ala Asp Ile Lys Thr Asn Arg Ile Tyr Arg Ile Leu Glu Leu
    290                 295                 300

Asn Gly Tyr Glu Pro Ala Tyr Ala Gly Ser Val Phe Leu Gly Trp Ala
305                 310                 315                 320

Gln Lys Arg Phe Gly Lys Arg Asn Thr Ile Trp Leu Phe Gly Pro Ala
                325                 330                 335

Thr Thr Gly Lys Thr Asn Ile Ala Glu Ala Ile Ala His Ala Val Pro
            340                 345                 350

Phe Tyr Gly Cys Val Asn Trp Thr Asn Glu Asn Phe Pro Phe Asn Asp
        355                 360                 365

Cys Val Asp Lys Met Val Ile Trp Trp Glu Glu Gly Lys Met Thr Ala
    370                 375                 380

Lys Val Val Glu Ser Ala Lys Ala Ile Leu Gly Gly Ser Lys Val Arg
385                 390                 395                 400

Val Asp Gln Lys Cys Lys Ser Ser Ala Gln Ile Asp Pro Thr Pro Val
                405                 410                 415

Ile Val Thr Ser Asn Thr Asn Met Cys Ala Val Ile Asp Gly Asn Ser
            420                 425                 430

Thr Thr Phe Glu His Gln Gln Pro Leu Gln Asp Arg Met Phe Lys Phe
        435                 440                 445

Glu Leu Thr Arg Arg Leu Glu His Asp Phe Gly Lys Val Thr Lys Gln
    450                 455                 460

Glu Val Lys Glu Phe Phe Arg Trp Ala Gln Asp His Val Thr Glu Val
465                 470                 475                 480

Ala His Glu Phe Tyr Val Arg Lys Gly Gly Ala Asn Lys Arg Pro Ala
                485                 490                 495

Pro Asp Asp Ala Asp Lys Ser Glu Pro Lys Arg Ala Cys Pro Ser Val
            500                 505                 510

Ala Asp Pro Ser Thr Ser Asp Ala Glu Gly Ala Pro Val Asp Phe Ala
        515                 520                 525

Asp Arg Tyr Gln Asn Lys Cys Ser Arg His Ala Gly Met Leu Gln Met
    530                 535                 540

Leu Phe Pro Cys Lys Thr Cys Glu Arg Met Asn Gln Asn Phe Asn Ile
545                 550                 555                 560

Cys Phe Thr His Gly Thr Arg Asp Cys Ser Glu Cys Phe Pro Gly Val
                565                 570                 575

Ser Glu Ser Gln Pro Val Val Arg Lys Arg Thr Tyr Arg Lys Leu Cys
            580                 585                 590

Ala Ile His His Leu Leu Gly Arg Ala Pro Glu Ile Ala Cys Ser Ala
        595                 600                 605

Cys Asp Leu Val Asn Val Asp Leu Asp Asp Cys Val Ser Glu Gln
    610                 615                 620

<210>  3
<211>  736
<212>  PRT
<213>  AAV-1

<400>  3
Met Ala Ala Asp Gly Tyr Leu Pro Asp Trp Leu Glu Asp Asn Leu Ser
1               5                   10                  15

Glu Gly Ile Arg Glu Trp Trp Asp Leu Lys Pro Gly Ala Pro Lys Pro

20                  25                  30

Lys Ala Asn Gln Gln Lys Gln Asp Asp Gly Arg Gly Leu Val Leu Pro
        35                  40                  45

Gly Tyr Lys Tyr Leu Gly Pro Phe Asn Gly Leu Asp Lys Gly Glu Pro
    50                  55                  60

Val Asn Ala Ala Asp Ala Ala Ala Leu Glu His Asp Lys Ala Tyr Asp
65                  70                  75                  80

Gln Gln Leu Lys Ala Gly Asp Asn Pro Tyr Leu Arg Tyr Asn His Ala
                85                  90                  95

Asp Ala Glu Phe Gln Glu Arg Leu Gln Glu Asp Thr Ser Phe Gly Gly
            100                 105                 110

Asn Leu Gly Arg Ala Val Phe Gln Ala Lys Lys Arg Val Leu Glu Pro
        115                 120                 125

Leu Gly Leu Val Glu Glu Gly Ala Lys Thr Ala Pro Gly Lys Lys Arg
    130                 135                 140

Pro Val Glu Gln Ser Pro Gln Glu Pro Asp Ser Ser Ser Gly Ile Gly
145                 150                 155                 160

Lys Thr Gly Gln Gln Pro Ala Lys Lys Arg Leu Asn Phe Gly Gln Thr
                165                 170                 175

Gly Asp Ser Glu Ser Val Pro Asp Pro Gln Pro Leu Gly Glu Pro Pro
            180                 185                 190

Ala Thr Pro Ala Ala Val Gly Pro Thr Thr Met Ala Ser Gly Gly Gly
        195                 200                 205

Ala Pro Met Ala Asp Asn Asn Glu Gly Ala Asp Gly Val Gly Asn Ala
    210                 215                 220

Ser Gly Asn Trp His Cys Asp Ser Thr Trp Leu Gly Asp Arg Val Ile
225                 230                 235                 240

Thr Thr Ser Thr Arg Thr Trp Ala Leu Pro Thr Tyr Asn Asn His Leu
                245                 250                 255

Tyr Lys Gln Ile Ser Ser Ala Ser Thr Gly Ala Ser Asn Asp Asn His
            260                 265                 270

Tyr Phe Gly Tyr Ser Thr Pro Trp Gly Tyr Phe Asp Phe Asn Arg Phe
        275                 280                 285

His Cys His Phe Ser Pro Arg Asp Trp Gln Arg Leu Ile Asn Asn Asn
    290                 295                 300

Trp Gly Phe Arg Pro Lys Arg Leu Asn Phe Lys Leu Phe Asn Ile Gln
305                 310                 315                 320

Val Lys Glu Val Thr Thr Asn Asp Gly Val Thr Thr Ile Ala Asn Asn
                325                 330                 335

Leu Thr Ser Thr Val Gln Val Phe Ser Asp Ser Glu Tyr Gln Leu Pro
            340                 345                 350

Tyr Val Leu Gly Ser Ala His Gln Gly Cys Leu Pro Pro Phe Pro Ala
        355                 360                 365

Asp Val Phe Met Ile Pro Gln Tyr Gly Tyr Leu Thr Leu Asn Asn Gly
    370                 375                 380

Ser Gln Ala Val Gly Arg Ser Ser Phe Tyr Cys Leu Glu Tyr Phe Pro
385                 390                 395                 400

Ser Gln Met Leu Arg Thr Gly Asn Asn Phe Thr Phe Ser Tyr Thr Phe
                405                 410                 415

Glu Glu Val Pro Phe His Ser Ser Tyr Ala His Ser Gln Ser Leu Asp
            420                 425                 430

Arg Leu Met Asn Pro Leu Ile Asp Gln Tyr Leu Tyr Tyr Leu Asn Arg
        435                 440                 445

Thr Gln Asn Gln Ser Gly Ser Ala Gln Asn Lys Asp Leu Leu Phe Ser
    450                 455                 460

Arg Gly Ser Pro Ala Gly Met Ser Val Gln Pro Lys Asn Trp Leu Pro
465                 470                 475                 480

Gly Pro Cys Tyr Arg Gln Gln Arg Val Ser Lys Thr Lys Thr Asp Asn
                485                 490                 495

Asn Asn Ser Asn Phe Thr Trp Thr Gly Ala Ser Lys Tyr Asn Leu Asn
            500                 505                 510

Gly Arg Glu Ser Ile Ile Asn Pro Gly Thr Ala Met Ala Ser His Lys
        515                 520                 525

Asp Asp Glu Asp Lys Phe Phe Pro Met Ser Gly Val Met Ile Phe Gly
    530                 535                 540

Lys Glu Ser Ala Gly Ala Ser Asn Thr Ala Leu Asp Asn Val Met Ile
545                 550                 555                 560

Thr Asp Glu Glu Glu Ile Lys Ala Thr Asn Pro Val Ala Thr Glu Arg
                565                 570                 575

Phe Gly Thr Val Ala Val Asn Phe Gln Ser Ser Ser Thr Asp Pro Ala
            580                 585                 590

Thr Gly Asp Val His Ala Met Gly Ala Leu Pro Gly Met Val Trp Gln
        595                 600                 605

Asp Arg Asp Val Tyr Leu Gln Gly Pro Ile Trp Ala Lys Ile Pro His
    610                 615                 620

Thr Asp Gly His Phe His Pro Ser Pro Leu Met Gly Gly Phe Gly Leu
625                 630                 635                 640

Lys Asn Pro Pro Pro Gln Ile Leu Ile Lys Asn Thr Pro Val Pro Ala
                645                 650                 655

Asn Pro Pro Ala Glu Phe Ser Ala Thr Lys Phe Ala Ser Phe Ile Thr
            660                 665                 670

Gln Tyr Ser Thr Gly Gln Val Ser Val Glu Ile Glu Trp Glu Leu Gln
        675                 680                 685

Lys Glu Asn Ser Lys Arg Trp Asn Pro Glu Val Gln Tyr Thr Ser Asn
    690                 695                 700

Tyr Ala Lys Ser Ala Asn Val Asp Phe Thr Val Asp Asn Asn Gly Leu
705                 710                 715                 720

Tyr Thr Glu Pro Arg Pro Ile Gly Thr Arg Tyr Leu Thr Arg Pro Leu
                725                 730                 735

<210>  4

<211>  1872
<212>  DNA
<213>  AAV-1

<220>
<221>  CDS
<222>  (1)..(1869)
<223>

<400>  4
atg ccg ggc ttc tac gag atc gtg atc aag gtg ccg agc gac ctg gac      48
Met Pro Gly Phe Tyr Glu Ile Val Ile Lys Val Pro Ser Asp Leu Asp
1               5                   10                  15

gag cac ctg ccg ggc att tct gac tcg ttt gtg agc tgg gtg gcc gag      96
Glu His Leu Pro Gly Ile Ser Asp Ser Phe Val Ser Trp Val Ala Glu
            20                  25                  30

aag gaa tgg gag ctg ccc ccg gat tct gac atg gat ctg aat ctg att      144
Lys Glu Trp Glu Leu Pro Pro Asp Ser Asp Met Asp Leu Asn Leu Ile
        35                  40                  45

gag cag gca ccc ctg acc gtg gcc gag aag ctg cag cgc gac ttc ctg      192
Glu Gln Ala Pro Leu Thr Val Ala Glu Lys Leu Gln Arg Asp Phe Leu
    50                  55                  60

gtc caa tgg cgc cgc gtg agt aag gcc ccg gag gcc ctc ttc ttt gtt      240
Val Gln Trp Arg Arg Val Ser Lys Ala Pro Glu Ala Leu Phe Phe Val
65                  70                  75                  80

cag ttc gag aag ggc gag tcc tac ttc cac ctc cat att ctg gtg gag      288
Gln Phe Glu Lys Gly Glu Ser Tyr Phe His Leu His Ile Leu Val Glu
                85                  90                  95

acc acg ggg gtc aaa tcc atg gtg ctg ggc cgc ttc ctg agt cag att      336
Thr Thr Gly Val Lys Ser Met Val Leu Gly Arg Phe Leu Ser Gln Ile
            100                 105                 110

agg gac aag ctg gtg cag acc atc tac cgc ggg atc gag ccg acc ctg      384
Arg Asp Lys Leu Val Gln Thr Ile Tyr Arg Gly Ile Glu Pro Thr Leu
        115                 120                 125

ccc aac tgg ttc gcg gtg acc aag acg cgt aat ggc gcc gga ggg ggg      432
Pro Asn Trp Phe Ala Val Thr Lys Thr Arg Asn Gly Ala Gly Gly Gly
    130                 135                 140

aac aag gtg gtg gac gag tgc tac atc ccc aac tac ctc ctg ccc aag      480
Asn Lys Val Val Asp Glu Cys Tyr Ile Pro Asn Tyr Leu Leu Pro Lys
145                 150                 155                 160

act cag ccc gag ctg cag tgg gcg tgg act aac atg gag gag tat ata      528
Thr Gln Pro Glu Leu Gln Trp Ala Trp Thr Asn Met Glu Glu Tyr Ile
                165                 170                 175

agc gcc tgt ttg aac ctg gcc gag cgc aaa cgg ctc gtg gcg cag cac      576
Ser Ala Cys Leu Asn Leu Ala Glu Arg Lys Arg Leu Val Ala Gln His
            180                 185                 190

ctg acc cac gtc agc cag acc cag gag cag aac aag gag aat ctg aac      624
Leu Thr His Val Ser Gln Thr Gln Glu Gln Asn Lys Glu Asn Leu Asn
        195                 200                 205

ccc aat tct gac gcg cct gtc atc cgg tca aaa acc tcc gcg cgc tac      672
Pro Asn Ser Asp Ala Pro Val Ile Arg Ser Lys Thr Ser Ala Arg Tyr
    210                 215                 220

atg gag ctg gtc ggg tgg ctg gtg gac cgg ggc atc acc tcc gag aag      720
Met Glu Leu Val Gly Trp Leu Val Asp Arg Gly Ile Thr Ser Glu Lys

225                 230                 235                 240

cag tgg atc cag gag gac cag gcc tcg tac atc tcc ttc aac gcc gct      768
Gln Trp Ile Gln Glu Asp Gln Ala Ser Tyr Ile Ser Phe Asn Ala Ala
                245                 250                 255

tcc aac tcg cgg tcc cag atc aag gcc gct ctg gac aat gcc ggc aag      816
Ser Asn Ser Arg Ser Gln Ile Lys Ala Ala Leu Asp Asn Ala Gly Lys
            260                 265                 270

atc atg gcg ctg acc aaa tcc gcg ccc gac tac ctg gta ggc ccc gct      864
Ile Met Ala Leu Thr Lys Ser Ala Pro Asp Tyr Leu Val Gly Pro Ala
        275                 280                 285

ccg ccc gcg gac att aaa acc aac cgc atc tac cgc atc ctg gag ctg      912
Pro Pro Ala Asp Ile Lys Thr Asn Arg Ile Tyr Arg Ile Leu Glu Leu
    290                 295                 300

aac ggc tac gaa cct gcc tac gcc ggc tcc gtc ttt ctc ggc tgg gcc      960
Asn Gly Tyr Glu Pro Ala Tyr Ala Gly Ser Val Phe Leu Gly Trp Ala
305                 310                 315                 320

cag aaa agg ttc ggg aag cgc aac acc atc tgg ctg ttt ggg ccg gcc     1008
Gln Lys Arg Phe Gly Lys Arg Asn Thr Ile Trp Leu Phe Gly Pro Ala
                325                 330                 335

acc acg ggc aag acc aac atc gcg gaa gcc atc gcc cac gcc gtg ccc     1056
Thr Thr Gly Lys Thr Asn Ile Ala Glu Ala Ile Ala His Ala Val Pro
            340                 345                 350

ttc tac ggc tgc gtc aac tgg acc aat gag aac ttt ccc ttc aat gat     1104
Phe Tyr Gly Cys Val Asn Trp Thr Asn Glu Asn Phe Pro Phe Asn Asp
        355                 360                 365

tgc gtc gac aag atg gtg atc tgg tgg gag gag ggc aag atg acg gcc     1152
Cys Val Asp Lys Met Val Ile Trp Trp Glu Glu Gly Lys Met Thr Ala
    370                 375                 380

aag gtc gtg gag tcc gcc aag gcc att ctc ggc ggc agc aag gtg cgc     1200
Lys Val Val Glu Ser Ala Lys Ala Ile Leu Gly Gly Ser Lys Val Arg
385                 390                 395                 400

gtg gac caa aag tgc aag tcg tcc gcc cag atc gac ccc acc ccc gtg     1248
Val Asp Gln Lys Cys Lys Ser Ser Ala Gln Ile Asp Pro Thr Pro Val
                405                 410                 415

atc gtc acc tcc aac acc aac atg tgc gcc gtg att gac ggg aac agc     1296
Ile Val Thr Ser Asn Thr Asn Met Cys Ala Val Ile Asp Gly Asn Ser
            420                 425                 430

acc acc ttc gag cac cag cag ccg ttg cag gac cgg atg ttc aaa ttt     1344
Thr Thr Phe Glu His Gln Gln Pro Leu Gln Asp Arg Met Phe Lys Phe
        435                 440                 445

gaa ctc acc cgc cgt ctg gag cat gac ttt ggc aag gtg aca aag cag     1392
Glu Leu Thr Arg Arg Leu Glu His Asp Phe Gly Lys Val Thr Lys Gln
    450                 455                 460

gaa gtc aaa gag ttc ttc cgc tgg gcg cag gat cac gtg acc gag gtg     1440
Glu Val Lys Glu Phe Phe Arg Trp Ala Gln Asp His Val Thr Glu Val
465                 470                 475                 480

gcg cat gag ttc tac gtc aga aag ggt gga gcc aac aaa aga ccc gcc     1488
Ala His Glu Phe Tyr Val Arg Lys Gly Gly Ala Asn Lys Arg Pro Ala
                485                 490                 495

ccc gat gac gcg gat aaa agc gag ccc aag cgg gcc tgc ccc tca gtc     1536
Pro Asp Asp Ala Asp Lys Ser Glu Pro Lys Arg Ala Cys Pro Ser Val

                  500                     505                     510

gcg gat cca tcg acg tca gac gcg gaa gga gct ccg gtg gac ttt gcc      1584
Ala Asp Pro Ser Thr Ser Asp Ala Glu Gly Ala Pro Val Asp Phe Ala
            515                     520                     525

gac agg tac caa aac aaa tgt tct cgt cac gcg ggc atg ctt cag atg      1632
Asp Arg Tyr Gln Asn Lys Cys Ser Arg His Ala Gly Met Leu Gln Met
    530                     535                     540

ctg ttt ccc tgc aag aca tgc gag aga atg aat cag aat ttc aac att      1680
Leu Phe Pro Cys Lys Thr Cys Glu Arg Met Asn Gln Asn Phe Asn Ile
545                     550                     555                     560

tgc ttc acg cac ggg acg aga gac tgt tca gag tgc ttc ccc ggc gtg      1728
Cys Phe Thr His Gly Thr Arg Asp Cys Ser Glu Cys Phe Pro Gly Val
                565                     570                     575

tca gaa tct caa ccg gtc gtc aga aag agg acg tat cgg aaa ctc tgt      1776
Ser Glu Ser Gln Pro Val Val Arg Lys Arg Thr Tyr Arg Lys Leu Cys
            580                     585                     590

gcc att cat cat ctg ctg ggg cgg gct ccc gag att gct tgc tcg gcc      1824
Ala Ile His His Leu Leu Gly Arg Ala Pro Glu Ile Ala Cys Ser Ala
        595                     600                     605

tgc gat ctg gtc aac gtg gac ctg gat gac tgt gtt tct gag caa taa      1872
Cys Asp Leu Val Asn Val Asp Leu Asp Asp Cys Val Ser Glu Gln
    610                     615                     620


<210>  5
<211>  623
<212>  PRT
<213>  AAV-1

<400>  5
Met Pro Gly Phe Tyr Glu Ile Val Ile Lys Val Pro Ser Asp Leu Asp
1               5                   10                  15

Glu His Leu Pro Gly Ile Ser Asp Ser Phe Val Ser Trp Val Ala Glu
            20                  25                  30

Lys Glu Trp Glu Leu Pro Pro Asp Ser Asp Met Asp Leu Asn Leu Ile
        35                  40                  45

Glu Gln Ala Pro Leu Thr Val Ala Glu Lys Leu Gln Arg Asp Phe Leu
    50                  55                  60

Val Gln Trp Arg Arg Val Ser Lys Ala Pro Glu Ala Leu Phe Phe Val
65                  70                  75                  80

Gln Phe Glu Lys Gly Glu Ser Tyr Phe His Leu His Ile Leu Val Glu
                85                  90                  95

Thr Thr Gly Val Lys Ser Met Val Leu Gly Arg Phe Leu Ser Gln Ile
            100                 105                 110

Arg Asp Lys Leu Val Gln Thr Ile Tyr Arg Gly Ile Glu Pro Thr Leu
        115                 120                 125

Pro Asn Trp Phe Ala Val Thr Lys Thr Arg Asn Gly Ala Gly Gly Gly
    130                 135                 140

Asn Lys Val Val Asp Glu Cys Tyr Ile Pro Asn Tyr Leu Leu Pro Lys
145                 150                 155                 160

Thr Gln Pro Glu Leu Gln Trp Ala Trp Thr Asn Met Glu Glu Tyr Ile

                165                 170                 175

Ser Ala Cys Leu Asn Leu Ala Glu Arg Lys Arg Leu Val Ala Gln His
            180                 185                 190

Leu Thr His Val Ser Gln Thr Gln Glu Gln Asn Lys Glu Asn Leu Asn
        195                 200                 205

Pro Asn Ser Asp Ala Pro Val Ile Arg Ser Lys Thr Ser Ala Arg Tyr
    210                 215                 220

Met Glu Leu Val Gly Trp Leu Val Asp Arg Gly Ile Thr Ser Glu Lys
225                 230                 235                 240

Gln Trp Ile Gln Glu Asp Gln Ala Ser Tyr Ile Ser Phe Asn Ala Ala
                245                 250                 255

Ser Asn Ser Arg Ser Gln Ile Lys Ala Ala Leu Asp Asn Ala Gly Lys
            260                 265                 270

Ile Met Ala Leu Thr Lys Ser Ala Pro Asp Tyr Leu Val Gly Pro Ala
        275                 280                 285

Pro Pro Ala Asp Ile Lys Thr Asn Arg Ile Tyr Arg Ile Leu Glu Leu
    290                 295                 300

Asn Gly Tyr Glu Pro Ala Tyr Ala Gly Ser Val Phe Leu Gly Trp Ala
305                 310                 315                 320

Gln Lys Arg Phe Gly Lys Arg Asn Thr Ile Trp Leu Phe Gly Pro Ala
                325                 330                 335

Thr Thr Gly Lys Thr Asn Ile Ala Glu Ala Ile Ala His Ala Val Pro
            340                 345                 350

Phe Tyr Gly Cys Val Asn Trp Thr Asn Glu Asn Phe Pro Phe Asn Asp
        355                 360                 365

Cys Val Asp Lys Met Val Ile Trp Trp Glu Glu Gly Lys Met Thr Ala
    370                 375                 380

Lys Val Val Glu Ser Ala Lys Ala Ile Leu Gly Gly Ser Lys Val Arg
385                 390                 395                 400

Val Asp Gln Lys Cys Lys Ser Ser Ala Gln Ile Asp Pro Thr Pro Val
                405                 410                 415

Ile Val Thr Ser Asn Thr Asn Met Cys Ala Val Ile Asp Gly Asn Ser
            420                 425                 430

Thr Thr Phe Glu His Gln Gln Pro Leu Gln Asp Arg Met Phe Lys Phe
        435                 440                 445

Glu Leu Thr Arg Arg Leu Glu His Asp Phe Gly Lys Val Thr Lys Gln
    450                 455                 460

Glu Val Lys Glu Phe Phe Arg Trp Ala Gln Asp His Val Thr Glu Val
465                 470                 475                 480

Ala His Glu Phe Tyr Val Arg Lys Gly Gly Ala Asn Lys Arg Pro Ala
                485                 490                 495

Pro Asp Asp Ala Asp Lys Ser Glu Pro Lys Arg Ala Cys Pro Ser Val
            500                 505                 510

Ala Asp Pro Ser Thr Ser Asp Ala Glu Gly Ala Pro Val Asp Phe Ala
        515                 520                 525

Asp Arg Tyr Gln Asn Lys Cys Ser Arg His Ala Gly Met Leu Gln Met
    530                 535                 540

Leu Phe Pro Cys Lys Thr Cys Glu Arg Met Asn Gln Asn Phe Asn Ile
545                 550                 555                 560

Cys Phe Thr His Gly Thr Arg Asp Cys Ser Glu Cys Phe Pro Gly Val
                565                 570                 575

Ser Glu Ser Gln Pro Val Val Arg Lys Arg Thr Tyr Arg Lys Leu Cys
            580                 585                 590

Ala Ile His His Leu Leu Gly Arg Ala Pro Glu Ile Ala Cys Ser Ala
        595                 600                 605

Cys Asp Leu Val Asn Val Asp Leu Asp Asp Cys Val Ser Glu Gln
    610                 615                 620


<210>  6
<211>  1641
<212>  DNA
<213>  AAV-1

<220>
<221>  CDS
<222>  (1)..(1638)
<223>

 <400>  6
atg ccg ggc ttc tac gag atc gtg atc aag gtg ccg agc gac ctg gac      48
Met Pro Gly Phe Tyr Glu Ile Val Ile Lys Val Pro Ser Asp Leu Asp
1               5                   10                  15

gag cac ctg ccg ggc att tct gac tcg ttt gtg agc tgg gtg gcc gag      96
Glu His Leu Pro Gly Ile Ser Asp Ser Phe Val Ser Trp Val Ala Glu
            20                  25                  30

aag gaa tgg gag ctg ccc ccg gat tct gac atg gat ctg aat ctg att     144
Lys Glu Trp Glu Leu Pro Pro Asp Ser Asp Met Asp Leu Asn Leu Ile
        35                  40                  45

gag cag gca ccc ctg acc gtg gcc gag aag ctg cag cgc gac ttc ctg     192
Glu Gln Ala Pro Leu Thr Val Ala Glu Lys Leu Gln Arg Asp Phe Leu
    50                  55                  60

gtc caa tgg cgc cgc gtg agt aag gcc ccg gag gcc ctc ttc ttt gtt     240
Val Gln Trp Arg Arg Val Ser Lys Ala Pro Glu Ala Leu Phe Phe Val
65                  70                  75                  80

cag ttc gag aag ggc gag tcc tac ttc cac ctc cat att ctg gtg gag     288
Gln Phe Glu Lys Gly Glu Ser Tyr Phe His Leu His Ile Leu Val Glu
                85                  90                  95

acc acg ggg gtc aaa tcc atg gtg ctg ggc cgc ttc ctg agt cag att     336
Thr Thr Gly Val Lys Ser Met Val Leu Gly Arg Phe Leu Ser Gln Ile
            100                 105                 110

agg gac aag ctg gtg cag acc atc tac cgc ggg atc gag ccg acc ctg     384
Arg Asp Lys Leu Val Gln Thr Ile Tyr Arg Gly Ile Glu Pro Thr Leu
        115                 120                 125

ccc aac tgg ttc gcg gtg acc aag acg cgt aat ggc gcc gga ggg ggg     432
Pro Asn Trp Phe Ala Val Thr Lys Thr Arg Asn Gly Ala Gly Gly Gly
    130                 135                 140

aac aag gtg gtg gac gag tgc tac atc ccc aac tac ctc ctg ccc aag     480
Asn Lys Val Val Asp Glu Cys Tyr Ile Pro Asn Tyr Leu Leu Pro Lys

145                 150                 155                 160

act cag ccc gag ctg cag tgg gcg tgg act aac atg gag gag tat ata      528
Thr Gln Pro Glu Leu Gln Trp Ala Trp Thr Asn Met Glu Glu Tyr Ile
                165                 170                 175

agc gcc tgt ttg aac ctg gcc gag cgc aaa cgg ctc gtg gcg cag cac      576
Ser Ala Cys Leu Asn Leu Ala Glu Arg Lys Arg Leu Val Ala Gln His
            180                 185                 190

ctg acc cac gtc agc cag acc cag gag cag aac aag gag aat ctg aac      624
Leu Thr His Val Ser Gln Thr Gln Glu Gln Asn Lys Glu Asn Leu Asn
        195                 200                 205

ccc aat tct gac gcg cct gtc atc cgg tca aaa acc tcc gcg cgc tac      672
Pro Asn Ser Asp Ala Pro Val Ile Arg Ser Lys Thr Ser Ala Arg Tyr
    210                 215                 220

atg gag ctg gtc ggg tgg ctg gtg gac cgg ggc atc acc tcc gag aag      720
Met Glu Leu Val Gly Trp Leu Val Asp Arg Gly Ile Thr Ser Glu Lys
225                 230                 235                 240

cag tgg atc cag gag gac cag gcc tcg tac atc tcc ttc aac gcc gct      768
Gln Trp Ile Gln Glu Asp Gln Ala Ser Tyr Ile Ser Phe Asn Ala Ala
                245                 250                 255

tcc aac tcg cgg tcc cag atc aag gcc gct ctg gac aat gcc ggc aag      816
Ser Asn Ser Arg Ser Gln Ile Lys Ala Ala Leu Asp Asn Ala Gly Lys
            260                 265                 270

atc atg gcg ctg acc aaa tcc gcg ccc gac tac ctg gta ggc ccc gct      864
Ile Met Ala Leu Thr Lys Ser Ala Pro Asp Tyr Leu Val Gly Pro Ala
        275                 280                 285

ccg ccc gcg gac att aaa acc aac cgc atc tac cgc atc ctg gag ctg      912
Pro Pro Ala Asp Ile Lys Thr Asn Arg Ile Tyr Arg Ile Leu Glu Leu
    290                 295                 300

aac ggc tac gaa cct gcc tac gcc ggc tcc gtc ttt ctc ggc tgg gcc      960
Asn Gly Tyr Glu Pro Ala Tyr Ala Gly Ser Val Phe Leu Gly Trp Ala
305                 310                 315                 320

cag aaa agg ttc ggg aag cgc aac acc atc tgg ctg ttt ggg ccg gcc     1008
Gln Lys Arg Phe Gly Lys Arg Asn Thr Ile Trp Leu Phe Gly Pro Ala
                325                 330                 335

acc acg ggc aag acc aac atc gcg gaa gcc atc gcc cac gcc gtg ccc     1056
Thr Thr Gly Lys Thr Asn Ile Ala Glu Ala Ile Ala His Ala Val Pro
            340                 345                 350

ttc tac ggc tgc gtc aac tgg acc aat gag aac ttt ccc ttc aat gat     1104
Phe Tyr Gly Cys Val Asn Trp Thr Asn Glu Asn Phe Pro Phe Asn Asp
        355                 360                 365

tgc gtc gac aag atg gtg atc tgg tgg gag gag ggc aag atg acg gcc     1152
Cys Val Asp Lys Met Val Ile Trp Trp Glu Glu Gly Lys Met Thr Ala
    370                 375                 380

aag gtc gtg gag tcc gcc aag gcc att ctc ggc ggc agc aag gtg cgc     1200
Lys Val Val Glu Ser Ala Lys Ala Ile Leu Gly Gly Ser Lys Val Arg
385                 390                 395                 400

gtg gac caa aag tgc aag tcg tcc gcc cag atc gac ccc acc ccc gtg     1248
Val Asp Gln Lys Cys Lys Ser Ser Ala Gln Ile Asp Pro Thr Pro Val
                405                 410                 415

atc gtc acc tcc aac acc aac atg tgc gcc gtg att gac ggg aac agc     1296
Ile Val Thr Ser Asn Thr Asn Met Cys Ala Val Ile Asp Gly Asn Ser

420                 425                 430

acc acc ttc gag cac cag cag ccg ttg cag gac cgg atg ttc aaa ttt        1344
Thr Thr Phe Glu His Gln Gln Pro Leu Gln Asp Arg Met Phe Lys Phe
        435                 440                 445

gaa ctc acc cgc cgt ctg gag cat gac ttt ggc aag gtg aca aag cag        1392
Glu Leu Thr Arg Arg Leu Glu His Asp Phe Gly Lys Val Thr Lys Gln
    450                 455                 460

gaa gtc aaa gag ttc ttc cgc tgg gcg cag gat cac gtg acc gag gtg        1440
Glu Val Lys Glu Phe Phe Arg Trp Ala Gln Asp His Val Thr Glu Val
465                 470                 475                 480

gcg cat gag ttc tac gtc aga aag ggt gga gcc aac aaa aga ccc gcc        1488
Ala His Glu Phe Tyr Val Arg Lys Gly Gly Ala Asn Lys Arg Pro Ala
                485                 490                 495

ccc gat gac gcg gat aaa agc gag ccc aag cgg gcc tgc ccc tca gtc        1536
Pro Asp Asp Ala Asp Lys Ser Glu Pro Lys Arg Ala Cys Pro Ser Val
            500                 505                 510

gcg gat cca tcg acg tca gac gcg gaa gga gct ccg gtg gac ttt gcc        1584
Ala Asp Pro Ser Thr Ser Asp Ala Glu Gly Ala Pro Val Asp Phe Ala
        515                 520                 525

gac agg tat ggc tgc cga tgg tta tct tcc aga ttg gct cga gga caa        1632
Asp Arg Tyr Gly Cys Arg Trp Leu Ser Ser Arg Leu Ala Arg Gly Gln
    530                 535                 540

cct ctc tga                                                            1641
Pro Leu
545


<210>  7
<211>  546
<212>  PRT
<213>  AAV-1

<400>  7
Met Pro Gly Phe Tyr Glu Ile Val Ile Lys Val Pro Ser Asp Leu Asp
1               5                   10                  15

Glu His Leu Pro Gly Ile Ser Asp Ser Phe Val Ser Trp Val Ala Glu
            20                  25                  30

Lys Glu Trp Glu Leu Pro Pro Asp Ser Asp Met Asp Leu Asn Leu Ile
        35                  40                  45

Glu Gln Ala Pro Leu Thr Val Ala Glu Lys Leu Gln Arg Asp Phe Leu
    50                  55                  60

Val Gln Trp Arg Arg Val Ser Lys Ala Pro Glu Ala Leu Phe Phe Val
65                  70                  75                  80

Gln Phe Glu Lys Gly Glu Ser Tyr Phe His Leu His Ile Leu Val Glu
                85                  90                  95

Thr Thr Gly Val Lys Ser Met Val Leu Gly Arg Phe Leu Ser Gln Ile
            100                 105                 110

Arg Asp Lys Leu Val Gln Thr Ile Tyr Arg Gly Ile Glu Pro Thr Leu
        115                 120                 125

Pro Asn Trp Phe Ala Val Thr Lys Thr Arg Asn Gly Ala Gly Gly Gly
    130                 135                 140

Asn Lys Val Val Asp Glu Cys Tyr Ile Pro Asn Tyr Leu Leu Pro Lys
145                 150                 155                 160

Thr Gln Pro Glu Leu Gln Trp Ala Trp Thr Asn Met Glu Glu Tyr Ile
                165                 170                 175

Ser Ala Cys Leu Asn Leu Ala Glu Arg Lys Arg Leu Val Ala Gln His
            180                 185                 190

Leu Thr His Val Ser Gln Thr Gln Glu Gln Asn Lys Glu Asn Leu Asn
        195                 200                 205

Pro Asn Ser Asp Ala Pro Val Ile Arg Ser Lys Thr Ser Ala Arg Tyr
    210                 215                 220

Met Glu Leu Val Gly Trp Leu Val Asp Arg Gly Ile Thr Ser Glu Lys
225                 230                 235                 240

Gln Trp Ile Gln Glu Asp Gln Ala Ser Tyr Ile Ser Phe Asn Ala Ala
                245                 250                 255

Ser Asn Ser Arg Ser Gln Ile Lys Ala Ala Leu Asp Asn Ala Gly Lys
            260                 265                 270

Ile Met Ala Leu Thr Lys Ser Ala Pro Asp Tyr Leu Val Gly Pro Ala
        275                 280                 285

Pro Pro Ala Asp Ile Lys Thr Asn Arg Ile Tyr Arg Ile Leu Glu Leu
    290                 295                 300

Asn Gly Tyr Glu Pro Ala Tyr Ala Gly Ser Val Phe Leu Gly Trp Ala
305                 310                 315                 320

Gln Lys Arg Phe Gly Lys Arg Asn Thr Ile Trp Leu Phe Gly Pro Ala
                325                 330                 335

Thr Thr Gly Lys Thr Asn Ile Ala Glu Ala Ile Ala His Ala Val Pro
            340                 345                 350

Phe Tyr Gly Cys Val Asn Trp Thr Asn Glu Asn Phe Pro Phe Asn Asp
        355                 360                 365

Cys Val Asp Lys Met Val Ile Trp Trp Glu Glu Gly Lys Met Thr Ala
    370                 375                 380

Lys Val Val Glu Ser Ala Lys Ala Ile Leu Gly Gly Ser Lys Val Arg
385                 390                 395                 400

Val Asp Gln Lys Cys Lys Ser Ser Ala Gln Ile Asp Pro Thr Pro Val
                405                 410                 415

Ile Val Thr Ser Asn Thr Asn Met Cys Ala Val Ile Asp Gly Asn Ser
            420                 425                 430

Thr Thr Phe Glu His Gln Gln Pro Leu Gln Asp Arg Met Phe Lys Phe
        435                 440                 445

Glu Leu Thr Arg Arg Leu Glu His Asp Phe Gly Lys Val Thr Lys Gln
    450                 455                 460

Glu Val Lys Glu Phe Phe Arg Trp Ala Gln Asp His Val Thr Glu Val
465                 470                 475                 480

Ala His Glu Phe Tyr Val Arg Lys Gly Gly Ala Asn Lys Arg Pro Ala
                485                 490                 495

Pro Asp Asp Ala Asp Lys Ser Glu Pro Lys Arg Ala Cys Pro Ser Val
            500                 505                 510

Ala Asp Pro Ser Thr Ser Asp Ala Glu Gly Ala Pro Val Asp Phe Ala
        515                 520                 525

Asp Arg Tyr Gly Cys Arg Trp Leu Ser Ser Arg Leu Ala Arg Gly Gln
    530                 535                 540

Pro Leu
545


<210>  8
<211>  1200
<212>  DNA
<213>  AAV-1

<220>
<221>  CDS
<222>  (1)..(1197)
<223>

<400>  8
atg gag ctg gtc ggg tgg ctg gtg gac cgg ggc atc acc tcc gag aag      48
Met Glu Leu Val Gly Trp Leu Val Asp Arg Gly Ile Thr Ser Glu Lys
1               5                   10                  15

cag tgg atc cag gag gac cag gcc tcg tac atc tcc ttc aac gcc gct      96
Gln Trp Ile Gln Glu Asp Gln Ala Ser Tyr Ile Ser Phe Asn Ala Ala
            20                  25                  30

tcc aac tcg cgg tcc cag atc aag gcc gct ctg gac aat gcc ggc aag      144
Ser Asn Ser Arg Ser Gln Ile Lys Ala Ala Leu Asp Asn Ala Gly Lys
        35                  40                  45

atc atg gcg ctg acc aaa tcc gcg ccc gac tac ctg gta ggc ccc gct      192
Ile Met Ala Leu Thr Lys Ser Ala Pro Asp Tyr Leu Val Gly Pro Ala
    50                  55                  60

ccg ccc gcg gac att aaa acc aac cgc atc tac cgc atc ctg gag ctg      240
Pro Pro Ala Asp Ile Lys Thr Asn Arg Ile Tyr Arg Ile Leu Glu Leu
65                  70                  75                  80

aac ggc tac gaa cct gcc tac gcc ggc tcc gtc ttt ctc ggc tgg gcc      288
Asn Gly Tyr Glu Pro Ala Tyr Ala Gly Ser Val Phe Leu Gly Trp Ala
                85                  90                  95

cag aaa agg ttc ggg aag cgc aac acc atc tgg ctg ttt ggg ccg gcc      336
Gln Lys Arg Phe Gly Lys Arg Asn Thr Ile Trp Leu Phe Gly Pro Ala
            100                 105                 110

acc acg ggc aag acc aac atc gcg gaa gcc atc gcc cac gcc gtg ccc      384
Thr Thr Gly Lys Thr Asn Ile Ala Glu Ala Ile Ala His Ala Val Pro
        115                 120                 125

ttc tac ggc tgc gtc aac tgg acc aat gag aac ttt ccc ttc aat gat      432
Phe Tyr Gly Cys Val Asn Trp Thr Asn Glu Asn Phe Pro Phe Asn Asp
    130                 135                 140

tgc gtc gac aag atg gtg atc tgg tgg gag gag ggc aag atg acg gcc      480
Cys Val Asp Lys Met Val Ile Trp Trp Glu Glu Gly Lys Met Thr Ala
145                 150                 155                 160

aag gtc gtg gag tcc gcc aag gcc att ctc ggc ggc agc aag gtg cgc      528
Lys Val Val Glu Ser Ala Lys Ala Ile Leu Gly Gly Ser Lys Val Arg
                165                 170                 175

gtg gac caa aag tgc aag tcg tcc gcc cag atc gac ccc acc ccc gtg      576
Val Asp Gln Lys Cys Lys Ser Ser Ala Gln Ile Asp Pro Thr Pro Val

180                 185                 190

atc gtc acc tcc aac acc aac atg tgc gcc gtg att gac ggg aac agc       624
Ile Val Thr Ser Asn Thr Asn Met Cys Ala Val Ile Asp Gly Asn Ser
        195                 200                 205

acc acc ttc gag cac cag cag ccg ttg cag gac cgg atg ttc aaa ttt       672
Thr Thr Phe Glu His Gln Gln Pro Leu Gln Asp Arg Met Phe Lys Phe
    210                 215                 220

gaa ctc acc cgc cgt ctg gag cat gac ttt ggc aag gtg aca aag cag       720
Glu Leu Thr Arg Arg Leu Glu His Asp Phe Gly Lys Val Thr Lys Gln
225                 230                 235                 240

gaa gtc aaa gag ttc ttc cgc tgg gcg cag gat cac gtg acc gag gtg       768
Glu Val Lys Glu Phe Phe Arg Trp Ala Gln Asp His Val Thr Glu Val
                245                 250                 255

gcg cat gag ttc tac gtc aga aag ggt gga gcc aac aaa aga ccc gcc       816
Ala His Glu Phe Tyr Val Arg Lys Gly Gly Ala Asn Lys Arg Pro Ala
            260                 265                 270

ccc gat gac gcg gat aaa agc gag ccc aag cgg gcc tgc ccc tca gtc       864
Pro Asp Asp Ala Asp Lys Ser Glu Pro Lys Arg Ala Cys Pro Ser Val
        275                 280                 285

gcg gat cca tcg acg tca gac gcg gaa gga gct ccg gtg gac ttt gcc       912
Ala Asp Pro Ser Thr Ser Asp Ala Glu Gly Ala Pro Val Asp Phe Ala
    290                 295                 300

gac agg tac caa aac aaa tgt tct cgt cac gcg ggc atg ctt cag atg       960
Asp Arg Tyr Gln Asn Lys Cys Ser Arg His Ala Gly Met Leu Gln Met
305                 310                 315                 320

ctg ttt ccc tgc aag aca tgc gag aga atg aat cag aat ttc aac att      1008
Leu Phe Pro Cys Lys Thr Cys Glu Arg Met Asn Gln Asn Phe Asn Ile
                325                 330                 335

tgc ttc acg cac ggg acg aga gac tgt tca gag tgc ttc ccc ggc gtg      1056
Cys Phe Thr His Gly Thr Arg Asp Cys Ser Glu Cys Phe Pro Gly Val
            340                 345                 350

tca gaa tct caa ccg gtc gtc aga aag agg acg tat cgg aaa ctc tgt      1104
Ser Glu Ser Gln Pro Val Val Arg Lys Arg Thr Tyr Arg Lys Leu Cys
        355                 360                 365

gcc att cat cat ctg ctg ggg cgg gct ccc gag att gct tgc tcg gcc      1152
Ala Ile His His Leu Leu Gly Arg Ala Pro Glu Ile Ala Cys Ser Ala
    370                 375                 380

tgc gat ctg gtc aac gtg gac ctg gat gac tgt gtt tct gag caa taa      1200
Cys Asp Leu Val Asn Val Asp Leu Asp Asp Cys Val Ser Glu Gln
385                 390                 395

<210>  9
<211>  399
<212>  PRT
<213>  AAV-1

<400>  9
Met Glu Leu Val Gly Trp Leu Val Asp Arg Gly Ile Thr Ser Glu Lys
1               5                   10                  15

Gln Trp Ile Gln Glu Asp Gln Ala Ser Tyr Ile Ser Phe Asn Ala Ala
            20                  25                  30

Ser Asn Ser Arg Ser Gln Ile Lys Ala Ala Leu Asp Asn Ala Gly Lys

35                  40                  45

Ile Met Ala Leu Thr Lys Ser Ala Pro Asp Tyr Leu Val Gly Pro Ala
    50                  55                  60

Pro Pro Ala Asp Ile Lys Thr Asn Arg Ile Tyr Arg Ile Leu Glu Leu
65                  70                  75                  80

Asn Gly Tyr Glu Pro Ala Tyr Ala Gly Ser Val Phe Leu Gly Trp Ala
                85                  90                  95

Gln Lys Arg Phe Gly Lys Arg Asn Thr Ile Trp Leu Phe Gly Pro Ala
            100                 105                 110

Thr Thr Gly Lys Thr Asn Ile Ala Glu Ala Ile Ala His Ala Val Pro
        115                 120                 125

Phe Tyr Gly Cys Val Asn Trp Thr Asn Glu Asn Phe Pro Phe Asn Asp
    130                 135                 140

Cys Val Asp Lys Met Val Ile Trp Trp Glu Glu Gly Lys Met Thr Ala
145                 150                 155                 160

Lys Val Val Glu Ser Ala Lys Ala Ile Leu Gly Gly Ser Lys Val Arg
                165                 170                 175

Val Asp Gln Lys Cys Lys Ser Ser Ala Gln Ile Asp Pro Thr Pro Val
            180                 185                 190

Ile Val Thr Ser Asn Thr Asn Met Cys Ala Val Ile Asp Gly Asn Ser
        195                 200                 205

Thr Thr Phe Glu His Gln Gln Pro Leu Gln Asp Arg Met Phe Lys Phe
    210                 215                 220

Glu Leu Thr Arg Arg Leu Glu His Asp Phe Gly Lys Val Thr Lys Gln
225                 230                 235                 240

Glu Val Lys Glu Phe Phe Arg Trp Ala Gln Asp His Val Thr Glu Val
                245                 250                 255

Ala His Glu Phe Tyr Val Arg Lys Gly Gly Ala Asn Lys Arg Pro Ala
            260                 265                 270

Pro Asp Asp Ala Asp Lys Ser Glu Pro Lys Arg Ala Cys Pro Ser Val
        275                 280                 285

Ala Asp Pro Ser Thr Ser Asp Ala Glu Gly Ala Pro Val Asp Phe Ala
    290                 295                 300

Asp Arg Tyr Gln Asn Lys Cys Ser Arg His Ala Gly Met Leu Gln Met
305                 310                 315                 320

Leu Phe Pro Cys Lys Thr Cys Glu Arg Met Asn Gln Asn Phe Asn Ile
                325                 330                 335

Cys Phe Thr His Gly Thr Arg Asp Cys Ser Glu Cys Phe Pro Gly Val
            340                 345                 350

Ser Glu Ser Gln Pro Val Val Arg Lys Arg Thr Tyr Arg Lys Leu Cys
        355                 360                 365

Ala Ile His His Leu Leu Gly Arg Ala Pro Glu Ile Ala Cys Ser Ala
    370                 375                 380

Cys Asp Leu Val Asn Val Asp Leu Asp Asp Cys Val Ser Glu Gln
385                 390                 395

<210>  10
<211>  969
<212>  DNA
<213>  AAV-1

<220>
<221>  CDS
<222>  (1)..(966)
<223>

<220>
<221>  misc_feature
<222>  (943)..(944)
<223>  minor splice site

<400>  10
atg gag ctg gtc ggg tgg ctg gtg gac cgg ggc atc acc tcc gag aag      48
Met Glu Leu Val Gly Trp Leu Val Asp Arg Gly Ile Thr Ser Glu Lys
1               5                   10                  15

cag tgg atc cag gag gac cag gcc tcg tac atc tcc ttc aac gcc gct      96
Gln Trp Ile Gln Glu Asp Gln Ala Ser Tyr Ile Ser Phe Asn Ala Ala
            20                  25                  30

tcc aac tcg cgg tcc cag atc aag gcc gct ctg gac aat gcc ggc aag      144
Ser Asn Ser Arg Ser Gln Ile Lys Ala Ala Leu Asp Asn Ala Gly Lys
        35                  40                  45

atc atg gcg ctg acc aaa tcc gcg ccc gac tac ctg gta ggc ccc gct      192
Ile Met Ala Leu Thr Lys Ser Ala Pro Asp Tyr Leu Val Gly Pro Ala
    50                  55                  60

ccg ccc gcg gac att aaa acc aac cgc atc tac cgc atc ctg gag ctg      240
Pro Pro Ala Asp Ile Lys Thr Asn Arg Ile Tyr Arg Ile Leu Glu Leu
65                  70                  75                  80

aac ggc tac gaa cct gcc tac gcc ggc tcc gtc ttt ctc ggc tgg gcc      288
Asn Gly Tyr Glu Pro Ala Tyr Ala Gly Ser Val Phe Leu Gly Trp Ala
                85                  90                  95

cag aaa agg ttc ggg aag cgc aac acc atc tgg ctg ttt ggg ccg gcc      336
Gln Lys Arg Phe Gly Lys Arg Asn Thr Ile Trp Leu Phe Gly Pro Ala
            100                 105                 110

acc acg ggc aag acc aac atc gcg gaa gcc atc gcc cac gcc gtg ccc      384
Thr Thr Gly Lys Thr Asn Ile Ala Glu Ala Ile Ala His Ala Val Pro
        115                 120                 125

ttc tac ggc tgc gtc aac tgg acc aat gag aac ttt ccc ttc aat gat      432
Phe Tyr Gly Cys Val Asn Trp Thr Asn Glu Asn Phe Pro Phe Asn Asp
    130                 135                 140

tgc gtc gac aag atg gtg atc tgg tgg gag gag ggc aag atg acg gcc      480
Cys Val Asp Lys Met Val Ile Trp Trp Glu Glu Gly Lys Met Thr Ala
145                 150                 155                 160

aag gtc gtg gag tcc gcc aag gcc att ctc ggc ggc agc aag gtg cgc      528
Lys Val Val Glu Ser Ala Lys Ala Ile Leu Gly Gly Ser Lys Val Arg
                165                 170                 175

gtg gac caa aag tgc aag tcg tcc gcc cag atc gac ccc acc ccc gtg      576
Val Asp Gln Lys Cys Lys Ser Ser Ala Gln Ile Asp Pro Thr Pro Val
            180                 185                 190

atc gtc acc tcc aac acc aac atg tgc gcc gtg att gac ggg aac agc      624
Ile Val Thr Ser Asn Thr Asn Met Cys Ala Val Ile Asp Gly Asn Ser
        195                 200                 205

acc acc ttc gag cac cag cag ccg ttg cag gac cgg atg ttc aaa ttt          672
Thr Thr Phe Glu His Gln Gln Pro Leu Gln Asp Arg Met Phe Lys Phe
    210                 215                 220

gaa ctc acc cgc cgt ctg gag cat gac ttt ggc aag gtg aca aag cag          720
Glu Leu Thr Arg Arg Leu Glu His Asp Phe Gly Lys Val Thr Lys Gln
225                 230                 235                 240

gaa gtc aaa gag ttc ttc cgc tgg gcg cag gat cac gtg acc gag gtg          768
Glu Val Lys Glu Phe Phe Arg Trp Ala Gln Asp His Val Thr Glu Val
                245                 250                 255

gcg cat gag ttc tac gtc aga aag ggt gga gcc aac aaa aga ccc gcc          816
Ala His Glu Phe Tyr Val Arg Lys Gly Gly Ala Asn Lys Arg Pro Ala
            260                 265                 270

ccc gat gac gcg gat aaa agc gag ccc aag cgg gcc tgc ccc tca gtc          864
Pro Asp Asp Ala Asp Lys Ser Glu Pro Lys Arg Ala Cys Pro Ser Val
        275                 280                 285

gcg gat cca tcg acg tca gac gcg gaa gga gct ccg gtg gac ttt gcc          912
Ala Asp Pro Ser Thr Ser Asp Ala Glu Gly Ala Pro Val Asp Phe Ala
    290                 295                 300

gac agg tat ggc tgc cga tgg tta tct tcc aga ttg gct cga gga caa          960
Asp Arg Tyr Gly Cys Arg Trp Leu Ser Ser Arg Leu Ala Arg Gly Gln
305                 310                 315                 320

cct ctc tga                                                              969
Pro Leu


<210>  11
<211>  322
<212>  PRT
<213>  AAV-1

<400>  11
Met Glu Leu Val Gly Trp Leu Val Asp Arg Gly Ile Thr Ser Glu Lys
1               5                   10                  15

Gln Trp Ile Gln Glu Asp Gln Ala Ser Tyr Ile Ser Phe Asn Ala Ala
            20                  25                  30

Ser Asn Ser Arg Ser Gln Ile Lys Ala Ala Leu Asp Asn Ala Gly Lys
        35                  40                  45

Ile Met Ala Leu Thr Lys Ser Ala Pro Asp Tyr Leu Val Gly Pro Ala
    50                  55                  60

Pro Pro Ala Asp Ile Lys Thr Asn Arg Ile Tyr Arg Ile Leu Glu Leu
65                  70                  75                  80

Asn Gly Tyr Glu Pro Ala Tyr Ala Gly Ser Val Phe Leu Gly Trp Ala
                85                  90                  95

Gln Lys Arg Phe Gly Lys Arg Asn Thr Ile Trp Leu Phe Gly Pro Ala
            100                 105                 110

Thr Thr Gly Lys Thr Asn Ile Ala Glu Ala Ile Ala His Ala Val Pro
        115                 120                 125

Phe Tyr Gly Cys Val Asn Trp Thr Asn Glu Asn Phe Pro Phe Asn Asp
    130                 135                 140

Cys Val Asp Lys Met Val Ile Trp Trp Glu Glu Gly Lys Met Thr Ala

145                 150                 155                 160

Lys Val Val Glu Ser Ala Lys Ala Ile Leu Gly Gly Ser Lys Val Arg
                165                 170                 175

Val Asp Gln Lys Cys Lys Ser Ser Ala Gln Ile Asp Pro Thr Pro Val
            180                 185                 190

Ile Val Thr Ser Asn Thr Asn Met Cys Ala Val Ile Asp Gly Asn Ser
        195                 200                 205

Thr Thr Phe Glu His Gln Gln Pro Leu Gln Asp Arg Met Phe Lys Phe
    210                 215                 220

Glu Leu Thr Arg Arg Leu Glu His Asp Phe Gly Lys Val Thr Lys Gln
225                 230                 235                 240

Glu Val Lys Glu Phe Phe Arg Trp Ala Gln Asp His Val Thr Glu Val
                245                 250                 255

Ala His Glu Phe Tyr Val Arg Lys Gly Gly Ala Asn Lys Arg Pro Ala
            260                 265                 270

Pro Asp Asp Ala Asp Lys Ser Glu Pro Lys Arg Ala Cys Pro Ser Val
        275                 280                 285

Ala Asp Pro Ser Thr Ser Asp Ala Glu Gly Ala Pro Val Asp Phe Ala
    290                 295                 300

Asp Arg Tyr Gly Cys Arg Trp Leu Ser Ser Arg Leu Ala Arg Gly Gln
305                 310                 315                 320

Pro Leu


<210>  12
<211>  2211
<212>  DNA
<213>  AAV-1

<220>
<221>  CDS
<222>  (1)..(2208)
<223>

<400>  12
atg gct gcc gat ggt tat ctt cca gat tgg ctc gag gac aac ctc tct      48
Met Ala Ala Asp Gly Tyr Leu Pro Asp Trp Leu Glu Asp Asn Leu Ser
1               5                   10                  15

gag ggc att cgc gag tgg tgg gac ttg aaa cct gga gcc ccg aag ccc      96
Glu Gly Ile Arg Glu Trp Trp Asp Leu Lys Pro Gly Ala Pro Lys Pro
            20                  25                  30

aaa gcc aac cag caa aag cag gac gac ggc cgg ggt ctg gtg ctt cct     144
Lys Ala Asn Gln Gln Lys Gln Asp Asp Gly Arg Gly Leu Val Leu Pro
        35                  40                  45

ggc tac aag tac ctc gga ccc ttc aac gga ctc gac aag ggg gag ccc     192
Gly Tyr Lys Tyr Leu Gly Pro Phe Asn Gly Leu Asp Lys Gly Glu Pro
    50                  55                  60

gtc aac gcg gcg gac gca gcg gcc ctc gag cac gac aag gcc tac gac     240
Val Asn Ala Ala Asp Ala Ala Ala Leu Glu His Asp Lys Ala Tyr Asp
65                  70                  75                  80

cag cag ctc aaa gcg ggt gac aat ccg tac ctg cgg tat aac cac gcc     288

Gln Gln Leu Lys Ala Gly Asp Asn Pro Tyr Leu Arg Tyr Asn His Ala
                85                  90                  95

gac gcc gag ttt cag gag cgt ctg caa gaa gat acg tct ttt ggg ggc 336
Asp Ala Glu Phe Gln Glu Arg Leu Gln Glu Asp Thr Ser Phe Gly Gly
            100                 105                 110

aac ctc ggg cga gca gtc ttc cag gcc aag aag cgg gtt ctc gaa cct 384
Asn Leu Gly Arg Ala Val Phe Gln Ala Lys Lys Arg Val Leu Glu Pro
        115                 120                 125

ctc ggt ctg gtt gag gaa ggc gct aag acg gct cct gga aag aaa cgt 432
Leu Gly Leu Val Glu Glu Gly Ala Lys Thr Ala Pro Gly Lys Lys Arg
    130                 135                 140

ccg gta gag cag tcg cca caa gag cca gac tcc tcc tcg ggc atc ggc 480
Pro Val Glu Gln Ser Pro Gln Glu Pro Asp Ser Ser Ser Gly Ile Gly
145                 150                 155                 160

aag aca ggc cag cag ccc gct aaa aag aga ctc aat ttt ggt cag act 528
Lys Thr Gly Gln Gln Pro Ala Lys Lys Arg Leu Asn Phe Gly Gln Thr
                165                 170                 175

ggc gac tca gag tca gtc ccc gat cca caa cct ctc gga gaa cct cca 576
Gly Asp Ser Glu Ser Val Pro Asp Pro Gln Pro Leu Gly Glu Pro Pro
            180                 185                 190

gca acc ccc gct gct gtg gga cct act aca atg gct tca ggc ggt ggc 624
Ala Thr Pro Ala Ala Val Gly Pro Thr Thr Met Ala Ser Gly Gly Gly
        195                 200                 205

gca cca atg gca gac aat aac gaa ggc gcc gac gga gtg ggt aat gcc 672
Ala Pro Met Ala Asp Asn Asn Glu Gly Ala Asp Gly Val Gly Asn Ala
    210                 215                 220

tca gga aat tgg cat tgc gat tcc aca tgg ctg ggc gac aga gtc atc 720
Ser Gly Asn Trp His Cys Asp Ser Thr Trp Leu Gly Asp Arg Val Ile
225                 230                 235                 240

acc acc agc acc cgc acc tgg gcc ttg ccc acc tac aat aac cac ctc 768
Thr Thr Ser Thr Arg Thr Trp Ala Leu Pro Thr Tyr Asn Asn His Leu
                245                 250                 255

tac aag caa atc tcc agt gct tca acg ggg gcc agc aac gac aac cac 816
Tyr Lys Gln Ile Ser Ser Ala Ser Thr Gly Ala Ser Asn Asp Asn His
            260                 265                 270

tac ttc ggc tac agc acc ccc tgg ggg tat ttt gat ttc aac aga ttc 864
Tyr Phe Gly Tyr Ser Thr Pro Trp Gly Tyr Phe Asp Phe Asn Arg Phe
        275                 280                 285

cac tgc cac ttt tca cca cgt gac tgg cag cga ctc atc aac aac aat 912
His Cys His Phe Ser Pro Arg Asp Trp Gln Arg Leu Ile Asn Asn Asn
    290                 295                 300

tgg gga ttc cgg ccc aag aga ctc aac ttc aaa ctc ttc aac atc caa 960
Trp Gly Phe Arg Pro Lys Arg Leu Asn Phe Lys Leu Phe Asn Ile Gln
305                 310                 315                 320

gtc aag gag gtc acg acg aat gat ggc gtc aca acc atc gct aat aac 1008
Val Lys Glu Val Thr Thr Asn Asp Gly Val Thr Thr Ile Ala Asn Asn
                325                 330                 335

ctt acc agc acg gtt caa gtc ttc tcg gac tcg gag tac cag ctt ccg 1056
Leu Thr Ser Thr Val Gln Val Phe Ser Asp Ser Glu Tyr Gln Leu Pro
            340                 345                 350

tac gtc ctc ggc tct gcg cac cag ggc tgc ctc cct ccg ttc ccg gcg 1104

Tyr Val Leu Gly Ser Ala His Gln Gly Cys Leu Pro Pro Phe Pro Ala
        355                 360                 365

gac gtg ttc atg att ccg caa tac ggc tac ctg acg ctc aac aat ggc      1152
Asp Val Phe Met Ile Pro Gln Tyr Gly Tyr Leu Thr Leu Asn Asn Gly
    370                 375                 380

agc caa gcc gtg gga cgt tca tcc ttt tac tgc ctg gaa tat ttc cct      1200
Ser Gln Ala Val Gly Arg Ser Ser Phe Tyr Cys Leu Glu Tyr Phe Pro
385                 390                 395                 400

tct cag atg ctg aga acg ggc aac aac ttt acc ttc agc tac acc ttt      1248
Ser Gln Met Leu Arg Thr Gly Asn Asn Phe Thr Phe Ser Tyr Thr Phe
                405                 410                 415

gag gaa gtg cct ttc cac agc agc tac gcg cac agc cag agc ctg gac      1296
Glu Glu Val Pro Phe His Ser Ser Tyr Ala His Ser Gln Ser Leu Asp
            420                 425                 430

cgg ctg atg aat cct ctc atc gac caa tac ctg tat tac ctg aac aga      1344
Arg Leu Met Asn Pro Leu Ile Asp Gln Tyr Leu Tyr Tyr Leu Asn Arg
        435                 440                 445

act caa aat cag tcc gga agt gcc caa aac aag gac ttg ctg ttt agc      1392
Thr Gln Asn Gln Ser Gly Ser Ala Gln Asn Lys Asp Leu Leu Phe Ser
    450                 455                 460

cgt ggg tct cca gct ggc atg tct gtt cag ccc aaa aac tgg cta cct      1440
Arg Gly Ser Pro Ala Gly Met Ser Val Gln Pro Lys Asn Trp Leu Pro
465                 470                 475                 480

gga ccc tgt tat cgg cag cag cgc gtt tct aaa aca aaa aca gac aac      1488
Gly Pro Cys Tyr Arg Gln Gln Arg Val Ser Lys Thr Lys Thr Asp Asn
                485                 490                 495

aac aac agc aat ttt acc tgg act ggt gct tca aaa tat aac ctc aat      1536
Asn Asn Ser Asn Phe Thr Trp Thr Gly Ala Ser Lys Tyr Asn Leu Asn
            500                 505                 510

ggg cgt gaa tcc atc atc aac cct ggc act gct atg gcc tca cac aaa      1584
Gly Arg Glu Ser Ile Ile Asn Pro Gly Thr Ala Met Ala Ser His Lys
        515                 520                 525

gac gac gaa gac aag ttc ttt ccc atg agc ggt gtc atg att ttt gga      1632
Asp Asp Glu Asp Lys Phe Phe Pro Met Ser Gly Val Met Ile Phe Gly
    530                 535                 540

aaa gag agc gcc gga gct tca aac act gca ttg gac aat gtc atg att      1680
Lys Glu Ser Ala Gly Ala Ser Asn Thr Ala Leu Asp Asn Val Met Ile
545                 550                 555                 560

aca gac gaa gag gaa att aaa gcc act aac cct gtg gcc acc gaa aga      1728
Thr Asp Glu Glu Glu Ile Lys Ala Thr Asn Pro Val Ala Thr Glu Arg
                565                 570                 575

ttt ggg acc gtg gca gtc aat ttc cag agc agc agc aca gac cct gcg      1776
Phe Gly Thr Val Ala Val Asn Phe Gln Ser Ser Ser Thr Asp Pro Ala
            580                 585                 590

acc gga gat gtg cat gct atg gga gca tta cct ggc atg gtg tgg caa      1824
Thr Gly Asp Val His Ala Met Gly Ala Leu Pro Gly Met Val Trp Gln
        595                 600                 605

gat aga gac gtg tac ctg cag ggt ccc att tgg gcc aaa att cct cac      1872
Asp Arg Asp Val Tyr Leu Gln Gly Pro Ile Trp Ala Lys Ile Pro His
    610                 615                 620

aca gat gga cac ttt cac ccg tct cct ctt atg ggc ggc ttt gga ctc      1920

Thr Asp Gly His Phe His Pro Ser Pro Leu Met Gly Gly Phe Gly Leu
625                 630                 635                 640

aag aac ccg cct cct cag atc ctc atc aaa aac acg cct gtt cct gcg      1968
Lys Asn Pro Pro Pro Gln Ile Leu Ile Lys Asn Thr Pro Val Pro Ala
                645                 650                 655

aat cct ccg gcg gag ttt tca gct aca aag ttt gct tca ttc atc acc      2016
Asn Pro Pro Ala Glu Phe Ser Ala Thr Lys Phe Ala Ser Phe Ile Thr
            660                 665                 670

caa tac tcc aca gga caa gtg agt gtg gaa att gaa tgg gag ctg cag      2064
Gln Tyr Ser Thr Gly Gln Val Ser Val Glu Ile Glu Trp Glu Leu Gln
        675                 680                 685

aaa gaa aac agc aag cgc tgg aat ccc gaa gtg cag tac aca tcc aat      2112
Lys Glu Asn Ser Lys Arg Trp Asn Pro Glu Val Gln Tyr Thr Ser Asn
    690                 695                 700

tat gca aaa tct gcc aac gtt gat ttt act gtg gac aac aat gga ctt      2160
Tyr Ala Lys Ser Ala Asn Val Asp Phe Thr Val Asp Asn Asn Gly Leu
705                 710                 715                 720

tat act gag cct cgc ccc att ggc acc cgt tac ctt acc cgt ccc ctg      2208
Tyr Thr Glu Pro Arg Pro Ile Gly Thr Arg Tyr Leu Thr Arg Pro Leu
                725                 730                 735

taa                                                                  2211


<210>  13
<211>  736
<212>  PRT
<213>  AAV-1

<400>  13
Met Ala Ala Asp Gly Tyr Leu Pro Asp Trp Leu Glu Asp Asn Leu Ser
1               5                   10                  15

Glu Gly Ile Arg Glu Trp Trp Asp Leu Lys Pro Gly Ala Pro Lys Pro
            20                  25                  30

Lys Ala Asn Gln Gln Lys Gln Asp Asp Gly Arg Gly Leu Val Leu Pro
        35                  40                  45

Gly Tyr Lys Tyr Leu Gly Pro Phe Asn Gly Leu Asp Lys Gly Glu Pro
    50                  55                  60

Val Asn Ala Ala Asp Ala Ala Ala Leu Glu His Asp Lys Ala Tyr Asp
65                  70                  75                  80

Gln Gln Leu Lys Ala Gly Asp Asn Pro Tyr Leu Arg Tyr Asn His Ala
                85                  90                  95

Asp Ala Glu Phe Gln Glu Arg Leu Gln Glu Asp Thr Ser Phe Gly Gly
            100                 105                 110

Asn Leu Gly Arg Ala Val Phe Gln Ala Lys Lys Arg Val Leu Glu Pro
        115                 120                 125

Leu Gly Leu Val Glu Glu Gly Ala Lys Thr Ala Pro Gly Lys Lys Arg
    130                 135                 140

Pro Val Glu Gln Ser Pro Gln Glu Pro Asp Ser Ser Ser Gly Ile Gly
145                 150                 155                 160

Lys Thr Gly Gln Gln Pro Ala Lys Lys Arg Leu Asn Phe Gly Gln Thr
                165                 170                 175

Gly Asp Ser Glu Ser Val Pro Asp Pro Gln Pro Leu Gly Glu Pro Pro
            180                 185                 190

Ala Thr Pro Ala Ala Val Gly Pro Thr Thr Met Ala Ser Gly Gly Gly
        195                 200                 205

Ala Pro Met Ala Asp Asn Asn Glu Gly Ala Asp Gly Val Gly Asn Ala
    210                 215                 220

Ser Gly Asn Trp His Cys Asp Ser Thr Trp Leu Gly Asp Arg Val Ile
225                 230                 235                 240

Thr Thr Ser Thr Arg Thr Trp Ala Leu Pro Thr Tyr Asn Asn His Leu
                245                 250                 255

Tyr Lys Gln Ile Ser Ser Ala Ser Thr Gly Ala Ser Asn Asp Asn His
            260                 265                 270

Tyr Phe Gly Tyr Ser Thr Pro Trp Gly Tyr Phe Asp Phe Asn Arg Phe
        275                 280                 285

His Cys His Phe Ser Pro Arg Asp Trp Gln Arg Leu Ile Asn Asn Asn
    290                 295                 300

Trp Gly Phe Arg Pro Lys Arg Leu Asn Phe Lys Leu Phe Asn Ile Gln
305                 310                 315                 320

Val Lys Glu Val Thr Thr Asn Asp Gly Val Thr Thr Ile Ala Asn Asn
                325                 330                 335

Leu Thr Ser Thr Val Gln Val Phe Ser Asp Ser Glu Tyr Gln Leu Pro
            340                 345                 350

Tyr Val Leu Gly Ser Ala His Gln Gly Cys Leu Pro Pro Phe Pro Ala
        355                 360                 365

Asp Val Phe Met Ile Pro Gln Tyr Gly Tyr Leu Thr Leu Asn Asn Gly
    370                 375                 380

Ser Gln Ala Val Gly Arg Ser Ser Phe Tyr Cys Leu Glu Tyr Phe Pro
385                 390                 395                 400

Ser Gln Met Leu Arg Thr Gly Asn Asn Phe Thr Phe Ser Tyr Thr Phe
                405                 410                 415

Glu Glu Val Pro Phe His Ser Ser Tyr Ala His Ser Gln Ser Leu Asp
            420                 425                 430

Arg Leu Met Asn Pro Leu Ile Asp Gln Tyr Leu Tyr Tyr Leu Asn Arg
        435                 440                 445

Thr Gln Asn Gln Ser Gly Ser Ala Gln Asn Lys Asp Leu Leu Phe Ser
    450                 455                 460

Arg Gly Ser Pro Ala Gly Met Ser Val Gln Pro Lys Asn Trp Leu Pro
465                 470                 475                 480

Gly Pro Cys Tyr Arg Gln Gln Arg Val Ser Lys Thr Lys Thr Asp Asn
                485                 490                 495

Asn Asn Ser Asn Phe Thr Trp Thr Gly Ala Ser Lys Tyr Asn Leu Asn
            500                 505                 510

Gly Arg Glu Ser Ile Ile Asn Pro Gly Thr Ala Met Ala Ser His Lys
        515                 520                 525

Asp Asp Glu Asp Lys Phe Phe Pro Met Ser Gly Val Met Ile Phe Gly

530                 535                 540

Lys Glu Ser Ala Gly Ala Ser Asn Thr Ala Leu Asp Asn Val Met Ile
545                 550                 555                 560

Thr Asp Glu Glu Glu Ile Lys Ala Thr Asn Pro Val Ala Thr Glu Arg
                565                 570                 575

Phe Gly Thr Val Ala Val Asn Phe Gln Ser Ser Ser Thr Asp Pro Ala
            580                 585                 590

Thr Gly Asp Val His Ala Met Gly Ala Leu Pro Gly Met Val Trp Gln
        595                 600                 605

Asp Arg Asp Val Tyr Leu Gln Gly Pro Ile Trp Ala Lys Ile Pro His
    610                 615                 620

Thr Asp Gly His Phe His Pro Ser Pro Leu Met Gly Gly Phe Gly Leu
625                 630                 635                 640

Lys Asn Pro Pro Pro Gln Ile Leu Ile Lys Asn Thr Pro Val Pro Ala
                645                 650                 655

Asn Pro Pro Ala Glu Phe Ser Ala Thr Lys Phe Ala Ser Phe Ile Thr
            660                 665                 670

Gln Tyr Ser Thr Gly Gln Val Ser Val Glu Ile Glu Trp Glu Leu Gln
        675                 680                 685

Lys Glu Asn Ser Lys Arg Trp Asn Pro Glu Val Gln Tyr Thr Ser Asn
    690                 695                 700

Tyr Ala Lys Ser Ala Asn Val Asp Phe Thr Val Asp Asn Asn Gly Leu
705                 710                 715                 720

Tyr Thr Glu Pro Arg Pro Ile Gly Thr Arg Tyr Leu Thr Arg Pro Leu
                725                 730                 735


<210>  14
<211>  1800
<212>  DNA
<213>  AAV-1

<220>
<221>  CDS
<222>  (1)..(1797)
<223>

<400>  14
acg gct cct gga aag aaa cgt ccg gta gag cag tcg cca caa gag cca      48
Thr Ala Pro Gly Lys Lys Arg Pro Val Glu Gln Ser Pro Gln Glu Pro
1               5                   10                  15

gac tcc tcc tcg ggc atc ggc aag aca ggc cag cag ccc gct aaa aag      96
Asp Ser Ser Ser Gly Ile Gly Lys Thr Gly Gln Gln Pro Ala Lys Lys
            20                  25                  30

aga ctc aat ttt ggt cag act ggc gac tca gag tca gtc ccc gat cca      144
Arg Leu Asn Phe Gly Gln Thr Gly Asp Ser Glu Ser Val Pro Asp Pro
        35                  40                  45

caa cct ctc gga gaa cct cca gca acc ccc gct gct gtg gga cct act      192
Gln Pro Leu Gly Glu Pro Pro Ala Thr Pro Ala Ala Val Gly Pro Thr
    50                  55                  60

aca atg gct tca ggc ggt ggc gca cca atg gca gac aat aac gaa ggc      240
Thr Met Ala Ser Gly Gly Gly Ala Pro Met Ala Asp Asn Asn Glu Gly

65                  70                  75                  80

gcc gac gga gtg ggt aat gcc tca gga aat tgg cat tgc gat tcc aca      288
Ala Asp Gly Val Gly Asn Ala Ser Gly Asn Trp His Cys Asp Ser Thr
                85                  90                  95

tgg ctg ggc gac aga gtc atc acc acc agc acc cgc acc tgg gcc ttg      336
Trp Leu Gly Asp Arg Val Ile Thr Thr Ser Thr Arg Thr Trp Ala Leu
            100                 105                 110

ccc acc tac aat aac cac ctc tac aag caa atc tcc agt gct tca acg      384
Pro Thr Tyr Asn Asn His Leu Tyr Lys Gln Ile Ser Ser Ala Ser Thr
        115                 120                 125

ggg gcc agc aac gac aac cac tac ttc ggc tac agc acc ccc tgg ggg      432
Gly Ala Ser Asn Asp Asn His Tyr Phe Gly Tyr Ser Thr Pro Trp Gly
    130                 135                 140

tat ttt gat ttc aac aga ttc cac tgc cac ttt tca cca cgt gac tgg      480
Tyr Phe Asp Phe Asn Arg Phe His Cys His Phe Ser Pro Arg Asp Trp
145                 150                 155                 160

cag cga ctc atc aac aac aat tgg gga ttc cgg ccc aag aga ctc aac      528
Gln Arg Leu Ile Asn Asn Asn Trp Gly Phe Arg Pro Lys Arg Leu Asn
                165                 170                 175

ttc aaa ctc ttc aac atc caa gtc aag gag gtc acg acg aat gat ggc      576
Phe Lys Leu Phe Asn Ile Gln Val Lys Glu Val Thr Thr Asn Asp Gly
            180                 185                 190

gtc aca acc atc gct aat aac ctt acc agc acg gtt caa gtc ttc tcg      624
Val Thr Thr Ile Ala Asn Asn Leu Thr Ser Thr Val Gln Val Phe Ser
        195                 200                 205

gac tcg gag tac cag ctt ccg tac gtc ctc ggc tct gcg cac cag ggc      672
Asp Ser Glu Tyr Gln Leu Pro Tyr Val Leu Gly Ser Ala His Gln Gly
    210                 215                 220

tgc ctc cct ccg ttc ccg gcg gac gtg ttc atg att ccg caa tac ggc      720
Cys Leu Pro Pro Phe Pro Ala Asp Val Phe Met Ile Pro Gln Tyr Gly
225                 230                 235                 240

tac ctg acg ctc aac aat ggc agc caa gcc gtg gga cgt tca tcc ttt      768
Tyr Leu Thr Leu Asn Asn Gly Ser Gln Ala Val Gly Arg Ser Ser Phe
                245                 250                 255

tac tgc ctg gaa tat ttc cct tct cag atg ctg aga acg ggc aac aac      816
Tyr Cys Leu Glu Tyr Phe Pro Ser Gln Met Leu Arg Thr Gly Asn Asn
            260                 265                 270

ttt acc ttc agc tac acc ttt gag gaa gtg cct ttc cac agc agc tac      864
Phe Thr Phe Ser Tyr Thr Phe Glu Glu Val Pro Phe His Ser Ser Tyr
        275                 280                 285

gcg cac agc cag agc ctg gac cgg ctg atg aat cct ctc atc gac caa      912
Ala His Ser Gln Ser Leu Asp Arg Leu Met Asn Pro Leu Ile Asp Gln
    290                 295                 300

tac ctg tat tac ctg aac aga act caa aat cag tcc gga agt gcc caa      960
Tyr Leu Tyr Tyr Leu Asn Arg Thr Gln Asn Gln Ser Gly Ser Ala Gln
305                 310                 315                 320

aac aag gac ttg ctg ttt agc cgt ggg tct cca gct ggc atg tct gtt      1008
Asn Lys Asp Leu Leu Phe Ser Arg Gly Ser Pro Ala Gly Met Ser Val
                325                 330                 335

cag ccc aaa aac tgg cta cct gga ccc tgt tat cgg cag cag cgc gtt      1056
Gln Pro Lys Asn Trp Leu Pro Gly Pro Cys Tyr Arg Gln Gln Arg Val

            340                 345                 350

tct aaa aca aaa aca gac aac aac aac agc aat ttt acc tgg act ggt 1104
Ser Lys Thr Lys Thr Asp Asn Asn Asn Ser Asn Phe Thr Trp Thr Gly
        355                 360                 365

gct tca aaa tat aac ctc aat ggg cgt gaa tcc atc atc aac cct ggc 1152
Ala Ser Lys Tyr Asn Leu Asn Gly Arg Glu Ser Ile Ile Asn Pro Gly
    370                 375                 380

act gct atg gcc tca cac aaa gac gac gaa gac aag ttc ttt ccc atg 1200
Thr Ala Met Ala Ser His Lys Asp Asp Glu Asp Lys Phe Phe Pro Met
385                 390                 395                 400

agc ggt gtc atg att ttt gga aaa gag agc gcc gga gct tca aac act 1248
Ser Gly Val Met Ile Phe Gly Lys Glu Ser Ala Gly Ala Ser Asn Thr
                405                 410                 415

gca ttg gac aat gtc atg att aca gac gaa gag gaa att aaa gcc act 1296
Ala Leu Asp Asn Val Met Ile Thr Asp Glu Glu Glu Ile Lys Ala Thr
            420                 425                 430

aac cct gtg gcc acc gaa aga ttt ggg acc gtg gca gtc aat ttc cag 1344
Asn Pro Val Ala Thr Glu Arg Phe Gly Thr Val Ala Val Asn Phe Gln
        435                 440                 445

agc agc agc aca gac cct gcg acc gga gat gtg cat gct atg gga gca 1392
Ser Ser Ser Thr Asp Pro Ala Thr Gly Asp Val His Ala Met Gly Ala
    450                 455                 460

tta cct ggc atg gtg tgg caa gat aga gac gtg tac ctg cag ggt ccc 1440
Leu Pro Gly Met Val Trp Gln Asp Arg Asp Val Tyr Leu Gln Gly Pro
465                 470                 475                 480

att tgg gcc aaa att cct cac aca gat gga cac ttt cac ccg tct cct 1488
Ile Trp Ala Lys Ile Pro His Thr Asp Gly His Phe His Pro Ser Pro
                485                 490                 495

ctt atg ggc ggc ttt gga ctc aag aac ccg cct cct cag atc ctc atc 1536
Leu Met Gly Gly Phe Gly Leu Lys Asn Pro Pro Pro Gln Ile Leu Ile
            500                 505                 510

aaa aac acg cct gtt cct gcg aat cct ccg gcg gag ttt tca gct aca 1584
Lys Asn Thr Pro Val Pro Ala Asn Pro Pro Ala Glu Phe Ser Ala Thr
        515                 520                 525

aag ttt gct tca ttc atc acc caa tac tcc aca gga caa gtg agt gtg 1632
Lys Phe Ala Ser Phe Ile Thr Gln Tyr Ser Thr Gly Gln Val Ser Val
    530                 535                 540

gaa att gaa tgg gag ctg cag aaa gaa aac agc aag cgc tgg aat ccc 1680
Glu Ile Glu Trp Glu Leu Gln Lys Glu Asn Ser Lys Arg Trp Asn Pro
545                 550                 555                 560

gaa gtg cag tac aca tcc aat tat gca aaa tct gcc aac gtt gat ttt 1728
Glu Val Gln Tyr Thr Ser Asn Tyr Ala Lys Ser Ala Asn Val Asp Phe
                565                 570                 575

act gtg gac aac aat gga ctt tat act gag cct cgc ccc att ggc acc 1776
Thr Val Asp Asn Asn Gly Leu Tyr Thr Glu Pro Arg Pro Ile Gly Thr
            580                 585                 590

cgt tac ctt acc cgt ccc ctg taa 1800
Arg Tyr Leu Thr Arg Pro Leu
        595

<210> 15

<211>  599
<212>  PRT
<213>  AAV-1

<400>  15
Thr Ala Pro Gly Lys Lys Arg Pro Val Glu Gln Ser Pro Gln Glu Pro
1               5                   10                  15

Asp Ser Ser Ser Gly Ile Gly Lys Thr Gly Gln Gln Pro Ala Lys Lys
            20                  25                  30

Arg Leu Asn Phe Gly Gln Thr Gly Asp Ser Glu Ser Val Pro Asp Pro
        35                  40                  45

Gln Pro Leu Gly Glu Pro Pro Ala Thr Pro Ala Ala Val Gly Pro Thr
    50                  55                  60

Thr Met Ala Ser Gly Gly Gly Ala Pro Met Ala Asp Asn Asn Glu Gly
65                  70                  75                  80

Ala Asp Gly Val Gly Asn Ala Ser Gly Asn Trp His Cys Asp Ser Thr
                85                  90                  95

Trp Leu Gly Asp Arg Val Ile Thr Thr Ser Thr Arg Thr Trp Ala Leu
            100                 105                 110

Pro Thr Tyr Asn Asn His Leu Tyr Lys Gln Ile Ser Ser Ala Ser Thr
        115                 120                 125

Gly Ala Ser Asn Asp Asn His Tyr Phe Gly Tyr Ser Thr Pro Trp Gly
    130                 135                 140

Tyr Phe Asp Phe Asn Arg Phe His Cys His Phe Ser Pro Arg Asp Trp
145                 150                 155                 160

Gln Arg Leu Ile Asn Asn Asn Trp Gly Phe Arg Pro Lys Arg Leu Asn
                165                 170                 175

Phe Lys Leu Phe Asn Ile Gln Val Lys Glu Val Thr Thr Asn Asp Gly
            180                 185                 190

Val Thr Thr Ile Ala Asn Asn Leu Thr Ser Thr Val Gln Val Phe Ser
        195                 200                 205

Asp Ser Glu Tyr Gln Leu Pro Tyr Val Leu Gly Ser Ala His Gln Gly
    210                 215                 220

Cys Leu Pro Pro Phe Pro Ala Asp Val Phe Met Ile Pro Gln Tyr Gly
225                 230                 235                 240

Tyr Leu Thr Leu Asn Asn Gly Ser Gln Ala Val Gly Arg Ser Ser Phe
                245                 250                 255

Tyr Cys Leu Glu Tyr Phe Pro Ser Gln Met Leu Arg Thr Gly Asn Asn
            260                 265                 270

Phe Thr Phe Ser Tyr Thr Phe Glu Glu Val Pro Phe His Ser Ser Tyr
        275                 280                 285

Ala His Ser Gln Ser Leu Asp Arg Leu Met Asn Pro Leu Ile Asp Gln
    290                 295                 300

Tyr Leu Tyr Tyr Leu Asn Arg Thr Gln Asn Gln Ser Gly Ser Ala Gln
305                 310                 315                 320

Asn Lys Asp Leu Leu Phe Ser Arg Gly Ser Pro Ala Gly Met Ser Val
                325                 330                 335

Gln Pro Lys Asn Trp Leu Pro Gly Pro Cys Tyr Arg Gln Gln Arg Val
            340                 345                 350

Ser Lys Thr Lys Thr Asp Asn Asn Asn Ser Asn Phe Thr Trp Thr Gly
        355                 360                 365

Ala Ser Lys Tyr Asn Leu Asn Gly Arg Glu Ser Ile Ile Asn Pro Gly
    370                 375                 380

Thr Ala Met Ala Ser His Lys Asp Asp Glu Asp Lys Phe Phe Pro Met
385                 390                 395                 400

Ser Gly Val Met Ile Phe Gly Lys Glu Ser Ala Gly Ala Ser Asn Thr
                405                 410                 415

Ala Leu Asp Asn Val Met Ile Thr Asp Glu Glu Glu Ile Lys Ala Thr
            420                 425                 430

Asn Pro Val Ala Thr Glu Arg Phe Gly Thr Val Ala Val Asn Phe Gln
        435                 440                 445

Ser Ser Ser Thr Asp Pro Ala Thr Gly Asp Val His Ala Met Gly Ala
    450                 455                 460

Leu Pro Gly Met Val Trp Gln Asp Arg Asp Val Tyr Leu Gln Gly Pro
465                 470                 475                 480

Ile Trp Ala Lys Ile Pro His Thr Asp Gly His Phe His Pro Ser Pro
                485                 490                 495

Leu Met Gly Gly Phe Gly Leu Lys Asn Pro Pro Pro Gln Ile Leu Ile
            500                 505                 510

Lys Asn Thr Pro Val Pro Ala Asn Pro Pro Ala Glu Phe Ser Ala Thr
        515                 520                 525

Lys Phe Ala Ser Phe Ile Thr Gln Tyr Ser Thr Gly Gln Val Ser Val
    530                 535                 540

Glu Ile Glu Trp Glu Leu Gln Lys Glu Asn Ser Lys Arg Trp Asn Pro
545                 550                 555                 560

Glu Val Gln Tyr Thr Ser Asn Tyr Ala Lys Ser Ala Asn Val Asp Phe
                565                 570                 575

Thr Val Asp Asn Asn Gly Leu Tyr Thr Glu Pro Arg Pro Ile Gly Thr
            580                 585                 590

Arg Tyr Leu Thr Arg Pro Leu
        595


<210>  16
<211>  1605
<212>  DNA
<213>  AAV-1

<220>
<221>  CDS
<222>  (1)..(1602)
<223>

<400>  16
atg gct tca ggc ggt ggc gca cca atg gca gac aat aac gaa ggc gcc      48
Met Ala Ser Gly Gly Gly Ala Pro Met Ala Asp Asn Asn Glu Gly Ala
1               5                   10                  15

gac gga gtg ggt aat gcc tca gga aat tgg cat tgc gat tcc aca tgg      96

Asp Gly Val Gly Asn Ala Ser Gly Asn Trp His Cys Asp Ser Thr Trp
            20                  25                  30

ctg ggc gac aga gtc atc acc acc agc acc cgc acc tgg gcc ttg ccc      144
Leu Gly Asp Arg Val Ile Thr Thr Ser Thr Arg Thr Trp Ala Leu Pro
        35                  40                  45

acc tac aat aac cac ctc tac aag caa atc tcc agt gct tca acg ggg      192
Thr Tyr Asn Asn His Leu Tyr Lys Gln Ile Ser Ser Ala Ser Thr Gly
    50                  55                  60

gcc agc aac gac aac cac tac ttc ggc tac agc acc ccc tgg ggg tat      240
Ala Ser Asn Asp Asn His Tyr Phe Gly Tyr Ser Thr Pro Trp Gly Tyr
65                  70                  75                  80

ttt gat ttc aac aga ttc cac tgc cac ttt tca cca cgt gac tgg cag      288
Phe Asp Phe Asn Arg Phe His Cys His Phe Ser Pro Arg Asp Trp Gln
                85                  90                  95

cga ctc atc aac aac aat tgg gga ttc cgg ccc aag aga ctc aac ttc      336
Arg Leu Ile Asn Asn Asn Trp Gly Phe Arg Pro Lys Arg Leu Asn Phe
            100                 105                 110

aaa ctc ttc aac atc caa gtc aag gag gtc acg acg aat gat ggc gtc      384
Lys Leu Phe Asn Ile Gln Val Lys Glu Val Thr Thr Asn Asp Gly Val
        115                 120                 125

aca acc atc gct aat aac ctt acc agc acg gtt caa gtc ttc tcg gac      432
Thr Thr Ile Ala Asn Asn Leu Thr Ser Thr Val Gln Val Phe Ser Asp
    130                 135                 140

tcg gag tac cag ctt ccg tac gtc ctc ggc tct gcg cac cag ggc tgc      480
Ser Glu Tyr Gln Leu Pro Tyr Val Leu Gly Ser Ala His Gln Gly Cys
145                 150                 155                 160

ctc cct ccg ttc ccg gcg gac gtg ttc atg att ccg caa tac ggc tac      528
Leu Pro Pro Phe Pro Ala Asp Val Phe Met Ile Pro Gln Tyr Gly Tyr
                165                 170                 175

ctg acg ctc aac aat ggc agc caa gcc gtg gga cgt tca tcc ttt tac      576
Leu Thr Leu Asn Asn Gly Ser Gln Ala Val Gly Arg Ser Ser Phe Tyr
            180                 185                 190

tgc ctg gaa tat ttc cct tct cag atg ctg aga acg ggc aac aac ttt      624
Cys Leu Glu Tyr Phe Pro Ser Gln Met Leu Arg Thr Gly Asn Asn Phe
        195                 200                 205

acc ttc agc tac acc ttt gag gaa gtg cct ttc cac agc agc tac gcg      672
Thr Phe Ser Tyr Thr Phe Glu Glu Val Pro Phe His Ser Ser Tyr Ala
    210                 215                 220

cac agc cag agc ctg gac cgg ctg atg aat cct ctc atc gac caa tac      720
His Ser Gln Ser Leu Asp Arg Leu Met Asn Pro Leu Ile Asp Gln Tyr
225                 230                 235                 240

ctg tat tac ctg aac aga act caa aat cag tcc gga agt gcc caa aac      768
Leu Tyr Tyr Leu Asn Arg Thr Gln Asn Gln Ser Gly Ser Ala Gln Asn
                245                 250                 255

aag gac ttg ctg ttt agc cgt ggg tct cca gct ggc atg tct gtt cag      816
Lys Asp Leu Leu Phe Ser Arg Gly Ser Pro Ala Gly Met Ser Val Gln
            260                 265                 270

ccc aaa aac tgg cta cct gga ccc tgt tat cgg cag cag cgc gtt tct      864
Pro Lys Asn Trp Leu Pro Gly Pro Cys Tyr Arg Gln Gln Arg Val Ser
        275                 280                 285

aaa aca aaa aca gac aac aac aac agc aat ttt acc tgg act ggt gct      912

Lys Thr Lys Thr Asp Asn Asn Asn Ser Asn Phe Thr Trp Thr Gly Ala
    290                 295                 300

tca aaa tat aac ctc aat ggg cgt gaa tcc atc atc aac cct ggc act      960
Ser Lys Tyr Asn Leu Asn Gly Arg Glu Ser Ile Ile Asn Pro Gly Thr
305                 310                 315                 320

gct atg gcc tca cac aaa gac gac gaa gac aag ttc ttt ccc atg agc      1008
Ala Met Ala Ser His Lys Asp Asp Glu Asp Lys Phe Phe Pro Met Ser
                325                 330                 335

ggt gtc atg att ttt gga aaa gag agc gcc gga gct tca aac act gca      1056
Gly Val Met Ile Phe Gly Lys Glu Ser Ala Gly Ala Ser Asn Thr Ala
            340                 345                 350

ttg gac aat gtc atg att aca gac gaa gag gaa att aaa gcc act aac      1104
Leu Asp Asn Val Met Ile Thr Asp Glu Glu Glu Ile Lys Ala Thr Asn
        355                 360                 365

cct gtg gcc acc gaa aga ttt ggg acc gtg gca gtc aat ttc cag agc      1152
Pro Val Ala Thr Glu Arg Phe Gly Thr Val Ala Val Asn Phe Gln Ser
    370                 375                 380

agc agc aca gac cct gcg acc gga gat gtg cat gct atg gga gca tta      1200
Ser Ser Thr Asp Pro Ala Thr Gly Asp Val His Ala Met Gly Ala Leu
385                 390                 395                 400

cct ggc atg gtg tgg caa gat aga gac gtg tac ctg cag ggt ccc att      1248
Pro Gly Met Val Trp Gln Asp Arg Asp Val Tyr Leu Gln Gly Pro Ile
                405                 410                 415

tgg gcc aaa att cct cac aca gat gga cac ttt cac ccg tct cct ctt      1296
Trp Ala Lys Ile Pro His Thr Asp Gly His Phe His Pro Ser Pro Leu
            420                 425                 430

atg ggc ggc ttt gga ctc aag aac ccg cct cct cag atc ctc atc aaa      1344
Met Gly Gly Phe Gly Leu Lys Asn Pro Pro Pro Gln Ile Leu Ile Lys
        435                 440                 445

aac acg cct gtt cct gcg aat cct ccg gcg gag ttt tca gct aca aag      1392
Asn Thr Pro Val Pro Ala Asn Pro Pro Ala Glu Phe Ser Ala Thr Lys
    450                 455                 460

ttt gct tca ttc atc acc caa tac tcc aca gga caa gtg agt gtg gaa      1440
Phe Ala Ser Phe Ile Thr Gln Tyr Ser Thr Gly Gln Val Ser Val Glu
465                 470                 475                 480

att gaa tgg gag ctg cag aaa gaa aac agc aag cgc tgg aat ccc gaa      1488
Ile Glu Trp Glu Leu Gln Lys Glu Asn Ser Lys Arg Trp Asn Pro Glu
                485                 490                 495

gtg cag tac aca tcc aat tat gca aaa tct gcc aac gtt gat ttt act      1536
Val Gln Tyr Thr Ser Asn Tyr Ala Lys Ser Ala Asn Val Asp Phe Thr
            500                 505                 510

gtg gac aac aat gga ctt tat act gag cct cgc ccc att ggc acc cgt      1584
Val Asp Asn Asn Gly Leu Tyr Thr Glu Pro Arg Pro Ile Gly Thr Arg
        515                 520                 525

tac ctt acc cgt ccc ctg taa                                          1605
Tyr Leu Thr Arg Pro Leu
    530

<210>  17
<211>  534
<212>  PRT
<213>  AAV-1

<400> 17
Met Ala Ser Gly Gly Gly Ala Pro Met Ala Asp Asn Asn Glu Gly Ala
1               5                   10                  15

Asp Gly Val Gly Asn Ala Ser Gly Asn Trp His Cys Asp Ser Thr Trp
            20                  25                  30

Leu Gly Asp Arg Val Ile Thr Thr Ser Thr Arg Thr Trp Ala Leu Pro
        35                  40                  45

Thr Tyr Asn Asn His Leu Tyr Lys Gln Ile Ser Ser Ala Ser Thr Gly
    50                  55                  60

Ala Ser Asn Asp Asn His Tyr Phe Gly Tyr Ser Thr Pro Trp Gly Tyr
65                  70                  75                  80

Phe Asp Phe Asn Arg Phe His Cys His Phe Ser Pro Arg Asp Trp Gln
                85                  90                  95

Arg Leu Ile Asn Asn Asn Trp Gly Phe Arg Pro Lys Arg Leu Asn Phe
            100                 105                 110

Lys Leu Phe Asn Ile Gln Val Lys Glu Val Thr Thr Asn Asp Gly Val
        115                 120                 125

Thr Thr Ile Ala Asn Asn Leu Thr Ser Thr Val Gln Val Phe Ser Asp
    130                 135                 140

Ser Glu Tyr Gln Leu Pro Tyr Val Leu Gly Ser Ala His Gln Gly Cys
145                 150                 155                 160

Leu Pro Pro Phe Pro Ala Asp Val Phe Met Ile Pro Gln Tyr Gly Tyr
                165                 170                 175

Leu Thr Leu Asn Asn Gly Ser Gln Ala Val Gly Arg Ser Ser Phe Tyr
            180                 185                 190

Cys Leu Glu Tyr Phe Pro Ser Gln Met Leu Arg Thr Gly Asn Asn Phe
        195                 200                 205

Thr Phe Ser Tyr Thr Phe Glu Glu Val Pro Phe His Ser Ser Tyr Ala
    210                 215                 220

His Ser Gln Ser Leu Asp Arg Leu Met Asn Pro Leu Ile Asp Gln Tyr
225                 230                 235                 240

Leu Tyr Tyr Leu Asn Arg Thr Gln Asn Gln Ser Gly Ser Ala Gln Asn
                245                 250                 255

Lys Asp Leu Leu Phe Ser Arg Gly Ser Pro Ala Gly Met Ser Val Gln
            260                 265                 270

Pro Lys Asn Trp Leu Pro Gly Pro Cys Tyr Arg Gln Gln Arg Val Ser
        275                 280                 285

Lys Thr Lys Thr Asp Asn Asn Asn Ser Asn Phe Thr Trp Thr Gly Ala
    290                 295                 300

Ser Lys Tyr Asn Leu Asn Gly Arg Glu Ser Ile Ile Asn Pro Gly Thr
305                 310                 315                 320

Ala Met Ala Ser His Lys Asp Asp Glu Asp Lys Phe Phe Pro Met Ser
                325                 330                 335

Gly Val Met Ile Phe Gly Lys Glu Ser Ala Gly Ala Ser Asn Thr Ala
            340                 345                 350

Leu Asp Asn Val Met Ile Thr Asp Glu Glu Glu Ile Lys Ala Thr Asn
        355                 360                 365

Pro Val Ala Thr Glu Arg Phe Gly Thr Val Ala Val Asn Phe Gln Ser
    370                 375                 380

Ser Ser Thr Asp Pro Ala Thr Gly Asp Val His Ala Met Gly Ala Leu
385                 390                 395                 400

Pro Gly Met Val Trp Gln Asp Arg Asp Val Tyr Leu Gln Gly Pro Ile
                405                 410                 415

Trp Ala Lys Ile Pro His Thr Asp Gly His Phe His Pro Ser Pro Leu
            420                 425                 430

Met Gly Gly Phe Gly Leu Lys Asn Pro Pro Pro Gln Ile Leu Ile Lys
        435                 440                 445

Asn Thr Pro Val Pro Ala Asn Pro Pro Ala Glu Phe Ser Ala Thr Lys
    450                 455                 460

Phe Ala Ser Phe Ile Thr Gln Tyr Ser Thr Gly Gln Val Ser Val Glu
465                 470                 475                 480

Ile Glu Trp Glu Leu Gln Lys Glu Asn Ser Lys Arg Trp Asn Pro Glu
                485                 490                 495

Val Gln Tyr Thr Ser Asn Tyr Ala Lys Ser Ala Asn Val Asp Phe Thr
            500                 505                 510

Val Asp Asn Asn Gly Leu Tyr Thr Glu Pro Arg Pro Ile Gly Thr Arg
        515                 520                 525

Tyr Leu Thr Arg Pro Leu
    530

<210>  18
<211>  4681
<212>  DNA
<213>  AAV-2

<400>  18
ttggccactc cctctctgcg cgctcgctcg ctcactgagg ccgggcgacc aaaggtcgcc      60

cgacgcccgg gctttgcccg ggcggcctca gtgagcgagc gagcgcgcag agagggagtg     120

gccaactcca tcactagggg ttcctggagg ggtggagtcg tgacgtgaat tacgtcatag     180

ggttagggag gtcctgtatt agaggtcacg tgagtgtttt gcgacatttt gcgacaccat     240

gtggtcacgc tgggtattta agcccgagtg agcacgcagg gtctccattt tgaagcggga     300

ggtttgaacg cgcagccgcc atgccggggt tttacgagat tgtgattaag gtccccagcg     360

accttgacgg gcatctgccc ggcatttctg acagctttgt gaactgggtg gccgagaagg     420

aatgggagtt gccgccagat tctgacatgg atctgaatct gattgagcag gcacccctga     480

ccgtggccga gaagctgcag cgcgactttc tgacggaatg gcgccgtgtg agtaaggccc     540

cggaggccct tttctttgtg caatttgaga agggagagag ctacttccac atgcacgtgc     600

tcgtggaaac caccggggtg aaatccatgg ttttgggacg tttcctgagt cagattcgcg     660

aaaaactgat tcagagaatt taccgcggga tcgagccgac tttgccaaac tggttcgcgg     720

tcacaaagac cagaaatggc gccggaggcg ggaacaaggt ggtggatgag tgctacatcc     780

ccaattactt gctccccaaa acccagcctg agctccagtg ggcgtggact aatatggaac 840
agtatttaag cgcctgtttg aatctcacgg agcgtaaacg gttggtggcg cagcatctga 900
cgcacgtgtc gcagacgcag gagcagaaca aagagaatca gaatcccaat tctgatgcgc 960
cggtgatcag atcaaaaact tcagccaggt acatggagct ggtcgggtgg ctcgtggaca 1020
aggggattac ctcggagaag cagtggatcc aggaggacca ggcctcatac atctccttca 1080
atgcggcctc caactcgcgg tcccaaatca aggctgcctt ggacaatgcg ggaaagatta 1140
tgagcctgac taaaaccgcc cccgactacc tggtgggcca gcagcccgtg gaggacattt 1200
ccagcaatcg gatttataaa attttggaac taaacgggta cgatccccaa tatgcggctt 1260
ccgtctttct gggatgggcc acgaaaaagt tcggcaagag gaacaccatc tggctgtttg 1320
ggcctgcaac taccgggaag accaacatcg cggaggccat agcccacact gtgcccttct 1380
acgggtgcgt aaactggacc aatgagaact ttcccttcaa cgactgtgtc gacaagatgg 1440
tgatctggtg ggaggagggg aagatgaccg ccaaggtcgt ggagtcggcc aaagccattc 1500
tcggaggaag caaggtgcgc gtggaccaga aatgcaagtc ctcggcccag atagacccga 1560
ctcccgtgat cgtcacctcc aacaccaaca tgtgcgccgt gattgacggg aactcaacga 1620
ccttcgaaca ccagcagccg ttgcaagacc ggatgttcaa atttgaactc acccgccgtc 1680
tggatcatga ctttgggaag gtcaccaagc aggaagtcaa agactttttc cggtgggcaa 1740
aggatcacgt ggttgaggtg gagcatgaat tctacgtcaa aaagggtgga gccaagaaaa 1800
gacccgcccc cagtgacgca gatataagtg agcccaaacg ggtgcgcgag tcagttgcgc 1860
agccatcgac gtcagacgcg gaagcttcga tcaactacgc agacaggtac caaaacaaat 1920
gttctcgtca cgtgggcatg aatctgatgc tgtttccctg cagacaatgc gagagaatga 1980
atcagaattc aaatatctgc ttcactcacg gacagaaaga ctgtttagag tgctttcccg 2040
tgtcagaatc tcaaccggtt tctgtcgtca aaaaggcgta tcagaaactg tgctacattc 2100
atcatatcat gggaaaggtg ccagacgctt gcactgcctg cgatctggtc aatgtggatt 2160
tggatgactg catctttgaa caataaatga tttaaatcag gtatggctgc cgatggttat 2220
cttccagatt ggctcgagga cactctctct gaaggaataa gacagtggtg gaagctcaaa 2280
cctggcccac caccaccaaa gcccgcagag cggcataagg acgacagcag gggtcttgtg 2340
cttcctgggt acaagtacct cggacccttc aacggactcg acaagggaga gccggtcaac 2400
gaggcagacg ccgcggccct cgagcacgac aaagcctacg accggcagct cgacagcgga 2460
gacaacccgt acctcaagta caaccacgcc gacgcggagt ttcaggagcg ccttaaagaa 2520
gatacgtctt ttgggggcaa cctcggacga gcagtcttcc aggcgaaaaa gagggttctt 2580
gaacctctcg gcctggttga ggaacctgtt aagacggctc cgggaaaaaa gaggccggta 2640
gagcactctc ctgtggagcc agactcctcc tcgggaaccg gaaagccggg ccagcagcct 2700
gcaagaaaaa gattgaattt tggtcagact ggagacgcag actcagtacc tgacccccag 2760
cctctcggac agccaccagc agccccctct ggtctgggaa ctaatacgat ggctacaggc 2820

agtggcgcac caatggcaga caataacgag ggcgccgacg gagtgggtaa ttcctccgga 2880
aattggcatt gcgattccac atggatgggc gacagagtca tcaccaccag cacccgaacc 2940
tgggccctgc ccacctacaa caaccacctc tacaaacaaa tttccagcca atcaggagcc 3000
tcgaacgaca atcactactt tggctacagc accccttggg ggtattttga cttcaacaga 3060
ttccactgcc acttttcacc acgtgactgg caaagactca tcaacaacaa ctggggattc 3120
cgacccaaga gactcaactt caacctcttt aacattcaag tcaaagaggt cacgcagaat 3180
gacggtacga cgacgattgc caataacctt accagcacgg ttcaggtgtt tactgactcg 3240
gagtaccagc tcccgtacgt cctcggctcg gcgcatcaag gatgcctccc gccgttccca 3300
gcagacgtct tcatggtgcc acagtatgga tacctcaccc tgaacaacgg gagtcaggca 3360
gtaggacgct cttcatttta ctgcctggag tactttcctt ctcagatgct gcgtaccgga 3420
aacaacttta ccttcagcta cacttttgag gacgttcctt tccacagcag ctacgctcac 3480
agccagagtc tggaccgtct catgaatcct ctcatcgacc agtacctgta ttacttgagc 3540
agaacaaaca ctccaagtgg aaccaccacg cagtcaaggc ttcagttttc tcaggcccca 3600
gccagtgaca ttcgggacca gtctaggaac tggcttcctg gaccctgtta ccgccagcag 3660
cgagtatgaa agacatctgc ggataacaac aacagtgaat actcgtggac tggagctacc 3720
aagtaccacc tcaatggcag agactctctg gtgaatccgg ggcccgccat ggcaagccac 3780
aaggacgatg aagaaaagtt ttttcctcag agcggggttc tcatctttgg gaagcaaggc 3840
tcagagaaaa caaatgtgaa cattgaaaag gtcatgatta cagacgaaga ggaaatccca 3900
acaaccaatc ccgtggctac ggagcagtat ggttctgtat ctaccaacct ccagagaggc 3960
aacagacaag cagctaccgc agatgtcaac acacaaggcg ttcttccagg catggtctgg 4020
caggacagag atgtgtacct tcaggggccc atctgggcaa agattccaca cacggacgga 4080
cattttcacc cctctcccct catgggtgga ttcggactta aacaccctcc tccacagatt 4140
ctcatcaaga acaccccggt acctgcgaat ccttcgacca ccttcagtgc ggcaaagttt 4200
gcttccttca tcacacagta ctccacggga cacggtcagc gtggagatcg agtgggagct 4260
gcagaacgaa aacagcaaac gctggaatcc cgaaattcag tacacttcca actacaacaa 4320
gtctgttaat cgtggacttt accgtggata ctaatggcgt gtattcagag cctcgcccca 4380
ttggcaccag atacctgact cgtaatctgt aattgcttgt taatcaataa accgtttaat 4440
tcgtttcagt tgaactttgg tctctgcgta tttctttctt atctagtttc catggctacg 4500
tagataagta gcatggcggg ttaatcatta actacaagga acccctagtg atggagttgg 4560
ccactccctc tctgcgcgct cgctcgctca ctgaggccgg gcgaccaaag gtcgcccgac 4620
gcccgggctt tgccccggcg gcctcagtga gcgagcgagc gcgcagagag ggagtgggca 4680
a 4681

<210> 19
<211> 4683

<212>  DNA
<213>  AAV-6

<400>  19
ttggccactc cctctctgcg cgctcgctcg ctcactgagg ccgggcgacc aaaggtcgcc      60

cgacgcccgg gctttgcccg ggcggcctca gtgagcgagc gagcgcgcag agagggagtg     120

gccaactcca tcactagggg ttcctggagg ggtggagtcg tgacgtgaat tacgtcatag     180

ggttagggag gtcctgtatt agaggtcacg tgagtgtttt gcgacatttt gcgacaccat     240

gtggtcacgc tgggtattta agcccgagtg agcacgcagg gtctccattt tgaagcggga     300

ggtttgaacg cgcagcgcca tgccggggtt ttacgagatt gtgattaagg tccccagcga     360

ccttgacgag catctgcccg gcatttctga cagctttgtg aactgggtgg ccgagaagga     420

atgggagttg ccgccagatt ctgacatgga tctgaatctg attgagcagg cacccctgac     480

cgtggccgag aagctgcagc gcgacttcct ggtccactgg cgccgcgtga gtaaggcccc     540

ggaggccctc ttctttgttc agttcgagaa gggcgagtcc tacttccacc tccatattct     600

ggtggagacc acgggggtca aatccatggt gctgggccgc ttcctgagtc agattagcga     660

caagctggtg cagaccatct accgcgggat cgagccgacc ctgcccaact ggttcgcggt     720

gaccaagacg cgtaatggcg ccggaggggg gaacaaggtg gtggacgagt gctacatccc     780

caactacctc ctgcccaaga ctcagcccga gctgcagtgg gcgtggacta acatggagga     840

gtatataagc gcgtgtttaa acctggccga gcgcaaacgg ctcgtggcgc acgacctgac     900

ccacgtcagc cagacccagg agcagaacaa ggagaatctg aaccccaatt ctgacgcgcc     960

tgtcatccgg tcaaaaacct ccgcacgcta catggagctg gtcgggtggc tggtggaccg    1020

gggcatcacc tccgagaagc agtggatcca ggaggaccag gcctcgtaca tctccttcaa    1080

cgccgcctcc aactcgcggt cccagatcaa ggccgctctg gacaatgccg gcaagatcat    1140

ggcgctgacc aaatccgcgc ccgactacct ggtaggcccc gctccgcccg ccgacattaa    1200

aaccaaccgc atttaccgca tcctggagct gaacggctac gaccctgcct acgccggctc    1260

cgtctttctc ggctgggccc agaaaaggtt cggaaaacgc aacaccatct ggctgtttgg    1320

gccggccacc acgggcaaga ccaacatcgc ggaagccatc gcccacgccg tgcccttcta    1380

cggctgcgtc aactggacca atgagaactt tcccttcaac gattgcgtcg acaagatggt    1440

gatctggtgg gaggagggca agatgacggc caaggtcgtg gagtccgcca aggccattct    1500

cggcggcagc aaggtgcgcg tggaccaaaa gtgcaagtcg tccgcccaga tcgatcccac    1560

ccccgtgatc gtcacctcca acaccaacat gtgcgccgtg attgacggga acagcaccac    1620

cttcgagcac cagcagccgt tgcaggaccg gatgttcaaa tttgaactca cccgccgtct    1680

ggagcatgac tttggcaagg tgacaaagca ggaagtcaaa gagttcttcc gctgggcgca    1740

ggatcacgtg accgaggtgg cgcatgagtt ctacgtcaga aagggtggag ccaacaacag    1800

acccgccccc gatgacgcgg ataaaagcga gcccaagcgg gcctgcccct cagtcgcgga    1860

tccatcgacg tcagacgcgg aaggagctcc ggtggacttt gccgacaggt accaaaacaa    1920

atgttctcgt cacgcgggca tgcttcagat gctgtttccc tgcaaaacat gcgagagaat 1980
gaatcagaat ttcaacattt gcttcacgca cgggaccaga gactgttcag aatgtttccc 2040
cggcgtgtca gaatctcaac cggtcgtcag aaagaggacg tatcggaaac tctgtgccat 2100
tcatcatctg ctggggcggg ctcccgagat tgcttgctcg gcctgcgatc tggtcaacgt 2160
ggatctggat gactgtgttt ctgagcaata aatgacttaa accaggtatg gctgccgatg 2220
gttatcttcc agattggctc gaggacaacc tctctgaggg cattcggcag tggtgggact 2280
tgaaacctgg agccccgaaa cccaaagcca accagcaaaa gcaggacgac ggccggggtc 2340
tggtgcttcc tggctacaag tacctcggac ccttcaacgg actcgacaag ggggagcccg 2400
tcaacgcggc ggatgcagcg gccctcgagc acgacaaggc ctacgaccag cagctcaaag 2460
cgggtgacaa tccgtacctg cggtataacc acgccgacgc cgagtttcag gagcgtctgc 2520
aagaagatac gtcttttggg ggcaacctcg ggcgagcagt cttccaggcc aagaagaggg 2580
ttctcgaacc ttttggtctg gttgaggaag gtgctaagac ggctcctgga aagaaacgtc 2640
cggtagagca gtcgccacaa gagccagact cctcctcggg cattggcaag acaggccagc 2700
agcccgctaa aaagagactc aattttggtc agactggcga ctcagagtca gtccccgacc 2760
cacaacctct cggagaacct ccagcaaccc ccgctgctgt gggacctact acaatggctt 2820
caggcggtgg cgcaccaatg gcagacaata acgaaggcgc cgacggagtg ggtaatgcct 2880
caggaaattg gcattgcgat tccacatggc tgggcgacag agtcatcacc accagcaccc 2940
gaacatgggc cttgcccacc tataacaacc acctctacaa gcaaatctcc agtgcttcaa 3000
cgggggccag caacgacaac cactacttcg gctacagcac cccctggggg tattttgatt 3060
tcaacagatt ccactgccat ttctcaccac gtgactggca gcgactcatc aacaacaatt 3120
ggggattccg gcccaagaga ctcaacttca agctcttcaa catccaagtc aaggaggtca 3180
cgacgaatga tggcgtcacg accatcgcta ataaccttac cagcacggtt caagtcttgt 3240
cggactcgga gtaccagttc ccgtacgtcc tcggctctgc gcaccagggc tgcctccctc 3300
cgttcccggc ggacgtgttc atgattccgc agtacggcta cctaacgctc aacaatggca 3360
gccaggcagt gggacgctca tccttttact gcctggaata tttcccatcg cagatgctga 3420
gaacgggcaa taactttacc ttcagctaca ccttcgagga cgtgcctttc cacagcagct 3480
acgcgcacag ccagagcctg gaccggctga tgaatcctct catcgaccag tacctgtatt 3540
acctgaacag aactcacaat cagtccggaa gtgcccaaaa caaggacttg ctgtttagcc 3600
gtgggtctcc agctggcatg tctgttcagc ccaaaaactg gctacctgga ccctgttacc 3660
ggcagcagcg cgtttctaaa acaaaaacag acaacaacaa cagcaacttt acctggactg 3720
gtgcttcaaa atataacctt aatgggcgtg aatctataat caaccctggc actgctatgg 3780
cctcacacaa agacgacaaa gacaagttct ttcccatgag cggtgtcatg atttttggaa 3840
aggagagcgc cggagcttca aacactgcat tggacaatgt catgatcaca gacgaagagg 3900
aaatcaaagc cactaacccc gtggccaccg aaagatttgg gactgtggca gtcaatctcc 3960

agagcagcag cacagaccct gcgaccggag atgtgcatgt tatgggagcc ttacctggaa 4020

tggtgtggca agacagagac gtatacctgc agggtcctat ttgggccaaa attcctcaca 4080

cggatggaca ctttcacccg tctcctctca tgggcggctt tggacttaag cacccgcctc 4140

ctcagatcct catcaaaaac acgcctgttc ctgcgaatcc tccggcagag ttttcggcta 4200

caaagtttgc ttcattcatc acccagtatt ccacaggaca agtgagcgtg gagattgaat 4260

gggagctgca gaaagaaaac agcaaacgct ggaatcccga agtgcagtat acatctaact 4320

atgcaaaatc tgccaacgtt gatttcactg tggacaacaa tggactttat actgagcctc 4380

gccccattgg cacccgttac ctcacccgtc ccctgtaatt gtgtgttaat caataaaccg 4440

gttaattcgt gtcagttgaa ctttggtctc atgtccttat tatcttatct ggtcaccata 4500

gcaaccggtt acacattaac tgcttagttg cgcttcgcga atacccctag tgatggagtt 4560

gcccactccc tctatgcgcg ctcgctcgct cggtggggcc ggcagagcag agctctgccg 4620

tctgcggacc tttggtccgc aggccccacc gagcgagcga gcgcgcatag agggagtggc 4680

caa 4683

<210> 20
<211> 16
<212> DNA
<213> Artifical sequence

<220>
<223> rep binding motif

<400> 20
gctcgctcgc tcgctg 16

## Description

This work was supported by the National Institutes of Health, grant no. P30 DK47757-06 and PO1 HD32649-04. The US government may have certain rights in this invention.

### Field of the Invention

This invention relates generally to viral vector, and more particularly, to recombinant viral vectors useful for gene delivery.

### Background of the Invention

Adeno-associated viruses are small, single-stranded DNA viruses which require helper virus to facilitate efficient replication [K.I. Berns, Parvoviridae: the viruses and their replication, p. 1007-1041, in F.N. Fields et al., Fundamental virology, 3rd ed., vol. 2, (Lippencott-Raven Publishers, Philadelphia, PA) (1995)]. The 4.7 kb genome of AAV is characterized by two inverted terminal repeats (ITR) and two open reading frames which encode the Rep proteins and Cap proteins, respectively. The Rep reading frame encodes four proteins of molecular weight 78 kD, 68 kD, 52 kD and 40 kD. These proteins function mainly in regulating AAV replication and integration of the AAV into a host cell's chromosomes. The Cap reading frame encodes three structural proteins in molecular weight 85 kD (VP 1), 72 kD (VP2) and 61 kD (VP3) [Berns, cited above]. More than 80% of total proteins in AAV virion comprise VP3. The two ITRs are the only cis elements essential for AAV replication, packaging and integration. There are two conformations of AAV ITRs called "flip" and "flop". These differences in conformation originated from the replication model of adeno-associated virus which use the ITR to initiate and reinitiate the replication [R.O. Snyder et al., J. Virol., 67:6096-6104 (1993); K.I. Berns, Microbiological Reviews, 54:316-329 (1990)].

AAVs have been found in many animal species, including primates, canine, fowl and human [F.A. Murphy et al., "The Classification and Nomenclature of Viruses: Sixth Report of the International Committee on Taxonomy of Viruses", Archives of Virology, (Springer-Verlag, Vienna) (1995)].

In addition to five known primate AAVs (AAV-1 to AAV-5), AAV-6, another serotype closely related to AAV-2 and AAV-1 has also been isolated [E. A. Rutledge et al., J. Virol., 72:309-319 (1998)]. Among all known AAV serotypes, AAV-2 is perhaps the most well-characterized serotype, because its infectious clone was the first made [R.J. Samulski et al., Proc. Natl, Acad, Sci. USA, 79:2077-2081 (1982)]. Subsequently, the full sequences for AAV-3A, AAV-3B, AAV-4 and AAV-6 have also been determined [Rutledge, cited above; J.A.Chiorini et al., J. Virol., 71:6823-6833 (1997); S. Muramatsu et al., Virol., 221:208-217 (1996)]. Generally, all AAVs share more than 80% homology in nucleotide sequence.

A number of unique properties make AAV a promising vector for human gene therapy [Muzyczka, Current Topics in Microbiology and Immunology. 158:97-129 (1992)]. Unlike other viral vectors, AAVs have not been shown to be associated with any known human disease and are generally not considered pathogenic. Wild type AAV is capable of integrating into host chromosomes in a site specific manner [R. M. Kotin et al., Proc, Natl. Acad. Sci, USA, 87:2211-2215 (1990)- R.J. Samulski, EMBO J., 10(12):3941-3950 (1991)]. Recombinant AAV vectors can integrate into tissue cultured cells in chromosome 19 if the rep proteins are supplied in *trans* [C. Balague et al., J. Virol., 71:3299-3306 (1997); R. T. Surosky et al., J. Virol., 71:7951-7959 (1997)]. The integrated genomes of AAV have been shown to allow long term gene expression in a number of tissues, including, muscle, liver, and brain [K. J. Fisher, Nature Med., 3(3):306-312 (1997); R. 0. Snyder et al., Nature Genetics, 16:270-276 (1997); X Xiao et al., Experimental Neurology, 144:113-124 (1997); Xiao, J. Virol., 70(11):8098-8108 (1996)].

AAV-2 has been shown to be present in about 80-90% of the human population. Earlier studies showed that neutralizing antibodies for AAV-2 are prevalent [W. P. Parks et al., J. Virol., 2:716-722 (1970)]. The presence of such antibodies may significantly decrease the usefulness of AAV vectors based on AAV-2 despite its other merits. What are needed in the art are vectors characterized by the advantages of AAV-2, including those described above, without the disadvantages, including the presence of neutralizing antibodies.

### Summary of the Invention

In one aspect, the invention provides an isolated AAV-1 nucleic acid molecule which is selected from among SEQ ID NO: 1, the strand complementary to SEQ ID NO: 1, and cDNA and RNA sequences complementary to SEQ ID NO: 1 and its complementary strand.

In another aspect, the present invention provides AAV ITR sequences, which include the 5' ITR sequences, nt 1 to 143 of SEQ ID NO: 1; the 3' ITR sequences, nt 4576 to 4718 of SEQ ID NO: 1, and fragments thereof.

In yet another aspect, the present invention provides a recombinant vector comprising an AAV-1 ITR and a selected transgene. Preferably, the vector comprises both the 5' and 3' AAV-1 ITRs between which the selected transgene is located.

In still another aspect, the invention provides a recombinant vector comprising an AAV-1 P5 promoter having the sequence of nt 236 to 299 of SEQ ID NO: 1 or a functional fragment thereof.

In a further aspect, the present invention provides a nucleic acid molecule encoding an AAV-1 rep coding region and an AAV-1 cap coding region. In still another aspect, the present invention provides a host cell transduced with a recombinant viral vector of the invention. The invention further provides a host cell stably transduced with an AAV-1 P5 promoter of the invention.

In still a further aspect, the present invention provides a pharmaceutical composition comprising a carrier and a vector of the invention.

In yet another aspect, the present invention provides a method for AAVmediated delivery of a transgene to a host involving the step of delivering to a selected host a recombinant viral vector comprising a selected transgene under the control of sequences which direct expression thereof and an adeno-associated virus 1 (AAV-1) virion.

In another aspect, the invention provides a method for in vitro production of a selected gene product using a vector of the invention.

Other aspects and advantages of the invention will be readily apparent to one of skill in the art from the detailed description of the invention.

### Brief Description of the Drawings

Figs. 1A-1C illustrate the alignment of nucleotides of AAV-1 [SEQ ID NO: 1], AAV-2 [SEQ ID NO: 18] and AAV-6. [SEQ ID NO: 19]. The alignment was done with MacVector 6.0. The full sequences of AAV-1 are shown in the top line. Nucleotides in AAV-2 and AAV-6 identical to AAV-1 are symbolized by "." and gaps by "-". Some of the conserved features among AAVs are marked in this figure. Note the 3' ITRs of AAV-1 and AAV-6 are shown in different orientations.
Fig. 2 illustrates the predicted secondary structure of AAV-1 ITR. The nucleotides in AAV-2 and AAV-6 are shown in italic and bold respectively.
Fig. 3A illustrates a hypothesis of how AAV-6 arose from the homologous recombination between AAV-1 and AAV-2. The major elements of AAV-1 are indicated in the graph. A region that is shared between AAV-1, AAV-2 and AAV-6 is shown in box with waved lines.
Fig. 3B is a detailed illustration of a 71 bp homologous region among AAV-1, AAV-2 and AAV-6. Nucleotides that differ among these serotypes are indicated by arrows.
Fig. 4A is a bar chart illustrating expression levels of human alpha 1 antitrypsin (α1AT) in serum following delivery of hAAT via recombinant AAV-1 and recombinant AAV-2 viruses.
Fig. 4B is a bar chart illustrating expression levels of erythropoietin (epo) in serum following delivery of the epo gene via recombinant AAV-1 and recombinant AAV-2 viruses.
Fig. 5A is a bar chart illustrating expression levels of α1AT in liver following delivery of α1AT as described in Example 7.
Fig. 5B is a bar chart demonstrating expression levels of epo in liver following delivery of epo as described in Example 7.
Fig. 5C is a bar chart demonstrating neutralizing antibodies (NAB) directed to AAV-1 following delivery of α1AT or epo to liver as described in Example 7.
Fig. 5D is a bar chart demonstrating neutralizing antibodies (NAB) directed to AAV-2 following delivery of α1AT or epo to liver as described in Example 7.
Fig. 6A is a bar chart illustrating expression levels of α1AT in muscle following delivery of α1AT as described in Example 7.
Fig. 6B is a bar chart demonstrating expression levels of epo in muscle following delivery of epo as described in Example 7.
Fig. 6C is a bar chart demonstrating neutralizing antibodies (NAB) directed to AAV-1 following delivery of α1AT or epo to muscle as described in Example 7.
Fig. 6D is a bar chart demonstrating neutralizing antibodies (NAB) directed to AAV-2 following delivery of α1AT or epo to muscle as described in Example 7.

### Detailed Description of the Invention

The present invention provides novel nucleic acid sequences for an adenoassociated virus of serotype 1 (AAV-1). Also provided are fragments of these AAV-1 sequences. Among particularly desirable AAV-1 fragments are the inverted terminal repeat sequences (ITRs), rep and cap. Each of these fragments may be readily utilized, e.g., as a cassette, in a variety of vector systems and host cells. Such fragments may be used alone, in combination with other AAV-1 sequences or fragments, or in combination with elements from other AAV or non-AAV viral sequences. In one particularly desirable embodiment, a cassette may contain the AAV-1 ITRs of the invention flanking a selected transgene. In another desirable embodiment, a cassette may contain the AAV-1 rep and/or cap proteins, e.g., for use in producing recombinant (rAAV) virus.

Thus, the AAV-1 sequences and fragments thereof are useful in production of rAAV, and are also useful as antisense delivery vectors, gene therapy vectors, or vaccine vectors. The invention further provides nucleic acid molecules, gene delivery vectors, and host cells which contain the AAV-1 sequences of the invention. Also provided a novel methods of gene delivery using AAV vectors.

As described herein, the vectors of the invention containing the AAV-1 capsid proteins of the invention are particularly well suited for use in applications in which the neutralizing antibodies diminish the effectiveness of other AAV serotype based vectors, as well as other viral vectors. The rAAV vectors of the invention are particularly advantageous in rAAV readministration and repeat gene therapy.

These and other embodiments and advantages of the invention are described in more detail below. As used throughout this specification and the claims, the term "comprising" is inclusive of other components, elements, integers, steps and the like.

### I. AAV-1 NUCLEIC ACID AND PROTEIN SEQUENCES

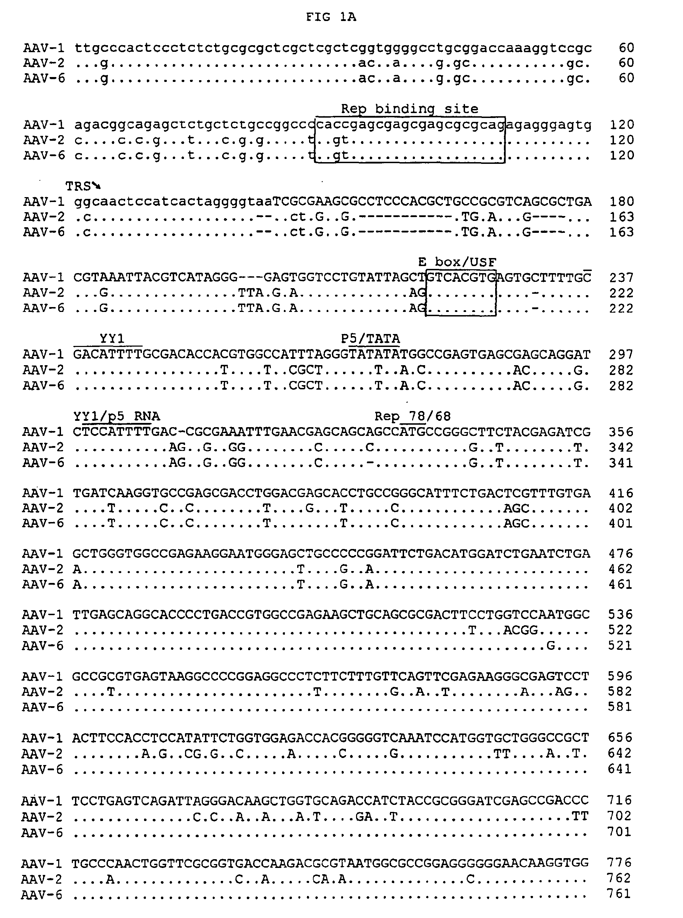

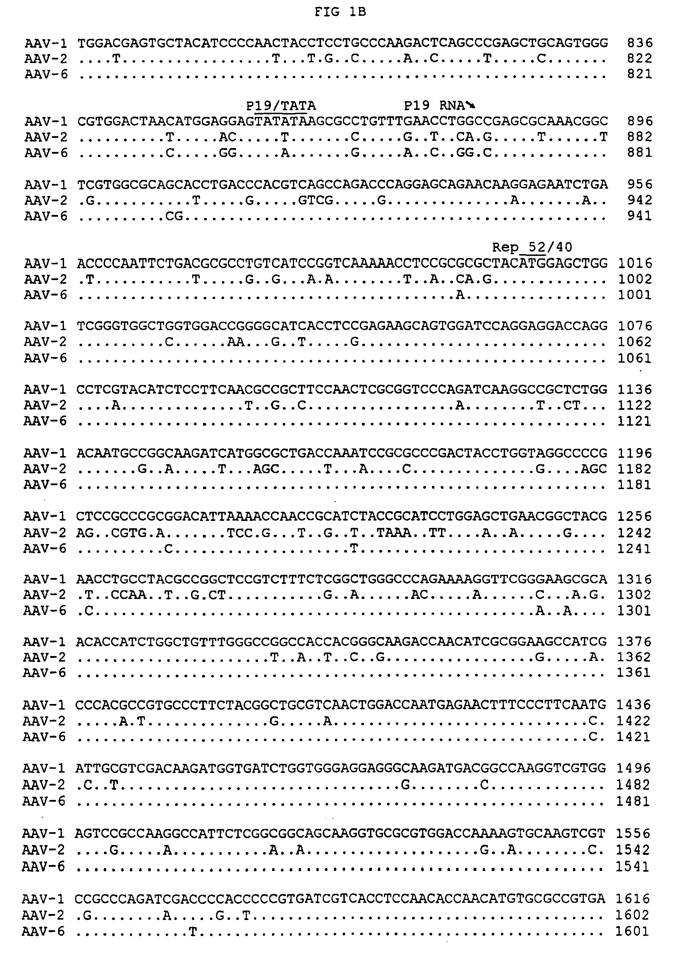

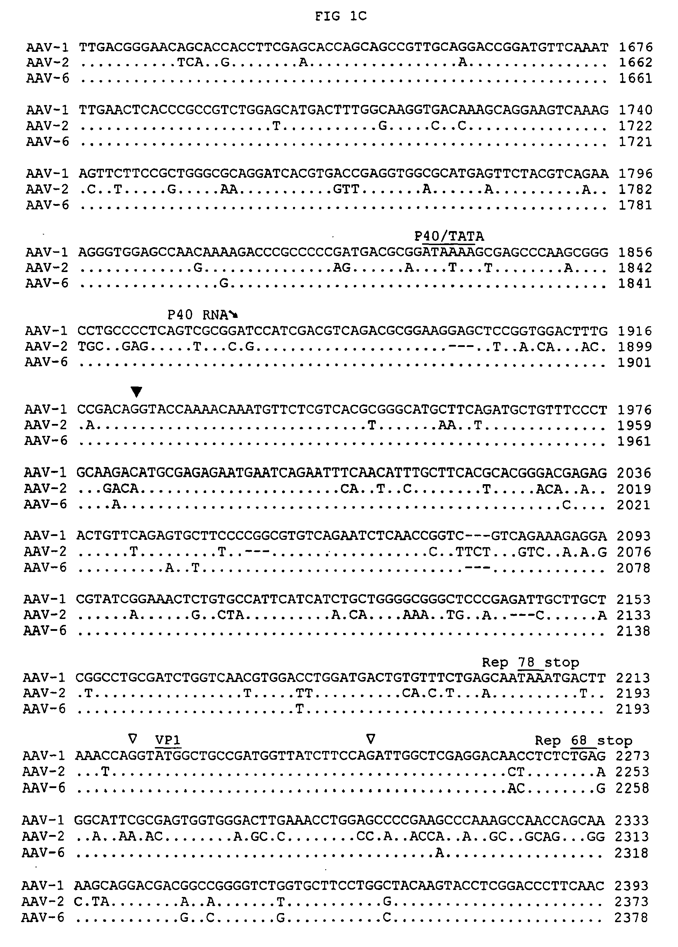

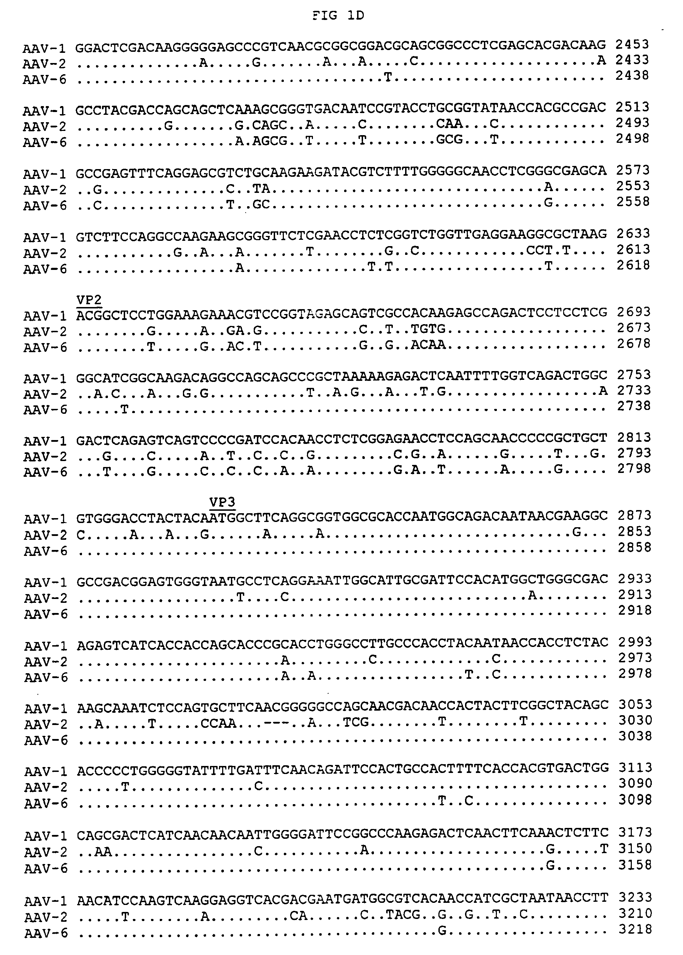

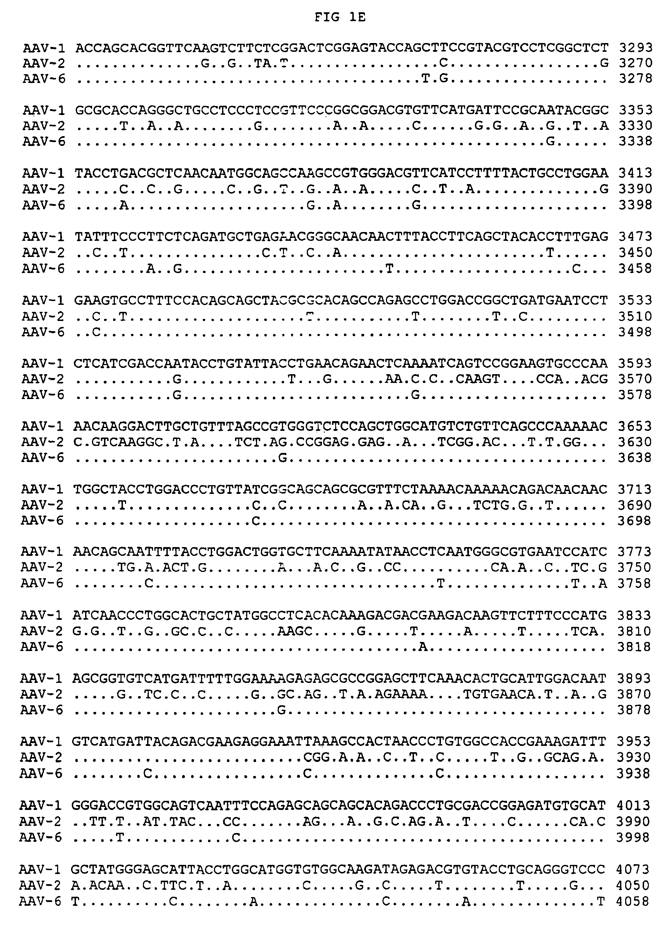

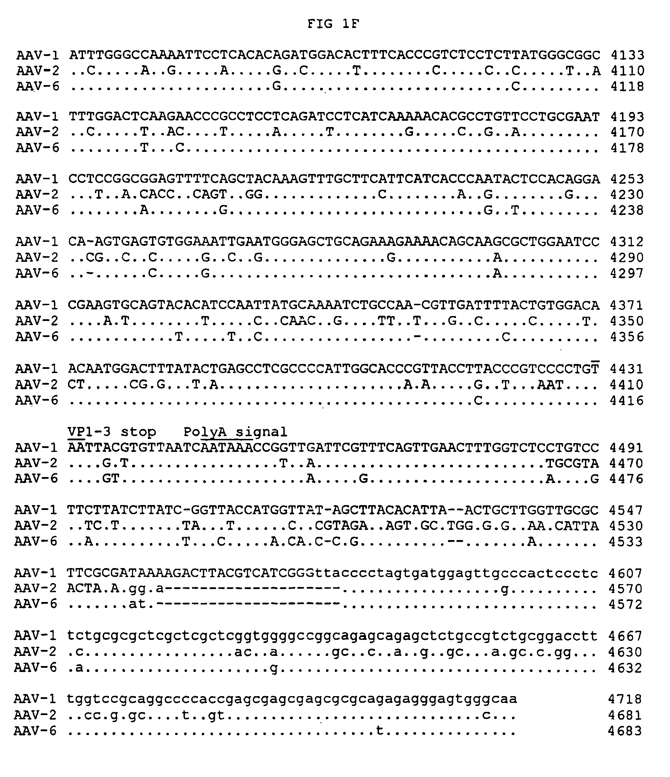

The AAV-1 nucleic acid sequences of the invention include the DNA sequences of SEQ ID NO: 1 (Figs. 1A-1C), which consists of 4718 nucleotides. The AAV-1 nucleic acid sequences of the invention further encompass the strand which is complementary to SEQ ID NO: 1, as well as the RNA and cDNA sequences corresponding to SEQ ID NO: 1 and its complementary strand. Also included in the nucleic acid sequences of the invention are natural variants and engineered modifications of SEQ ID NO: 1 and its complementary strand. Such modifications include, for example, labels which are known in the art, methylation, and substitution of one or more of the naturally occurring nucleotides with an analog.

Further included in this invention are nucleic acid sequences which are greater than 85%, preferably at least about 90%, more preferably at least about 95%, and most preferably at least about 98 - 99% identical or homologous to SEQ ID NO:1. The term "percent sequence identity" or "identical" in the context of nucleic acid sequences refers to the residues in the two sequences which are the same when aligned for maximum correspondence. The length of sequence identity comparison may be over the full-length sequence, or a fragment at least about nine nucleotides, usually at least about 20 - 24 nucleotides, at least about 28 - 32 nucleotides, and preferably at least about 36 or more nucleotides. There are a number of different algorithms known in the art which can be used to measure nucleotide sequence identity. For instance, polynucleotide sequences can be compared using Fasta, a program in GCG Version 6.1. Fasta provides alignments and percent sequence identity of the regions of the best overlap between the query and search sequences (Pearson, 1990, herein incorporated by reference). For instance, percent sequence identity between nucleic acid sequences can be determined using Fasta with its default parameters (a word size of 6 and the NOPAM factor for the scoring matrix) as provided in GCG Version 6.1, herein incorporated by reference.

The term "substantial homology" or "substantial similarity," when referring to a nucleic acid or fragment thereof, indicates that, when optimally aligned with appropriate nucleotide insertions or deletions with another nucleic acid (or its complementary strand), there is nucleotide sequence identity in at least about 95 - 99% of the sequence.

Also included within the invention are fragments of SEQ ID NO: 1, its complementary strand, cDNA and RNA complementary thereto. Suitable fragments are at least 15 nucleotides in length, and encompass functional fragments which are of biological interest. Certain of these fragments may be identified by reference to Figs. 1A-1C. Examples of particularly desirable functional fragments include the AAV-1 inverted terminal repeat (ITR) sequences of the invention. In contrast to the 145 nt ITRs of AAV-2, AAV-3, and AAV-4, the AAV-1 ITRs have been found to consist of only 143 nucleotides, yet advantageously are characterized by the T-shaped hairpin structure which is believed to be responsible for the ability of the AAV-2 ITRs to direct site-specific integration. In addition, AAV-1 is unique among other AAV serotypes, in that the 5' and 3' ITRs are identical. The full-length 5' ITR sequences of AAV-1 are provided at nucleotides 1-143 of SEQ ID NO: 1 (Fig. 1A) and the full-length 3' ITR sequences of AAV-1 are provided at nt 4576-4718 of SEQ ID NO: 1 (Fig. 1C). One of skill in the art can readily utilize less than the full-length 5' and/or 3' ITR sequences for various purposes and may construct modified ITRs using conventional techniques, e.g., as described for AAV-2 ITRs in Samulski et al, Cell, 33:135-143 (1983).

Another desirable functional fragment of the AAV-1 genome is the P5 promoter of AAV-1 which has sequences unique among AAV P5 promoters, while maintaining critical regulatory elements and functions. This promoter is located within nt 236 - 299 of SEQ ID NO: 1 (Fig. 1A). Other examples of functional fragments of interest include the sequences at the junction of the rep/cap, e.g., the sequences spanning nt 2306-2223, as well as larger fragments which encompass this junction which may comprise 50 nucleotides on either side of this junction. Still other examples of functional fragments include the sequences encoding the rep proteins. Rep 78 is located in the region of nt 334 - 2306 of SEQ ID NO: 1; Rep 68 is located in the region of nt 334-2272, and contains an intron spanning nt 1924-2220 of SEQ ID NO: 1. Rep 52 is located in the region of nt 1007 - 2304 of SEQ ID NO: 1; rep 40 is located in the region of nt 1007 - 2272, and contains an intron spanning nt 1924-2246 of SEQ ID NO: 1. Also of interest are the sequences encoding the capsid proteins, VP 1 [nt 2223-4431 of SEQ ID NO: 1], VP2 [nt 2634-4432 of SEQ ID NO: 1] and VP3 [nt 2829-4432 of SEQ ID NO: 1]. Other fragments of interest may include the AAV-1 P19 sequences, AAV-1 P40 sequences, the rep binding site, and the terminal resolute site (TRS).

The invention further provides the proteins and fragments thereof which are encoded by the AAV-1 nucleic acids of the invention. Particularly desirable proteins include the rep and cap proteins, which are encoded by the nucleotide sequences identified above. These proteins include rep 78 [SEQ ID NO:5], rep 68 [SEQ ID NO:7], rep 52 [SEQ ID NO:9], rep 40 [SEQ ID NO: 11], vp1 [SEQ ID NO: 13], vp2 [SEQ ID NO: 15], and vp3 [SEQ IID NO: 17] and functional fragments thereof while the sequences of the rep and cap proteins have been found to be closely related to those of AAV-6, there are differences in the amino acid sequences (see Table 1 below), as well as differences in the recognition of these proteins by the immune system. However, one of skill in the art may readily select other suitable proteins or protein fragments of biological interest. Suitably, such fragments are at least 8 amino acids in length. However, fragments of other desired lengths may be readily utilized. Such fragments may be produced recombinantly or by other suitable means, e.g., chemical synthesis.

The sequences, proteins, and fragments of the invention may be produced by any suitable means, including recombinant production, chemical synthesis, or other synthetic means. Such production methods are within the knowledge of those of skill in the art and are not a limitation of the present invention.

### II. VIRAL VECTORS

In another aspect, the present invention provides vectors which utilize the AAV-1 sequences of the invention, including fragments thereof, for delivery of a heterologous gene or other nucleic acid sequences to a target cell. Suitably, these heterologous sequences (i.e., a transgene) encode a protein or gene product which is capable of being expressed in the target cell. Such a transgene may be constructed in the form of a "minigene". Such a "minigene" includes selected heterologous gene sequences and the other regulatory elements necessary to transcribe the gene and express the gene product in a host cell. Thus, the gene sequences are operatively linked to regulatory components in a manner which permit their transcription. Such components include conventional regulatory elements necessary to drive expression of the transgene in a cell containing the viral vector. The minigene may also contain a selected promoter which is linked to the transgene and located, with other regulatory elements, within the selected viral sequences of the recombinant vector.

Selection of the promoter is a routine matter and is not a limitation of this invention. Useful promoters may be constitutive promoters or regulated (inducible) promoters, which will enable control of the timing and amount of the transgene to be expressed. For example, desirable promoters include the cytomegalovirus (CMV) immediate early promoter/enhancer [see, e.g., Boshart et al, Cell, 41:521-530 (1985)], the Rous sarcoma virus LTR promoter/enhancer, and the chicken cytoplasmic β-actin promoter [T. A. Kost et al, Nucl, Acids Res., 11(23):8287 (1983)]. Still other desirable promoters are the albumin promoter and an AAV P5 promoter. Optionally, the selected promoter is used in conjunction with a heterologous enhancer, e.g., the β-actin promoter may be used in conjunction with the CMV enhancer. Yet other suitable or desirable promoters and enhancers may be selected by one of skill in the art.

The minigene may also desirably contain nucleic acid sequences heterologous to the viral vector sequences including sequences providing signals required for efficient polyadenylation of the transcript (poly-A or pA) and introns with functional splice donor and acceptor sites. A common poly-A sequence which is employed in the exemplary vectors of this invention is that derived from the papovavirus SV-40. The poly-A sequence generally is inserted in the minigene downstream of the transgene sequences and upstream of the viral vector sequences. A common intron sequence is also derived from SV-40, and is referred to as the SV40 T intron sequence. A minigene of the present invention may also contain such an intron, desirably located between the promoter/enhancer sequence and the transgene. Selection of these and other common vector elements are conventional [see, e.g., Sambrook et al, "Molecular Cloning. A Laboratory Manual", 2d edit., Cold Spring Harbor Laboratory, New York (1989) and references cited therein] and many such sequences are available from commercial and industrial sources as well as from Genebank.

The selection of the transgene is not a limitation of the present invention. Suitable transgenes may be readily selected from among desirable reporter genes, therapeutic genes, and optionally, genes encoding immunogenic polypeptides. Examples of suitable reporter genes include β-galactosidase (β-gal), an alkaline phosphatase gene, and green fluorescent protein (GFP). Examples of therapeutic genes include, cytokines, growth factors, hormones, and differentiation factors, among others. The transgene may be readily selected by one of skill in the art. See, e.g., WO 98/09657, which identifies other suitable transgenes.

Suitably, the vectors of the invention contain, at a minimum, cassettes which consist of fragments of the AAV-1 sequences and proteins. In one embodiment, a vector of the invention comprises a selected transgene, which is flanked by a 5' ITR and a 3' ITR, at least one of which is an AAV-1 ITR of the invention. Suitably, vectors of the invention may contain a AAV-1 P5 promoter of the invention. In yet another embodiment, a plasmid or vector of the invention contains AAV-1 rep sequences. In still another embodiment, a plasmid or vector of the invention contains at least one of the AAV-1 cap proteins of the invention. Most suitably, these AAV-1-derived vectors are assembled into viral vectors, as described herein.

### A. AAV Viral Vectors

In one aspect, the present invention provides a recombinant AAV-1 viral vector produced using the AAV-1 capsid proteins of the invention. The packaged rAAV-1 virions of the invention may contain, in addition to a selected minigene, other AAV-1 sequences, or may contain sequences from other AAV serotypes.

Methods of generating rAAV virions are well known and the selection of a suitable method is not a limitation on the present invention. See, e.g., K. Fisher et al, J. Virol., 70:520-532 (1993) and US Patent 5,478,745. In one suitable method, a selected host cell is provided with the AAV sequence encoding a rep protein, the gene encoding the AAV cap protein and with the sequences for packaging and subsequent delivery. Desirably, the method utilizes the sequences encoding the AAV-1 rep and/or cap proteins of the invention.

In one embodiment, the rep/cap genes and the sequences for delivery are supplied by co-transfection of vectors carrying these genes and sequences. In one currently preferred embodiment, a cis (vector) plasmid, a trans plasmid containing the rep and cap genes, and a plasmid containing the adenovirus helper genes are cotransfected into a suitable cell line, e.g., 293. Alternatively, one or more of these functions may be provided in trans via separate vectors, or may be found in a suitably engineered packaging cell line.

An exemplary cis plasmid will contain, in 5' to 3' order, AAV 5' ITR, the selected transgene, and AAV 3' ITR. In one desirable embodiment, at least one of the AAV ITRs is a 143 nt AAV-1 ITR. However, other AAV serotype ITRs may be readily selected. Suitably, the full-length ITRs are utilized. However, one of skill in the art can readily prepare modified AAV ITRs using conventional techniques. Similarly, methods for construction of such plasmids is well known to those of skill in the art.

A trans plasmid for use in the production of the rAAV-1 virion particle may be prepared according to known techniques. In one desired embodiment, this plasmid contains the rep and cap proteins of AAV-1, or functional fragments thereof. Alternatively, the rep sequences may be from another selected AAV serotype.

The cis and trans plasmid may then be co-transfected with a wild-type helper virus (e.g., Ad2, Ad5, or a herpesvirus), or more desirably, a replication - defective adenovirus, into a selected host cell. Alternatively, the cis and trans plasmid may be co-transfected into a selected host cell together with a transfected plasmid which provides the necessary helper functions. Selection of a suitable host cell is well within the skill of those in the art and include such mammalian cells as 293 cells, HeLa cells; among others.

Alternatively, the cis plasmid and, optionally the trans plasmid, may be transfected into a packaging cell line which provides the remaining helper functions necessary for production of a rAAV containing the desired AAV-1 sequences of the invention. An example of a suitable packaging cell line, where an AAV-2 capsid is desired, is B-50, which stably expresses AAV-2 rep and cap genes under the control of a homologous P5 promoter. This cell line is characterized by integration into the cellular chromosome of multiple copies (at least 5 copies) of PS-rep-cap gene cassettes in a concatomer form. This B-50 cell line was deposited with the American Type Culture Collection, 10801 University Boulevard, Manassas, Virginia 20110-2209, on September 18, 1997 under Accession No. CRL-12401 pursuant to the provisions of the Budapest Treaty. However, the present invention is not limited as to the selection of the packaging cell line.

Exemplary transducing vectors based on AAV-1 capsid proteins have been tested both *in vivo and in vitro,* as described in more detail in Example 4. In these studies, it was demonstrated that recombinant AAV vector with an AAV-1 virion can transduce both mouse liver and muscle. These, and other AAV-1 based gene therapy vectors which may be generated by one of skill in the art are beneficial for gene delivery to selected host cells and gene therapy patients since the neutralization antibodies of AAV-1 present in much of the human population exhibit different patterns from other AAV serotypes and therefore do not neutralize the AAV-1 virions. One of skill in the art may readily prepare other rAAV viral vectors containing the AAV-1 capsid proteins provided herein using a variety of techniques known to those of skill in the art. One may similarly prepare still other rAAV viral vectors containing AAV-1 sequence and AAV capsids of another serotype.

### B. Other Viral Vectors

One of skill in the art will readily understand that the AAV-1 sequences of the invention can be readily adapted for use in these and other viral vector systems for *in vitro, ex vivo or in vivo* gene delivery. Particularly well suited for use in such viral vector systems are the AAV-1 ITR sequences, the AAV-1 rep, the AAV-1 cap, and the AAV-1 P5 promoter sequences.

For example, in one desirable embodiment, the AAV-1 ITR sequences of the invention may be used in an expression cassette which includes AAV- 5' ITR, a non-AAV DNA sequences of interest (e.g., a minigene), and 3' ITR and which lacks functional rep/cap. Such a cassette containing an AAV-1 ITR may be located on a plasmid for subsequent transfection into a desired host cell, such as the cis plasmid described above. This expression cassette may further be provided with an AAV capsid of a selected serotype to permit infection of a cell or stably transfected into a desired host cell for packaging of rAAV virions. Such an expression cassette may be readily adapted for use in other viral systems, including adenovirus systems and lentivirus systems. Methods of producing Ad/AAV vectors are well known to those of skill in the art. One desirable method is described in PCT/US95/14018. However, the present invention is not limited to any particular method.

Another aspect of the present invention is the novel AAV-1 P5 promoter sequences which are located in the region spanning nt 236 - 299 of SEQ ID NO: 1. This promoter is useful in a variety of viral vectors for driving expression of a desired transgene.

Similarly, one of skill in the art can readily select other fragments of the AAV-1 genome of the invention for use in a variety of vector systems. Such vectors systems may include, e.g., lentiviruses, retroviruses, poxviruses, vaccinia viruses, and adenoviral systems, among others. Selection of these vector systems is not a limitation of the present invention.

### C. Host Cells And Packaging Cell Lines

In yet another aspect, the present invention provides host cells which may be transiently transfected with AAV-1 nucleic acid sequences of the invention to permit expression of a desired transgene or production of a rAAV particle. For example, a selected host cell may be transfected with the AAV-1 P5 promoter sequences and/or the AAV-1 5' ITR sequences using conventional techniques. Providing AAV helper functions to the transfected cell lines of the invention results in packaging of the rAAV as infectious rAAV particles. Such cell lines may be produced in accordance with known techniques [see, e.g, US Patent No. 5,658,785], making use of the AAV-1 sequences of the invention.

Alternatively, host cells of the invention may be stably transfected with a rAAV expression cassette of the invention, and with copies of AAV-1 rep and cap genes. Suitable parental cell lines include mammalian cell lines and it may be desirable to select host cells from among non-simian mammalian cells. Examples of suitable parental cell lines include, without limitation, HeLa [ATCC CCL 2], A549 [ATCC Accession No. CCL 185], KB [CCL 17], Detroit [e.g., Detroit 510, CCL 72] and WI-38 [CCL 75] cells. These cell lines are all available from the American Type Culture Collection, 10801 University Boulevard, Manassas, Virginia 20110-2209 USA. Other suitable parent cell lines may be obtained from other sources and may be used to construct stable cell lines containing the P5 and/or AAV rep and cap sequences of the invention.

Recombinant vectors generated as described above are useful for delivery of the DNA of interest to cells.

### III. METHODS OF DELIVERING GENES VIA AAV-1 DERIVED VECTORS

In another aspect, the present invention provides a method for delivery of a transgene to a host which involves transfecting or infecting a selected host cell with a recombinant viral vector generated with the AAV-1 sequences (or functional fragments thereof) of the invention. Methods for delivery are well known to those of skill in the art and are not a limitation of the present invention.

In one desirable embodiment, the invention provides a method for AAVmediated delivery of a transgene to a host. This method involves transfecting or infecting a selected host cell with a recombinant viral vector containing a selected transgene under the control of sequences which direct expression thereof and AAV-1 capsid proteins.

Optionally, a sample from the host may be first assayed for the presence of antibodies to a selected AAV serotype. A variety of assay formats for detecting neutralizing antibodies are well known to those of skill in the art. The selection of such an assay is not a limitation of the present invention. See, e.g., Fisher et al, Nature Med., 3(3):306-312 (March 1997) and W. C. Manning et al, Human Gene Therapy, 9:477-485 (March 1, 1998). The results of this assay may be used to determine which AAV vector containing capsid proteins of a particular serotype are preferred for delivery, e.g., by the absence of neutralizing antibodies specific for that capsid serotype.

In one aspect of this method, the delivery of vector with AAV-1 capsid proteins may precede or follow delivery of a gene via a vector with a different serotype AAV capsid protein. Thus, gene delivery via rAAV vectors may be used for repeat gene delivery to a selected host cell. Desirably, subsequently administered rAAV vectors carry the same transgene as the first rAAV vector, but the subsequently administered vectors contain capsid proteins of serotypes which differ from the first vector. For example, if a first vector has AAV-2 capsid proteins, subsequently administered vectors may have capsid proteins selected from among the other serotypes, including AAV-1, AAV-3A, AAV-3B, AAV-4 and AAV-6.

Thus, a rAAV-1-derived recombinant viral vector of the invention provides an efficient gene transfer vehicle which can deliver a selected transgene to a selected host cell in *vivo or ex vivo* even where the organism has neutralizing antibodies to one or more AAV serotypes. These compositions are particularly well suited to gene delivery for therapeutic purposes. However, the compositions of the invention may also be useful in immunization. Further, the compositions of the invention may also be used for production of a desired gene product *in vitro.*

The above-described recombinant vectors may be delivered to host cells according to published methods. An AAV viral vector bearing the selected transgene may be administered to a patient, preferably suspended in a biologically compatible solution or pharmaceutically acceptable delivery vehicle. A suitable vehicle includes sterile saline. Other aqueous and non-aqueous isotonic sterile injection solutions and aqueous and non-aqueous sterile suspensions known to be pharmaceutically acceptable carriers and well known to those of skill in the art may be employed for this purpose.

The viral vectors are administered in sufficient amounts to transfect the cells and to provide sufficient levels of gene transfer and expression to provide a therapeutic benefit without undue adverse effects, or with medically acceptable physiological effects, which can be determined by those skilled in the medical arts. Conventional and pharmaceutically acceptable routes of administration include, but are not limited to, direct delivery to the liver, oral, intranasal, intravenous, intramuscular, subcutaneous, intradermal, and other parental routes of administration. Routes of administration may be combined, if desired.

Dosages of the viral vector will depend primarily on factors such as the condition being treated, the age, weight and health of the patient, and may thus vary among patients. For example, a therapeutically effective human dosage of the viral vector is generally in the range of from about 1 ml to about 100 ml of solution containing concentrations of from about 1 x 10⁹ to 1 x 10¹⁶ genomes virus vector. A preferred human dosage may be about 1 x 10¹³ to 1 x 10¹⁶ AAV genomes. The dosage will be adjusted to balance the therapeutic benefit against any side effects and such dosages may vary depending upon the therapeutic application for which the recombinant vector is employed. The levels of expression of the transgene can be monitored to determine the frequency of dosage resulting in viral vectors, preferably AAV vectors containing the minigene. Optionally, dosage regimens similar to those described for therapeutic purposes may be utilized for immunization using the compositions of the invention. For *in vitro* production, a desired protein may be obtained from a desired culture following transfection of host cells with a rAAV containing the gene encoding the desired protein and culturing the cell culture under conditions which permits expression. The expressed protein may then be purified and isolated, as desired. Suitable techniques for transfection, cell culturing, purification, and isolation are known to those of skill in the art.

The following examples illustrate several aspects and embodiments of the invention.

### Example 1 - Generation of Infectious Clone of AAV-1

The replicated form DNA of AAV-1 was extracted from 293 cells that were infected by AAV-1 and wild type adenovirus type 5.

### A. Cell Culture and Virus

AAV-free 293 cells and 84-31 cells were provided by the human application laboratory of the University of Pennsylvania. These cells were cultured in Dulbecco's Modified Eagle Medium with 10% fetal bovine serum (Hyclone), penicillin (100 U/ml) and streptomycin at 37°C in a moisturized environment supplied with 5% CO₂. The 84-31 cell line constitutively expresses adenovirus genes Ela, Elb, E4/ORF6, and has been described previously [K. J. Fisher, J. Virol., 70:520-532 (1996)]. AAV-1 (ATCC VR-645) seed stock was purchased from American Type Culture Collection (ATCC, Manassas, VA). AAV viruses were propagated in 293 cells with wild type Ad5 as a helper virus.

### B. Recombinant AAV Generation

The recombinant AAV viruses were generated by transfection using an adenovirus free method. Briefly, the cis plasmid (with AAV ITR), trans plasmid (with AAV rep gene and cap gene) and helper plasmid (pF△13, with essential regions from the adenovirus genome) were simultaneously co-transfected into 293 cells in a ratio of 1:1:2 by calcium phosphate precipitation. The pF△13 helper plasmid has an 8 kb deletion in the adenovirus E2B region and has deletions in most of the late genes. This helper plasmid was generated by deleting the RsrII fragment from pFG140 (Microbix, Canada). Typically, 50 µg ofDNA (cis:trans:PF△13 at ratios of 1:1:2, respectively) was transfected onto a 15 cm tissue culture dish. The cells were harvested 96 hours post-transfection, sonicated and treated with 0.5% sodium deoxycholate (37°C for 10 min). Cell lysates were then subjected to two rounds of a CsCl gradient. Peak fractions containing AAV vector were collected, pooled, and dialyzed against PBS before injecting into animals. To make rAAV virus with AAV-1 virion, the pAVIH or p5E18 (2/1) was used as the *trans* plasmid to provide rep and cap function.

For the generation of rAAV based on AAV-2, p5E18 was used as the *trans* plasmid since it greatly improved the rAAV yield. This plasmid, p5E 18(2/2), expresses AAV-2 Rep and Cap and contains a P5 promoter relocated to a position 3' to the Cap gene, thereby minimizing expression of Rep78 and Rep68. The strategy was initially described by Li et al, J. Virol., 71:5236-5243 (1997). P5E18(2/2) was constructed in the following way. The previously described pMMTV-trans vector (i.e., the mouse mammary tumor virus promoter substituted for the P5 promoter in an AAV-2-based vector) was digested with *Sma*I and *Cla*I, filled in with the Klenow enzyme, and then recircularized with DNA ligase. The resulting construct was digested with *Xba*I, filled in, and ligated to the blunt-ended BamHI-*Xba*I fragment from pCR-p5, constructed in the following way. The P5 promoter of AAV was amplified by PCR and the amplified fragment was subsequently cloned into pCR2.1 (Invitrogen) to yield pCR-P5. The helper plasmid pAV1H was constructed by cloning the *Bfa*I fragment of pAAV-2 into pBluescript II-SK(+) at the *Bcor*V and *Sma*l sites. The 3.0-kb *Xba*I*-Kpn*I fragment from p5E18(2/2), the 2.3-kb *Xba*I*-Kpn*I fragment from pAVIH, and the 1.7-kb KpnI fragment from p5E18(2/2) were incorporated into a separate plasmid P5E18(2/1), which contains AAV-2 Rep, AAV-1 Cap, and the AAV-2 P5 promoter located 3' to the Cap gene. Plasmid p5E18(2/1) produced 10- to 20-fold higher quantities of the vector than pAV1H (i.e., 10¹² genomes/50 15-cm² plates).

### C. DNA Techniques

Hirt DNA extraction was performed as described in the art with minor modification [R.J. Samulski et al., Cell, 33:135-143 (1983)]. More particularly, Hirst solution without SDS was used instead of using original Hirt solution containing SDS. The amount of SDS present in the original Hirst solution was added after the cells had been fully suspended. To construct AAV-1 infectious clone, the Hirt DNA from AAV-1 infected 293 cells was repaired with Klenow enzyme (New England Biolabs) to ensure the ends were blunt. The treated AAV-1 Hirt DNA was then digested with *BamH*I and cloned into three vectors, respectively. The internal *BamH*I was cloned into pBlueScript H-SK+ cut with *BamH*I to get pAVI-BM. The left and right fragments were cloned into pBlueScfipt II-SK+ cut with *BamH*I + EcoRV to obtain pAV1-BL and pAV1-BR, respectively. The AAV sequence in these three plasmids were subsequently assembled into the same vector to get AAV-1 infectious clone pAAV-1. The helper plasmid for recombinant AAV-1 virus generation was constructed by cloning the Bfa I fragment of pAAV-1 into pBlueScript II-SK+ at the EcoRV site.

Analysis of the Hirt DNA revealed three bands, a dimer at 9.4 kb, a monomer at 4.7 kb and single-stranded DNA at 1.7 kb, which correlated to different replication forms of AAV-1. The monomer band was excised from the gel and then digested with *BamH*I*.* This resulted in three fragments of 1.1 kb, 0,8 kb and 2.8 kb. This pattern is in accordance with the description by Bantel-schaal and zur Hausen, Virol., 134(1):52-63 (1984). The 1.1 kb and 2.8 kb *Bam*HI fragments were cloned into pBlueScfipt-KS(+) at *BamH*I and EcoRV site. The internal 0.8 kb fragment was cloned into *BamH*I site of pBlueScript-KS(+).

These three fragments were then subcloned into the same construct to obtain a plasmid (pAAV-1) that contained the full sequence of AAV-1. The pAAV-1 was then tested for its ability to rescue from the plasmid backbone and package infectious virus. The pAAV-1 was then transfected to 293 cells and supplied with adenovirus type as helper at MOI 10. The virus supernatant was used to reinfect 293 cells.

For Southern blot analysis, Hirt DNA was digested with *Dpn*I to remove bacteria-borne plasmid and probed with internal *BamH*I fragment of AAV-1. The membrane was then washed at high stringency conditions, which included: twice 30 minutes with 2X SSC, 0.1% SDS at 65°C and twice 30 minutes with 0.1X SSC, 0.1% SDS at 65°C. The membrane was then analyzed by both phosphor image and X-ray autoradiography. The results confirmed that pAAV-1 is indeed an infectious clone of AAV serotype 1.

### Example 2- Sequencing Analysis of AAV-1

The entire AAV-1 genome was then determined by automatic sequencing and was found to be 4718 nucleotides in length (Figs. 1A-1C). For sequencing, an ABI 373 automatic sequencer as used to determine the sequences for all plasmids and PCR fragments related to this study using the FS dye chemistry. All sequences were confirmed by sequencing both plus and minus strands. These sequences were also confirmed by sequencing two independent clones of pAV-BM, pAV-BL and pAV-BR. Since the replicated form of AAV-1 DNA served as the template for sequence determination, these sequences were also confirmed by sequencing a series of PCR products using original AAV-1 seed stock as a template.

The length of AAV-1 was found to be within the range of the other serotypes: AAV-3 (4726 nucleotides), AAV-4 (4774 nucleotides), AAV-2 (4681 nucleotides), and AAV-6 (4683 nucleotides).

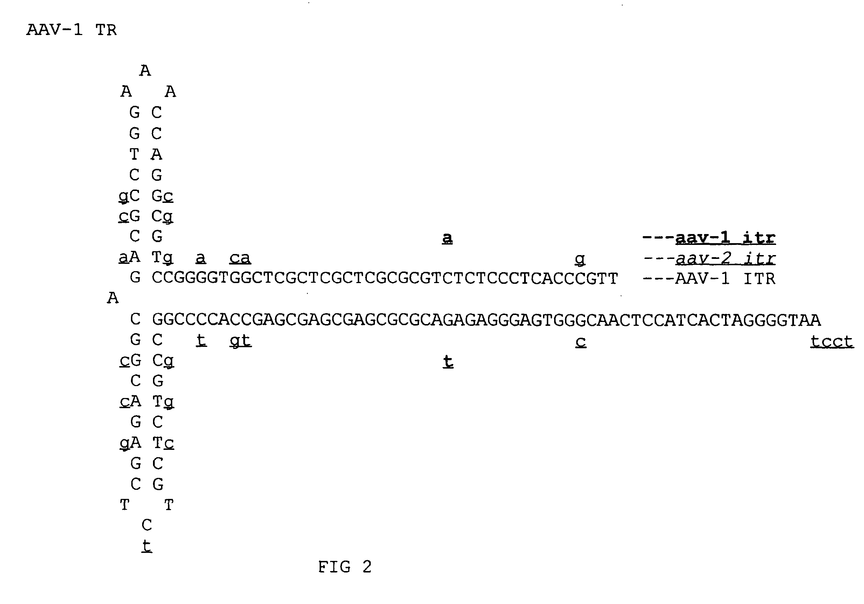

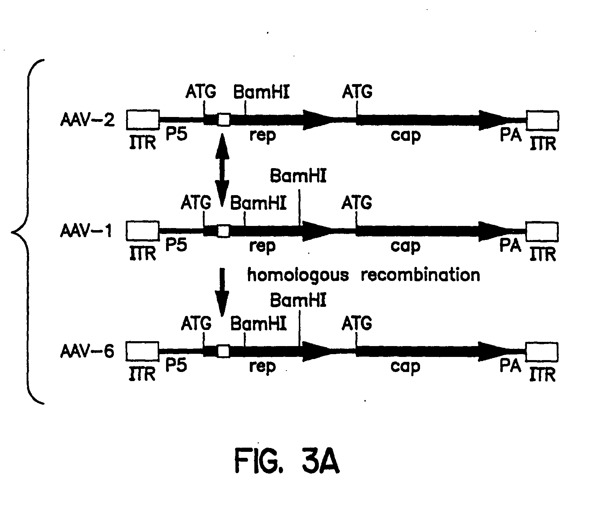

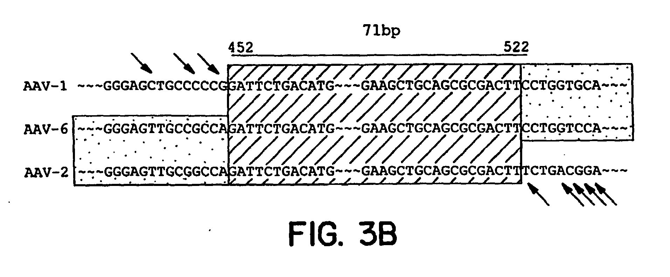

The AAV-1 genome exhibited similarities to other serotypes of adeno-associated viruses. Overall, it shares more than 80% identity with other known AAV viruses as determined by the computer program Megalign using default settings [DNASTAR, Madison, WI]. The key features in AAV-2 can also be found in AAV-1. First, AAV-1 has the same type of inverted terminal repeat which is capable of forming T-shaped hairpin structures, despite the differences at the nucleotide level (Figs. 2 and 3). The sequences of right ITRs and left ITRs of AAV-1 are identical. The AAV TR sequence is subdivided into A, A', B, B', C, C', D and D' [Bern, cited above].

These AAV ITR sequences are also virtually the same as those found in AAV-6 right ITR, there being one nucleotide difference in each of A and A' sequence, and the last nucleotide of the D sequence. Second, the AAV-2 rep binding motif [GCTCGCTCGCTCGCTG (SEQ ID NO: 20)] is well conserved. Such motif can also be found in the human chromosome 19 AAV-2 pre-integration region. Finally, non-structural and structural coding regions, and regulatory elements similar to those of other AAV serotypes also exist in AAV-1 genome.

Although the overall features of AAV terminal repeats are very much conserved, the total length of the AAV terminal repeat exhibits divergence. The terminal repeat of AAV-1 consists of 143 nucleotides while those of AAV-2, AAV-3, and AAV-4 are about 145 or 146 nucleotides. The loop region of AAV-1 ITR most closely resembles that of AAV-4 in that it also uses TCT instead of the TTT found in AAV-2 and AAV-3. The possibility of sequencing error was eliminated using restriction enzyme digestion, since these three nucleotides are part of the SacI site (gagctc; nt 69-74 of SEQ ID NO: 1). The p5 promoter region of AAV-1 shows more variations in nucleotide sequences with other AAV serotypes. However, it still maintains the critical regulatory elements. The two copies of YY1 [See, Fig. 1A-1C] sites seemed to be preserved in all known AAV serotypes, which have been shown to be involved in regulating AAV gene expression. In AAV-4, there are 56 additional nucleotides inserted between YY 1 and E-box/USF site, while in AAV-1, there are 26 additional nucleotides inserted before the E-box/USF site. The p19 promoter, p40 promoter and polyA can also be identified from the AAV-1 genome by analogy to known AAV serotypes, which are also highly conserved.

Thus, the analysis of AAV terminal repeats of various serotypes showed that the A and A' sequence is very much conserved. One of the reasons may be the Rep binding motif (GCTC)₃GCTG [SEQ ID NO: 20]. These sequences appear to be essential for AAV DNA replication and site-specific integration. The same sequence has also been shown to be preserved in a monkey genome [Samulski, personal communication]. The first 8 nucleotides of the D sequence are also identical in all known AAV serotypes. This is in accordance with the observation of the Srivastava group that only the first 10 nucleotides are essential for AAV packaging [X.S. Wang et al, J. Virol., 71:3077-3082 (1997); X.S. Wang et al, J. Virol., 71:1140-1146 (1997)]. The function of the rest of the D sequences still remain unclear. They may be somehow related to their tissue specificities. The variation of nucleotide in B and C sequence may also suggest that the secondary structure of the ITRs is more critical for its biological function, which has been demonstrated in many previous publications.

### Example 3- Comparison of AAV-1 Sequences

The nucleotide sequences of AAV-1, obtained as described above, were compared with known AAV sequences, including AAV-2, AAV-4 and AAV-6 using DNA Star Megalign. This comparison revealed a stretch of 71 identical nucleotides shared by AAV-1, AAV-2 and AAV-6. See, Figs. 1A-1C.

This comparison further suggested that AAV-6 is a hybrid formed by homologous recombination of AAV-1 and AAV-2. See, Figs. 3A and 3B. These nucleotides divide the AAV-6 genome into two regions. The 5' half of AAV-6 of 522 nucleotides is identical to that of AAV-2 except in 2 positions. The 3' half of AAV-6 including the majority of the rep gene, complete cap gene and 3' ITR is 98% identical to AAV-1.

Biologically, such recombination may enable AAV-1 to acquire the ability to transmit through the human population. It is also interesting to note that the ITRs of AAV-6 comprise one AAV-1 ITR and one AAV-2 ITR. The replication model of defective parvovirus can maintain this special arrangement. Studies on AAV integration have shown that a majority of AAV integrants carries deletions in at least one of the terminal repeats. These deletions have been shown to be able to be repaired through gene conversion using the other intact terminal repeat as a template. Therefore, it would be very difficult to maintain AAV-6 as a homogenous population when an integrated copy of AAV-6 is rescued from host cells with helper virus infection. The AAV-6 with two identical AAV-2 ITRs or two identical AAV-1 ITRs should be the dominant variants. The AAV-6 with two AAV-1 ITRs has been observed by Russell's group [Rutledge, cited above (1998)]. So far there is no report on AAV-6 with two AAV-2 ITRs. Acquirement of AAV-2 P5 promoter by AAV-6 may have explained that AAV-6 have been isolated from human origin while AAV-1 with the same virion has not. The regulation of P5 promoter between different species of AAV may be different *in vivo.* This observation suggests the capsid proteins of AAV were not the only determinants for tissue specificity.

Although it is clear that AAV-6 is a hybrid of AAV-1 and AAV-2, AAV-6 has already exhibited divergence from either AAV-1 or AAV-2. There are two nucleotide differences between AAV-6 and AAV-2 in their first 450 nucleotides. There are about 1% differences between AAV-6 and AAV-1 in nucleotide levels from nucleotides 522 to the 3' end. There also exists a quite divergent region (nucleotide 4486-4593) between AAV-6 and AAV-1 (Figs. 1A-1C). This region does not encode any known proteins for AAVs. These differences in nucleotide sequences may suggest that AAV-6 and AAV-1 have gone through some evolution since the recombination took place. Another possible explanation is that there exists another variant of AAV-1 which has yet to be identified. So far, there is no evidence to rule out either possibility. It is still unknown if other hybrids (AAV-2 to AAV-4, etc.) existed in nature.

The coding region of AAV-1 was deduced by comparison with other known AAV serotypes. Table 1 illustrates the coding region differences between AAV-1 and AAV-6. The amino acid residues are deduced according to AAV-2.

With reference to the amino acid position of AAV-1, Table 1 lists the amino acids of AAV-1 which have been changed to the corresponding ones of AAV-6. The amino acids of AAV-1 are shown to the left of the arrow. Reference may be made to SEQ ID NO: 5 of the amino acid sequence of AAV-1 Rep 78 and to SEQ ID NO: 13 for the amino acid sequence of AAV-1 VP1.

**Table 1**

| Coding region variations between AAV-1 and AAV-6 | | | | |
|---|---|---|---|---|
| Rep protein (Rep78) | | | Cap protein (VP1) | |
| Position(s) | Amino acids | | Position(s) | Amino acids |
| 28 | S→N | | 129 | L-F |
| 191 | Q→H | | 418 | E→D |
| 192 | H→D | | 531 | E→K |
| 308 | E→D | | 584 | F→L |
| | | | 598 | A→V |
| | | | 642 | N→H |

It was surprising to see that the sequence of the AAV-1 coding region is almost identical to that of AAV-6 from position 452 to the end of coding region (99%). The first 508 nucleotides of AAV-6 have been shown to be identical to those of AAV-2 [Rutledge, cited above (1998)]. Since the components of AAV-6 genome seemed to be AAV-2 left ITR - AAV-2 p5 promoter - AAV-1 coding region - AAV-1 right ITR, it was concluded that AAV-6 is a naturally occurred hybrid between AAV-1 and AAV-2.

### Example 4 - Gene Therapy Vector Based on AAV-1

Recombinant gene transfer vectors based on AAV-1 viruses were constructed by the methods described in Example 1. To produce a hybrid recombinant virus with AAV-1 virion and AAV-2 ITR, the AAV-1 trans plasmid (pAV1H) and the AAV-2 cis-lacZ plasmid (with AAV-2 ITR) were used. The AAV-2 ITR was used in this vector in view of its known ability to direct site-specific integration. Also constructed for use in this experiment was an AAV-1 vector carrying the green fluorescent protein (GFP) marker gene under the control of the immediate early promoter of CMV using pAV1H as the trans plasmid.

### A. rAAV-1 Viruses Transfect Host Cells in Vitro

84-31 cells, which are subclones of 293 cells (which express adenovirus E1a, E1b) which stably express E4/ORF5, were infected with rAAV-1 GFP or rAAV-lacZ. High levels of expression of GFP and lacZ was detected in the cultured 84-31 cells. This suggested that rAAV-1 based vector was very similar to AAV-2 based vectors in ability to infect and expression levels.

### B. rAAV- 1 Viruses Transfect Cells in Vivo

The performance of AAV-1 based vectors was also tested *in vivo.* The rAAV-1 CMV-α1AT virus was constructed as follows. The EcoRI fragment of pAT85 (ATCC) containing human α1-antitrypsin (α1AT) cDNA fragment was blunted and cloned into PCR (Promega) at a SmaI site to obtain PCR-α1AT. The CMV promoter was cloned into PCR-α1AT at the Xbal site. The Alb-α1AT expression cassette was removed by XhoI and Clal and cloned into pAV1H at the Xbal site. This vector plasmid was used to generate AAV-1-CMV-α1AT virus used in the experiment described below.

For screening human antibodies against AAV, purified AAV virus is lysed with Ripa buffer (10 mM Tris pH 8.2, 1% Triton X-100, 1% SDS, 0.15 M NaCI) and separated in 10% SDS-PAGE gel. The heat inactivated human serum was used at a I to 1000 dilution in this assay. The rAAV-1 CMV-α1AT viruses were injected into Rag-1 mice through tail vein injection at different dosages. The concentration of human al-antitrypsin in mouse serum was measured using ELISA. The coating antibody is rabbit anti-human human al-antitrypsin (Sigma). The goat-antihuman α1-antitrypsin (Sigma) was used as the primary detection antibodies. The sensitivity of this assay is around 0.3 ng/ml to 30 ng/ml. The expression of human α-antitrypsin in mouse blood can be detected in a very encouraging level. This result is shown in Table 2.

**Table 2**

| Human Antitrypsin Expressed in Mouse Liver | | |
|---|---|---|
| Amount of virus injected | Week 2 (ng/ml) | Week 4 (ng/ml) |
| 2x10¹⁰ genomes | 214.2 | 171.4 |
| 1x10¹⁰ genomes | 117.8 | 109.8 |
| 5x10¹⁰ genomes | 64.5 | 67.8 |
| 2.5x10¹⁰ genomes | 30.9 | 58.4 |

rAAV-1 CMV-lacZ viruses were also injected into the muscle of C57BL6 mice and similar results were obtained. Collectively, these results suggested that AAV-1 based vector would be appropriate for both liver and muscle gene delivery.

### Example 5 - Neutralizing Antibodies Against AAV-1

Simple and quantitative assays for neutralizing antibodies (NAB) to AAV-1 and AAV-2 were developed with recombinant vectors. A total of 33 rhesus monkeys and 77 normal human subjects were screened.

### A. Nonhuman Primates

Wild-caught juvenile rhesus monkeys were purchased from Covance (Alice, Tex.) and LABS of Virginia (Yemassee, SC) and kept in full quarantine. The monkeys weighed approximately 3 to 4 kg. The nonhuman primates used in the Institute for Human Gene Therapy research program are purposefully bred in the United States from specific-pathogen-free closed colonies. All vendors are US Department of Agriculture class A dealers. The rhesus macaques are therefore not infected with important simian pathogens, including the tuberculosis agent, major simian lentiviruses (simian immunodeficiency virus and simian retroviruses), and cercopithecine herpesvirus. The animals are also free of internal and external parasites. The excellent health status of these premium animals minimized the potential for extraneous variables. For this study, serum was obtained from monkeys prior to initiation of any protocol.

NAB titers were analyzed by assessing the ability of serum antibody to inhibit the transduction of reporter virus expressing green fluorescent protein (GFP) (AAV1-GFP or AAV2-GFP) into 84-31 cells. Various dilutions of antibodies preincubated with reporter virus for 1 hour at 37°C were added to 90% confluent cell cultures. Cells were incubated for 48 hours and the expression of green fluorescent protein was measured by Fluorolmaging (Molecular Dynamics). NAB titers were calculated as the highest dilution at which 50% of the cells stained green.

Analysis of NAB in rhesus monkeys showed that 61 % of animals tested positive for AAV-1; a minority (24%) has NAB to AAV-2. Over one-third of animals had antibodies to AAV-1 but not AAV-2 (i.e., were monospecific for AAV-1), whereas no animals were positive for AAV-2 without reacting to AAV-1. These data support the hypothesis that AAV-1 is endemic in rhesus monkeys. The presence of true AAV-2 infections in this group of nonhuman primates is less clear, since cross-neutralizing activity of an AAV-1 response to AAV-2 can not be ruled out. It is interesting that there is a linear relationship between AAV-2 NAB and AAV-1 NAB in animals that had both.

### B. Humans

For these neutralization antibody assays, human serum samples were incubated at 56°C for 30 min to inactivate complement and then diluted in DMEM. The virus (rAAV or rAd with either lacZ or GFP) was then mixed with each serum dilution (20X, 400X, 2000X, 4000X, etc.) and incubated for 1 hour at 37°C before applied to 90% confluent cultures of84-31 cells (for AAV) or Hela cells (for adenovirus) in 96-well plates. After 60 minutes of incubation at culture condition, 100 µl additional media containing 20% FCS was added to make final culture media containing 10% FCS.

The result is summarized in Table 3.

**Table 3**

| Adenovirus | AAV-1 | AAV-2 | # of samples | Percentage |
|---|---|---|---|---|
| - | - | - | 41 | 53.2% |
| + | - | - | 16 | 20.8% |
| - | + | - | 0 | 0.0% |
| - | - | + | 2 | 2.6% |
| - | + | + | 2 | 2.6% |
| + | - | + | 3 | 3.9% |
| + | + | - | 0 | 0.0% |
| + | + | + | 13 | 16.9% |
| | | Total | 77 | 100% |

The human neutralizing antibodies against these three viruses seemed to be unrelated since the existence of neutralizing antibodies against AAV are not indications for antibodies against adenovirus. However, AAV requires adenovirus as helper virus, in most of the cases, the neutralizing antibodies against AAV correlated with the existence of neutralizing antibodies to adenovirus. Among the 77 human serum samples screened, 41% of the samples can neutralize the infectivity of recombinant adenovirus based on Ad5. 15/77 (19%) of serum samples can neutralize the transduction of rAAV-1 while 20/77 (20%) of the samples inhibit rAAV-2 transduction at 1 to 80 dilutions or higher. All serum samples positive in neutralizing antibodies for AAV-1 in are also positive for AAV-2. However, there are five (6%) rAAV-2 positive samples that failed to neutralize rAAV-1. In samples that are positive for neutralizing antibodies, the titer of antibodies also varied in the positive ones. The results from screening human sera for antibodies against AAVs supported the conclusion that AAV-1 presents the same epitome as that of AAV-2 to interact with cellular receptors since AAV-1 neutralizing human serums can also decrease the infectivity of AAV-2. However, the profile of neutralizing antibodies for these AAVs is not identical, there are additional specific receptors for each AAV serotype.

### Example 6 - Recombinant AAV Viruses Exhibit Tissue Tropism

The recombinant AAV-1 vectors of the invention and the recombinant AAV-2 vectors [containing the gene encoding human α1-antitrypsin (α1AT) or murine erythropoietin (Epo) from a cytomegalovirus-enhanced β-actin promoter (CB)] were evaluated in a direct comparison to equivalent copies of AAV-2 vectors containing the same vector genes.

Recombinant viruses with AAV-1 capsids were constructed using the techniques in Example 1. To make rAAV with AAV-1 virions, pAV1H or p5E18 (2/1) was used as the *trans* plasmid to provide Rep and Cap functions. For the generation of the rAAV based on AAV-2, p5E18(2/2) was used as the *trans* plasmid, since it greatly improved the rAAV yield. [Early experiments indicated similar *in vivo* performances of AAV-1 vectors produced with pAVIH and p5E19 (2/1). All subsequent studies used AAV-1 vectors derived from p5E18(2/1) because of the increased yield.]

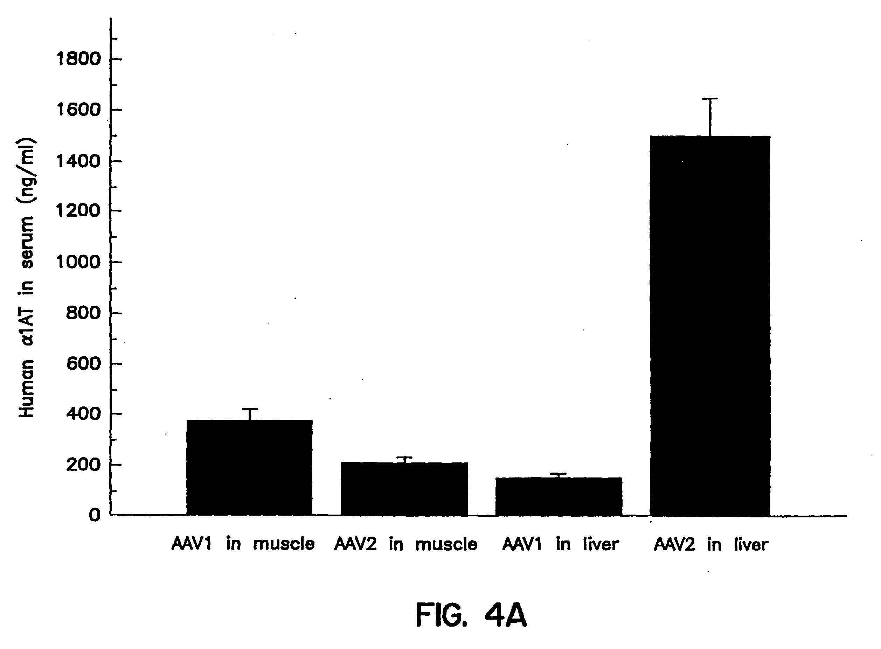

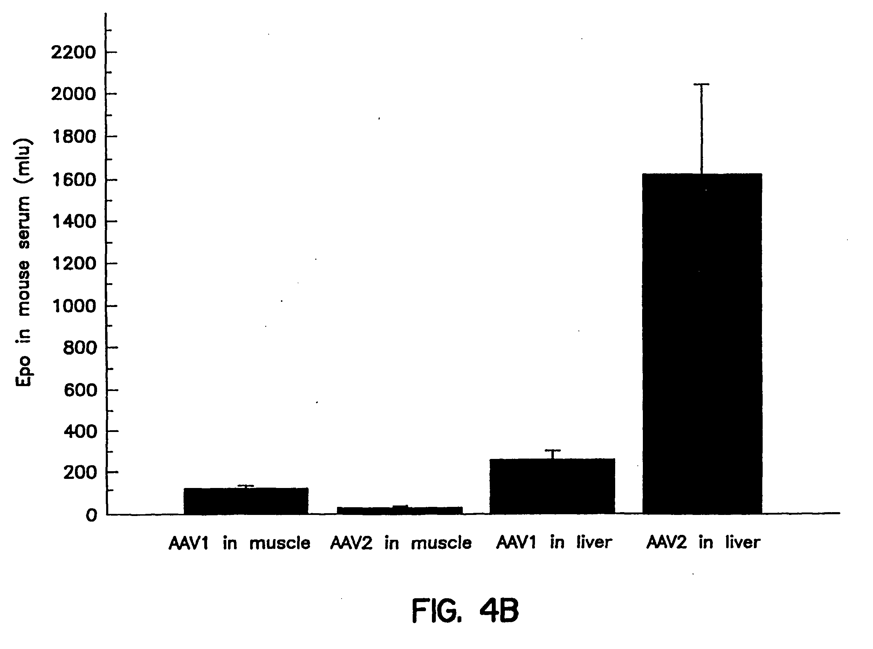

Equivalent stocks of the AAV-1 and AAV-2 vectors were injected intramuscularly (5 x 10¹⁰ genomes) or liver via the portal circulation (I x 10¹¹ genomes) into immunodeficient mice, and the animals (four groups) were analyzed on day 30 for expression of transgene. See, Figs. 4A and 4B.

AAV-2 vectors consistently produced 10- to 50-fold more serum erythropoietin or α1-antitrypsin when injected into liver compared to muscle. (However, the AAV-1-delivered genes did achieve acceptable expression levels in the liver.) This result was very different from that for AAV-1 vectors, with which muscle expression was equivalent to or greater than liver expression. In fact, AAV.1 outperformed AAV-2 in muscle when equivalent titers based on genomes were administered.

### Example 7 - Gene Delivery via rAAV-1

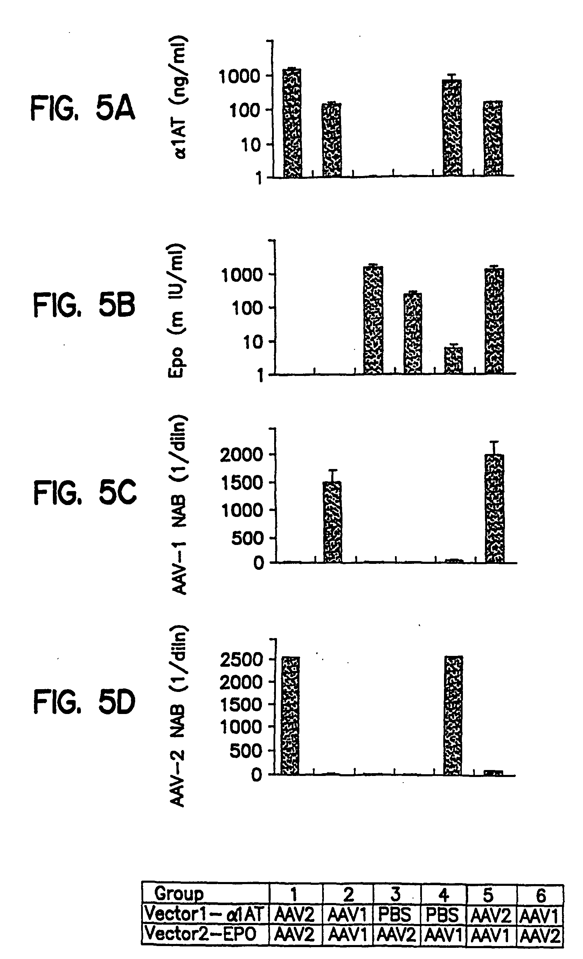

C57BL/6 mice (6- to 8-week old males, Jackson Laboratories) were analyzed for AAV mediated gene transfer to liver following intrasplenic injection of vector (i.e., targeted to liver). A total of 10¹¹ genome equivalents of rAAV-1 or rAAV-2 vector were injected into the circulation in 100 µl buffered saline. The first vector contained either an AAV-1 capsid or an AAV-2 capsid and expressed α1AT under the control of the chicken β-actin (CB) promoter. Day 28 sera were analyzed for antibodies against AAV-1 or AAV-2 and serum α1AT levels were checked. Animals were then injected with an AAV-1 or AAV-2 construct expressing erythropoietin (Epo, also under the control of the CB promoter). One month later sera was analyzed for serum levels of Epo. The following groups were analyzed (Figs. 5A-5D).

In Group 1, vector 1 was AAV-2 expressing α1AT and vector 2 was AAV-2 expressing Epo. Animals generated antibodies against AAV-2 following the first vector administration which prevented the readministration of the AAV-2 based vector. There was no evidence for cross-neutralizing the antibody to AAV-1.

In Group 2, vector 1 was AAV-1 expressing α1AT while vector 2 was AAV-1 expressing Epo. The first vector administration did result in significant α1AT expression at one month associated with antibodies to neutralizing antibodies to AAV-1. The animals were not successfully readministered with the AAV-1 Epo expressing construct.

In Group 3, the effectiveness of an AAV-2 vector expressing Epo injected into a naive animal was measured. The animals were injected with PBS and injected with AAV-2 Epo vector at day 28 and analyzed for Epo expression one month later. The neutralizing antibodies were evaluated at day 28 so we did not expect to see anything since they received PBS with the first vector injection. This shows that in naive animals AAV-2 is very efficient at transferring the Epo gene as demonstrated by high level of serum Epo one month later.

Group 4 was an experiment similar to Group 3 in which the animals originally received PBS for vector 1 and then the AAV-1 expressing Epo construct 28 days later. At the time of vector injection, there obviously were no antibodies to either AAV-1 or AAV-2. The AAV-1 based vector was capable of generating significant expression ofEpo when measured one month later.

Group 5 is a cross-over experiment where the initial vector is AAV-2 expressing α1AT followed by the AAV-1 construct expressing Epo. The animals, as expected, were efficiently infected with the AAV-2 vector expressing α1AT as shown by high levels of the protein in blood at 28 days. This was associated with significant neutralizing antibodies to AAV-2. Importantly, the animals were successfully administered AAV-1 following the AAV-2 vector as shown by the presence of Epo in serum 28 days following the second vector administration. At the time of this vector administration, there was high level AAV-2 neutralizing antibodies and very low cross-reaction to AAV-1. The level ofEpo was slightly diminished possibly due to a small amount of cross-reactivity. Group 6 was the opposite cross-over experiment in which the initial vector was AAV-1 based, whereas the second experiment was AAV-2 based. The AAV-1 vector did lead to significant gene expression of α1AT, which also resulted in high level AAV-1 neutralizing antibody. The animals were very efficiently administered AAV-2 following the initial AAV-1 vector as evidenced by high level Epo.

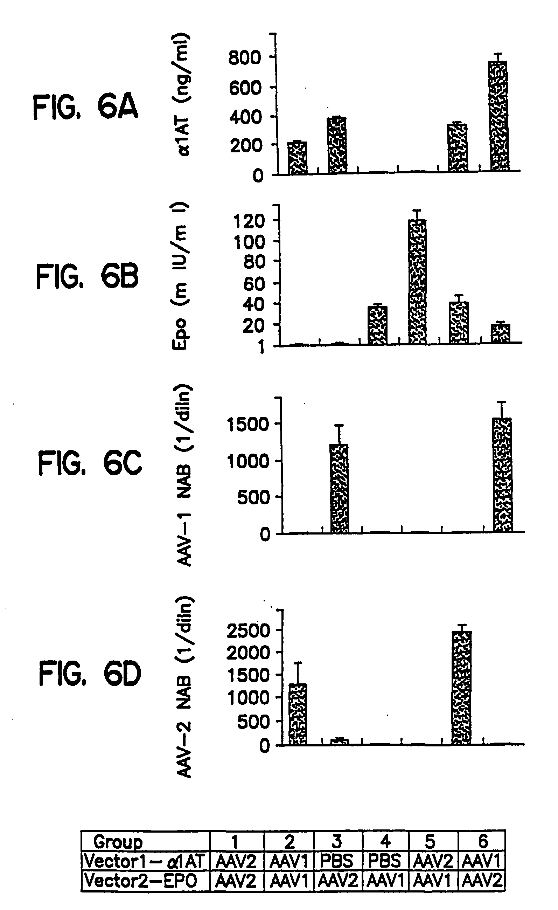

A substantially identical experiment was performed in muscle in which 5 x 10¹⁰ genomes were injected into the tibialis anterior of C57BL/6 mice as a model for muscle directed gene therapy. The results are illustrated in Figs. 6A-6D and are essentially the same as for liver.

In summary, this experiment demonstrates the utility of using an AAV-1 vector in patients who have pre-existing antibodies to AAV-2 or who had initially received an AAV-2 vector and need readministration.

### Example 8 - Construction of Recombinant Viruses Containing AAV-1 ITRs

This example illustrates the construction of recombinant AAV vectors which contain AAV-1 ITRs of the invention.

An AAV-1 cis plasmid is constructed as follows. A 160 bp Xho-NruI AAV-1 fragment containing the AAV-1 5' ITR is obtained from pAV1-BL pAV1-BL was generated as described in Example 1. The Xho-NruI fragment is then cloned into a second pAV1-BL plasmid at an XbaI site to provide the plasmid with two AAV-1 ITRs. The desired transgene is then cloned into the modified pAV-1BL at the Nrul and BamHI site, which is located between the AAV-1 ITR sequences. The resulting AAV-1 cis plasmid contains AAV-1 ITRs flanking the transgene and lacks functional AAV-1 rep and cap.

Recombinant AAV is produced by simultaneously transfecting three plasmids into 293 cells. These include the AAV-1 cis plasmid described above; a trans plasmid which provides AAV rep/cap functions and lacks AAV ITRs; and a plasmid providing adenovirus helper functions. The rep and/or cap functions may be provided in trans by AAV-1 or another AAV serotype, depending on the immunity profile of the intended recipient. Alternatively, the rep or cap functions may be provided in cis by AAV-1 or another serotype, again depending on the patient's immunity profile.

In a typical cotransfection, 50 µg of DNA (cis:trans: helper at ratios of 1:1:2, respectively) is transfected onto a 15 cm tissue culture dish. Cells are harvested 96 hours post transfection, sonicated and treated with 0.5% sodium deoxycholate (37° for 10 min). Cell lysates are then subjected to 2-3 rounds of ultracentrifugation in a cesium gradient. Peak fractions containing rAAV are collected, pooled and dialyzed against PBS. A typical yield is 1 x 10¹³ genomes/10⁹ cells.

Using this method, one recombinant virus construct is prepared which contains the AAV-1 ITRs flanking the transgene, with an AAV-1 capsid. Another recombinant virus construct is prepared with contains the AAV-1 ITRs flanking the transgene, with an AAV-2 capsid.

All publications cited in this specification are incorporated herein by reference. While the invention has been described with reference to a particularly preferred embodiments, it will be appreciated that modifications can be made without departing from the spirit of the invention. Such modifications are intended to fall within the scope of the claims.

## Claims

1. An isolated AAV-1 nucleic acid molecule comprising a sequence selected from the group consisting of
(a) SEQ ID NO: 1;
(b) a DNA sequence complementary to SEQ ID NO: 1;
(c) cDNA complementary to (a) or (b); and
(d) RNA complementary to any of (a) to (c).

2. A nucleic acid molecule comprising an AAV-1 inverted terminal repeat (ITR) sequence selected from the group consisting of:
(a) nt 1 to 143 of SEQ ID NO: 1;
(b) nt 4576 to 4718 of SEQ ID NO: 1;
(c) a nucleic acid sequence complementary to (a) or (b); and
(d) a functional fragment of (a), (b), or (c).

3. A recombinant vector comprising a 5' AAV-1 inverted terminal repeat (ITR) and a selected transgene, wherein said ITR has the sequence selected from the group consisting of
(a) nt 1 to 143 of SEQ ID NO: 1;
(b) a nucleic acid sequence complementary to (a); and
(c) a functional fragment of (a) or (b).

4. The recombinant vector according to claim 3, wherein said vector further comprises a 3' AAV-1 ITR.

5. A recombinant vector comprising a 3' AAV-1 inverted terminal repeat (ITR) and a selected transgene, wherein said ITR has the sequence selected from the group consisting of:
(a) nt 4576 to 4718 of SEQ ID NO: 1;
(b) a nucleic acid sequence complementary to (a); and
(c) a functional fragment of (a) or (b).

6. The recombinant vector according to claim 5, wherein said vector further comprises a 5' AAV-1 ITR.

7. The recombinant vector according to any of claims 3-6, wherein said vector further comprises AAV-1 capsid proteins having the sequence of SEQ ID NO: 13, 15 or 17 or functional fragments thereof

8. The recombinant vector according to any of claims 3-6, wherein said vector further comprises adenovirus sequences.

9. A recombinant vector comprising an AAV-1 P5 promoter having the sequence of nt 236 to 299 of SEQ ID NO: 1 or a functional fragment thereof.

10. A nucleic acid molecule encoding AAV-1 helper functions, said molecule comprising an AAV rep coding region and an AAV cap coding region, wherein said cap coding region comprises at least one member is selected from the group consisting of:
(a) vp1, nt 2223 to 4431 of SEQ ID NO: 1;
(b) vp2, nt 2634 to 4432 of SEQ ID NO: 1; and
(c) vp3, nt 2829 to 4432 of SEQ ID NO: 1.

11. A nucleic acid molecule encoding AAV-1 helper functions, said molecule comprising an AAV rep coding region and an AAV cap coding region, wherein said rep coding region comprises an AAV-1 rep coding region comprising at least one member selected from the group consisting of:
(a) rep 78, nt 335 to 2304 of SEQ ID NO: 1;
(b) rep 68, nt 335 to 2272 of SEQ ID NO: 1 or the cDNA corresponding thereto;
(c) rep 52, nt 1007 to 2304 of SEQ ID NO: 1; and
(d) rep 40, nt 1007 to 2272 of SEQ ID NO: 1 or the cDNA corresponding thereto.

12. A host cell transduced with a recombinant viral vector according to any of claims 3-6.

13. A host cell transduced with a nucleic acid molecule according to any of claims 1, 2, 10 or 11.

14. A host cell stably transduced with an AAV-1 P5 promoter having the sequence of nt 236 to 299 of SEQ ID NO: 1.

15. A pharmaceutical composition comprising a carrier and a virus comprising the vector according to any of claims 3-6.

16. A pharmaceutical composition comprising a carrier and a virus comprising the vector according to claim 7.

17. A pharmaceutical composition comprising a carrier and a virus comprising the vector according to claim 8.

18. A method for AAV-mediated delivery of a transgene comprising the step of delivering to a host cell an AAV virion which comprises:
(a) a capsid comprising at least one capsid protein encoded by an AAV-1 cap gene; and
(b) a DNA molecule comprising a transgene under the control of regulatory sequences directing its expression.

19. A method for AAV-mediated delivery of a transgene to a host comprising the steps of:
(a) assaying a sample from the host to determine the presence of neutralizing antibodies specific against any serotype of AAV; and
(b) delivering to the host an AAV virion which comprises:
(i) a capsid comprising at least one capsid protein encoded by a cap gene of an AAV serotype against which the host has no antibodies as determined in step (a); and
(ii) a DNA molecule comprising a transgene under the control of regulatory sequences directing its expression.

20. The method according to claim 19, comprising the additional step of repeating steps (a) and (b).

21. Use of an AAV virion which comprises a capsid comprising (a) at least one capsid protein encoded by a cap gene of an AAV serotype against which the host has antibodies, and (b) a DNA molecule comprising a transgene operably linked to regulatory sequences directing its expression,
in the preparation of a medicament for delivery of a transgene to a host, wherein said host has no preexisting neutralizing antibodies against the AAV serotype of said cap gene.

22. A method for delivery of a transgene comprising the step of delivering to a host cell a recombinant virus comprising a recombinant vector according to any of claims 3-8.

23. A method for producing a selected gene product comprising the steps of transfecting a mammalian cell with the molecule according to claim 1 or a functional fragment thereof and culturing said cell under conditions suitable to express said gene product.
